# EUROPEAN PATENT SPECIFICATION

(11) **EP 0 911 328 B1**
(45) Date of publication and mention of the grant of the patent: **08.02.2006**
(21) Application number: 96938533.5
(22) Date of filing: 22.11.1996
(51) Int. Cl.: C07D 401/04, C07D 498/06, A01N 43/42, A01N 43/84

(54) **SUBSTITUTED AMINOCYCLOALKYLPYRROLIDINE DERIVATIVES**
SUBSTITUIERTE AMINOCYCLOALKYLPYRROLIDINDERIVATE
DERIVES A BASE D'AMINOCYCLOALKYLPYRROLIDINE SUBSTITUEE

(30) Priority: 22.11.1995 JP 30412995; 23.07.1996 JP 19263796
(43) Date of publication of application: 28.04.1999
(73) Proprietor: DAIICHI PHARMACEUTICAL CO., LTD., Chuo-ku, Tokyo 103 (JP)
(72) Inventor: TAKEMURA, Makoto, Daiichi Phar. Co., Ltd., Edogawa-ku, Tokyo 134 (JP); KIMURA, Youichi, Daiichi Phar. Co., Ltd., Edogawa-ku, Tokyo 134 (JP); TAKAHASHI, Hisashi, Daiichi Phar. Co., Ltd., Edogawa-ku, Tokyo 134 (JP); KIMURA, Kenichi, Daiichi Phar. Co., Ltd., Edogawa-ku, Tokyo 134 (JP); MIYAUCHI, Satoru, Daiichi Phar. Co., Ltd., Edogawa-ku, Tokyo 134 (JP); OHKI, Hitoshi, Daiichi Phar. Co., Ltd., Edogawa-ku, Tokyo 134 (JP)
(74) Representative: Kinzebach, Werner
(86) International application number: PCT/JP1996/003440
(87) International publication number: WO 1997/019072

(56) References cited:
- WO-A-96/23782
- JP-A- 5 163 244
- JP-A- 7 300 416
- JP-A- 62 234 082
- CHEM. PHARM. BULL., 42(7), 1994, KIMURA, YOUICHI et al., pages 1442-54.

## Description

### TECHNICAL FIELD

This invention relates to an antibacterial compound useful for pharmaceutical preparations, animal drugs, drugs for fisheries use and antibacterial preservatives and also to antibacterial drugs and antibacterial preparations which contains the compound.

### BACKGROUND ART

Since the discovery of norfloxacin, antibacterial activity and distribution after administration of quinolone antibacterial agents have been improved, and many compounds are now used in the clinical field as chemotherapeutic agents which are effective in treating almost systemic infectious diseases.

In recent years, however, bacteria having low sensitivity to these drugs have been increasing in the clinical field. Also, like the case of a *Staphylococcus aureus* variant (MRSA) which is insensitive to β-lactam antibiotics, bacteria having low sensitivity to quinolone antibacterial agents are increasing among bacteria which are resistant to other drugs than quinolone antibacterial agents. In consequence, development of drugs having high efficacy has been called for in the field of clinics.

In addition, because a side effect in which severe convulsion is induced by the concomitant use of a non-steroidal anti-inflammatory drug and other side effects such as phototoxicity and the like have been revealed, great concern has also been directed toward the development of quinolone compounds having higher safety.

Kimura et al., *Pharm. Bull.* 42, p. 1442-1454 (1994) describe quinolones in which the 7-substituent may be a 3-(1-aminoalkyl)pyrrolidinyl or 3-(1-aminocycloalkyl)pyrrolidinyl group and the 1-substituent may be an ethyl or a cyclopropyl group. The same type of quinolones is described in EP 0 207 420 A1.

EP 0 593 766 A2 describes quinolones wherein the 7-substituent may be 3-aminopyrrolidinyl group which, in turn, may be further alkyl-substituted in 4-position of said pyrrolidinyl group. According to a particular embodiment said alkyl substituents form a spiro unit thereby resulting in, for instance, a 7-amino-5-azaspiro[2.4]heptan-5-yl group. These quinolones have a 2-halogenocyclopropyl group in 1-position.

EP 0 341493 A2 describes quinolones in which the 7-substituent may be, for instance, an optionally substituted 3-aminopyrrolidinyl or a 3-(1-aminoalkyl)pyrrolidinyl group. The substituent in 1-position is a 2-halogenocyclopropyl group.

EP 0 807 630 A2 describes quinolones wherein the 7-substituent may be a 3-(1-aminocycloalkyl)pyrrolidinyl, a 3-(1-aminoalkyl)pyrrolidinyl or a 1-amino-3-azabicyclo[3.1.0]hexan-3-yl group. The substituent in 1-position is a 2-halogenocyclopropyl group.

### DISCLOSURE OF INVENTION

In view of the above, the inventors of the present invention have conducted intensive studies with the aim of providing an excellent compound which can satisfy the aforementioned requirements and found as the result that a substituted aminomethylpyrrolidine derivative represented by the following formula (I) and salts thereof have a broad range of antibacterial spectrum, show strong antibacterial activity against quinolone-resistant bacteria including Gram positive bacteria, particularly MRSA, and also have excellent distribution and safety. The present invention has been accomplished on the basis of these findings.

Accordingly, the present invention relates to a compound represented by formula (I): 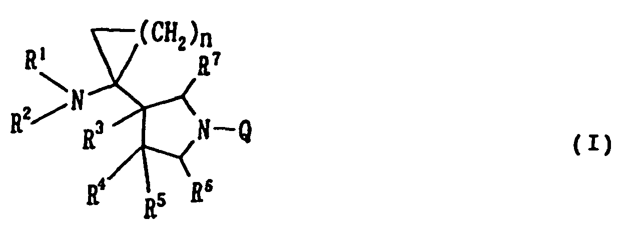 {wherein R¹ represents a hydrogen atom or an alkyl group having 1 to 6 carbon atoms;
R² represents a hydrogen atom or an alkyl group having 1 to 6 carbon atoms,
wherein the alkyl group may have at least one substituent selected from the group consisting of a hydroxyl group, a halogen atom, an alkylthio group having 1 to 6 carbon atoms and an alkoxyl group having 1 to 6 carbon atoms; R³ represents a hydrogen atom, a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms,
wherein the alkyl group may have at least one substituent selected from the group consisting of a hydroxyl group, a halogen atom and an alkoxyl group having 1 to 6 carbon atoms;
R⁴ represents a hydrogen atom, a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, and R⁵ represents a hydroxyl group, a halogen atom, a carbamoyl group, an alkyl group having 1 to 6 carbon atoms, an alkoxyl group having 1 to 6 carbon atoms or an alkylthio group having 1 o 6 carbon atoms,
wherein the alkyl group may have at least one substituent selected from the group consisting of a hydroxyl group, a halogen atom and an alkoxyl group having 1 to 6 carbon atoms, and
R⁴ and R⁵ may be combined to form a hydroxyimino group, a methylene chain having 3 to 6 carbon atoms (so as to form a spiro cyclic structure together with the pyrrolidine ring) or an alkyloxyimino group having 1 to 6 carbon atoms;
R⁶ and R⁷, each independently represents a hydrogen atom or an alkyl group having 1 to 6 carbon atoms;
n is an integer of 1 to 3; and
Q is a partial structure represented by formula (II): 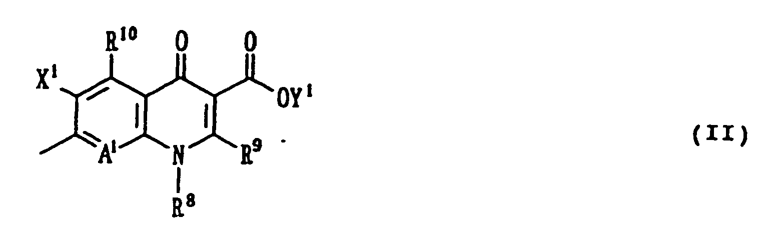 [wherein R⁸ represents a 2-halogenocyclopropyl group;
R⁹ represents a hydrogen atom or an alkylthio group having 1 to 6 carbon atoms,
or wherein R⁸ and R⁹ may be combined to form a cyclic structure including a part of the mother nucleus, and the ring may contain a sulfur atom as a ring constituting atom and may further have an alkyl group having 1 to 6 carbon atoms as a substituent;
X¹ represents a halogen atom or a hydrogen atom;
R¹⁰ represents a hydrogen atom, an amino group, a hydroxyl group, a thiol group, a halogenomethyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms,
wherein the amino group may have at least one substituent selected from the group consisting of a formyl group, an alkyl group having 1 to 6 carbon atoms and an acyl group having 2 to 5 carbon atoms;
A¹ represents a nitrogen atom, or a partial structure represented by formula (III): 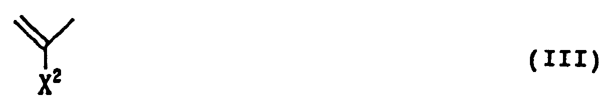 (wherein X² represents a hydrogen atom, an amino group, a halogen atom, a cyano group, a halogenomethyl group, a halogenomethoxyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms,
wherein the amino group may have at least one substituent selected from the group consisting of a formyl group, an alkyl group having 1 to 6 carbon atoms and an acyl group having 2 to 5 carbon atoms, and
X² and R⁸ may be combined to form a cyclic structure including a part of the mother nucleus, and the ring may contain an oxygen atom, a nitrogen atom or a sulfur atom as a ring constituting atom and may further have an alkyl group having 1 to 6 carbon atoms as a substituent); and
Y¹ represents a hydrogen atom, a phenyl group, an acetoxymethyl group, a pivaloyloxymethyl group, an ethoxycarbonyl group, a choline group, a dimethylaminoethyl group, a 5-indanyl group, a phthalidinyl group, a 5-alkyl-2-oxo-1,3-dioxol-4-ylmethyl group, a 3-acetoxy-2-oxobutyl group, an alkyl group having 1 to 6 carbon atoms, an alkoxymethyl group having 2 to 7 carbon atoms or a phenylalkyl group composed of an alkylene group having 1 to 6 carbon atoms and a phenyl group] or Q is a partial structure represented by formula (IV): 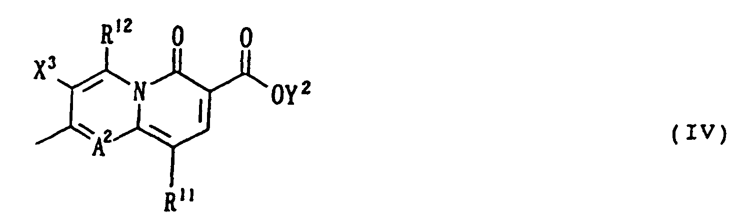 [wherein R¹¹ represents a 2-halogenocyclopropyl group
R¹² represents a hydrogen atom, an amino group, a hydroxyl group, a thiol group, a halogenomethyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms,
wherein the amino group may have at least one substituent selected from the group consisting of a formyl group, an alkyl group having 1 to 6 carbon atoms and an acyl group having 2 to 5 carbon atoms;
X³ represents a halogen atom or a hydrogen atom;
A² represents a nitrogen atom or a partial structure represented by formula (V): 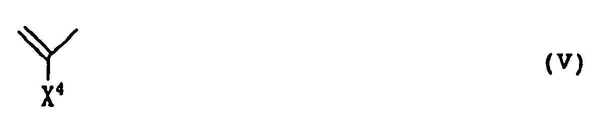 (wherein X⁴ represents a hydrogen atom, an amino group, a halogen atom, a cyano group, a halogenomethyl group, a halogenomethoxyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms,
wherein the amino group may have at least one substituent selected from the group consisting of a formyl group, an alkyl group having 1 to 6 carbon atoms and an acyl group having 2 to 5 carbon atoms, and
X⁴ and R¹¹ may be combined to form a cyclic structure including a part of the mother nucleus, and the ring may contain an oxygen atom, a nitrogen atom or a sulfur atom as a ring constituting atom and may further have an alkyl group having 1 to 6 carbon atoms as a substituent); and
Y² represents a hydrogen atom, a phenyl group, an acetoxymethyl group, a pivaloyloxymethyl group, an ethoxycarbonyl group, a choline group, a dimethylaminoethyl group, a 5-indanyl group, a phthalidinyl group, a 5-alkyl-2-oxo-1,3-dioxol-4-ylmethyl group, a 3-acetoxy-2-oxobutyl group, an alkyl group having 1 to 6 carbon atoms, an alkoxymethyl group having 2 to 7 carbon atoms or a phenylalkyl group composed of an alkylene group having 1 to 6 carbon atoms and a phenyl group]}, and its salts and hydrates thereof.

The present invention also relates to:
the aforementioned compound, its salts and hydrates thereof, wherein Q in the formula (I) has a structure represented by the formula (II);
the aforementioned compound, its salts and hydrates thereof, wherein the halogenocyclopropyl group in the formula (I) is a 1,2-cis-2-halogenocyclopropyl group;
the aforementioned compound, its salts and hydrates thereof, wherein the halogenocyclopropyl group in the formula (I) is a stereochemically pure substituent;
the aforementioned compound, its salts and hydrates thereof, wherein the halogenocyclopropyl group in the formula (I) is a (1R,2S)-2-halogenocyclopropyl group;
the aforementioned compound, its salts and hydrates thereof, wherein the halogen atom of the halogenocyclopropyl group in the formula (I) is a fluorine atom;
the aforementioned compound, its salts and hydrates thereof, wherein the compound of the formula (I) is a stereochemically pure compound;
the aforementioned compound, its salts and hydrates thereof, namely 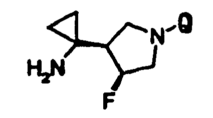
   wherein Q is a partial structure selected from the group consisting of: 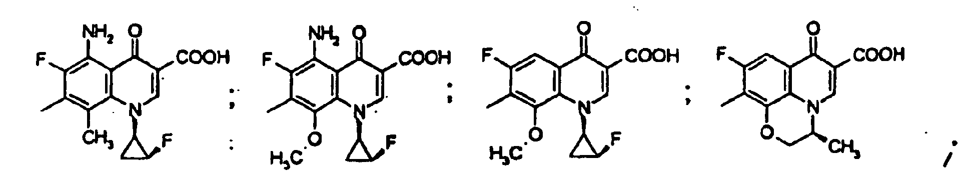
the aforementioned compound, its salts and hydrates thereof, namely 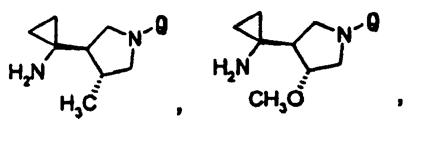 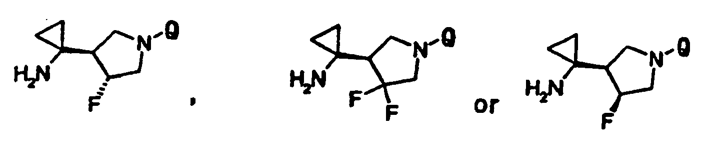 wherein Q is the partial structure: 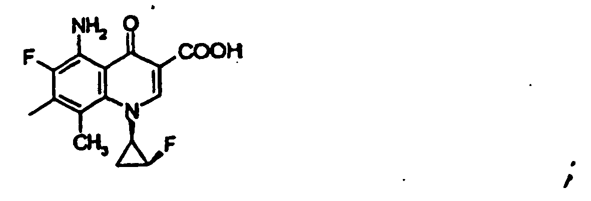
a pharmaceutical preparation which comprises the compound of formula (I) or its salt or a hydrate thereof as an active ingredient; and
an antibacterial drug or antibacterial preparation which comprises the compound of formula (I) or its salt or a hydrate thereof as an active ingredient.

Other objects and advantages of the present invention will be made apparent as the description progresses.

### DETAILED DESCRIPTION OF THE INVENTION

Substituents of the compound of the present invention represented by the formula (I) are described in the following.

The substituent R¹ represents a hydrogen atom or an alkyl group having 1 to 6 carbon atoms, and the alkyl group may be either straight- or branched-chain having 1 to 6 carbon atoms. Preferably, the alkyl group is methyl, ethyl, n-propyl or isopropyl group.

The substituent R² represents a hydrogen atom or an alkyl group having 1 to 6 carbon atoms, and the alkyl group may have at least one substituent selected from the group consisting of a hydroxyl group, a halogen atom, an alkylthio group having 1 to 6 carbon atoms and an alkoxyl group having 1 to 6 carbon atoms.

The alkyl group may be either straight- or branched-chain having 1 to 6 carbon atoms and is preferably methyl, ethyl, n-propyl or isopropyl group.

When the alkyl group has a hydroxyl group as a substituent, the alkyl group may be either straight- or branched-chain having 1 to 6 carbon atoms, and the hydroxyl group may most preferably be substituted on the terminal carbon atom of the alkyl group. Preferred examples of the alkyl group having a hydroxyl group are those which have up to 3 carbon atoms, such as hydroxymethyl, 2-hydroxyethyl, 2-hydroxypropyl, 3-hydroxypropyl and the like.

When the alkyl group has a halogen atom(s) as a substituent(s), the alkyl group may be either straight- or branched-chain having 1 to 6 carbon atoms, and fluorine atom is desirable as the halogen atom.

When the alkyl group has an alkylthio group as a substituent, the alkyl group may be either straight- or branched-chain having 1 to 6 carbon atoms, and the alkylthio group may also be either straight- or branched chain having 1 to 6 carbon atoms. Preferred examples of the alkyl group having an alkylthio group include an alkylthiomethyl group, an alkylthioethyl group and an alkylthiopropyl group, and the alkylthio group may preferably have up to 3 carbon atoms. Most preferred examples include methylthiomethyl, ethylthiomethyl and methylthioethyl groups.

When the alkyl group has an alkoxyl group as a substituent, the alkyl group may be either straight- or branched-chain having 1 to 6 carbon atoms, and the alkoxyl group may also be either straight- or branched chain having 1 to 6 carbon atoms. Preferred examples of the alkyl group having an alkoxyl group include an alkoxymethyl group, an alkoxyethyl group and an alkoxypropyl group, and the alkoxyl group may preferably have up to 3 carbon atoms. Most preferred examples include methoxymethyl, ethoxymethyl and methoxyethyl groups.

The substituent R³ represents a hydrogen atom, a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, and the alkyl group may have at least one substituent selected from the group consisting of a hydroxyl group, a halogen atom and an alkoxyl group having 1 to 6 carbon atoms.

As the halogen atom, fluorine or chlorine atom is preferable.

The alkyl group may be either straight- or branched-chain having 1 to 6 carbon atoms, and methyl, ethyl, n-propyl or isopropyl group is preferred.

The alkoxyl group may be either straight- or branched-chain having 1 to 6 carbon atoms, and methoxyl or ethoxyl group is preferred.

The alkylthio group may be either straight- or branched-chain having 1 to 6 carbon atoms, and methylthio or ethylthio group is preferred.

The alkyl group of 1 to 6 carbon atoms having a hydroxyl group may be either straight- or branched-chain, and the hydroxyl group may most preferably be substituted on the terminal carbon atom of the alkyl group. Preferred examples of the alkyl group of 1 to 6 carbon atoms substituted with a hydroxyl group include hydroxymethyl, 2-hydroxyethyl and 3-hydroxypropyl groups.

As the halogen atom of the alkyl group having a halogen atom, fluorine or chlorine atom is preferred, and fluorine atom is particularly preferred. The alkyl group may be either straight- or branched-chain.

In the alkyl group of 1 to 6 carbon atoms having an alkoxyl group, each alkyl moiety may be either straight- or branched-chain, and an alkoxymethyl group or an alkoxyethyl group is preferred. Its most preferred examples include methoxymethyl, ethoxymethyl and 2-methoxyethyl groups.

The substituent R⁴ represents a hydrogen atom, a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, and the alkyl moiety of these groups may have at least one substituent selected from the group consisting of a hydroxyl group, a halogen atom and an alkoxyl group having 1 to 6 carbon atoms, and the substituent R⁵ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms.

In addition, R⁴ and R⁵ may be combined together to form a hydroxyimino group, a methylene chain having 3 to 6 carbon atoms (so as to form a spiro cyclic structure together with the pyrrolidine ring) or an alkyloxyimino group having 1 to 6 carbon atoms.

As the halogen atom, fluorine or chlorine atom is desirable.

The alkyl group may be either straight- or branched-chain having 1 to 6 carbon atoms, and methyl, ethyl, n-propyl or isopropyl group is preferred.

The alkoxyl group may be either straight- or branched-chain having 1 to 6 carbon atoms, and methoxyl or ethoxyl group is preferred.

The alkylthio group may be either straight- or branched-chain having 1 to 6 carbon atoms, and methylthio or ethylthio group is preferred.

The alkyl group of 1 to 6 carbon atoms having a hydroxyl group may be either straight- or branched-chain, and the hydroxyl group may most preferably be substituted on the terminal carbon atom of the alkyl group. Preferred examples of the alkyl group of 1 to 6 carbon atoms substituted with hydroxyl group include hydroxymethyl, 2-hydroxyethyl and 3-hydroxypropyl groups.

As the halogen atom of the alkyl group having a halogen atom, fluorine or chlorine atom is preferred, and fluorine atom is particularly preferred. The alkyl group may be either straight- or branched-chain.

In the alkyl group of 1 to 6 carbon atoms having an alkoxyl group, each alkyl moiety may be either straight- or branched-chain, and an alkoxymethyl group or an alkoxyethyl group is preferred. Its most preferred examples include methoxymethyl, ethoxymethyl and 2-methoxyethyl groups.

When the substituents R⁴ and R⁵ are combined to form a methylene chain, a three- to six-membered ring is newly formed, thereby forming a spiro cyclic structure together with the pyrrolidine ring. As the newly formed ring, a cyclopropyl or cyclobutyl ring having a size of 2 or 3 carbon atoms as the methylene chain is desirable.

Also, when R⁴ and R⁵ are combined to form an alkyloxyimino group, =N-O-Alkyl, the alkyl group may be either straight- or branched-chain. As the alkyloxyimino group, methoxyimino or ethoxyimino group is preferred.

The substituents R⁶ and R⁷, each independently represents a hydrogen atom or an alkyl group having 1 to 6 carbon atoms, and the alkyl group may be either straight- or branched-chain having 1 to 6 carbon atoms and is preferably methyl, ethyl, n-propyl or isopropyl group.

The character n is an integer of 1 to 3, and the corresponding ring may be a cyclopropane to cyclobutane ring. The compound of the present invention is characterized in that this moiety is a cyclic structure. As the n, 1 is particularly preferred.

Q is a partial structure of a fused heterocyclic system represented by the following formula (II) or (IV).

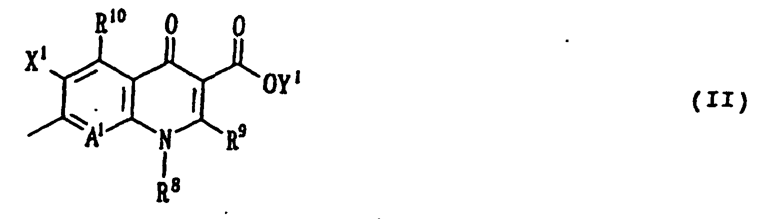

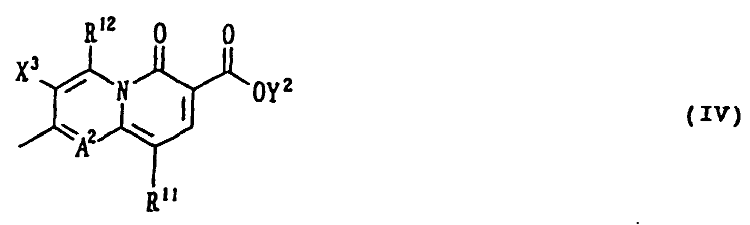

The substituents R⁸ and R¹¹, each independently represents a 2-halogenocyclopropyl group.

A fluorine atom is particularly preferable as the halogen atom of the 2-halogenocyclopropyl group.

The substituent R⁹ represents a hydrogen atom or an alkylthio group having 1 to 6 carbon atoms, or R⁸ and R⁹ may be combined to form a cyclic structure including a part of the mother nucleus (i.e., including the nitrogen atom to which R⁸ is attached and the carbon atom to which R⁹ is attached). The thus formed ring may contain a sulfur atom as a ring constituting atom and may further have an alkyl group having 1 to 6 carbon atoms as a substituent. The thus formed ring may have a ring size of from four-membered to six-membered and it may be saturated, partially saturated or unsaturated.

The substituents X¹ and X³, each independently represents a halogen atom or a hydrogen atom, and fluorine atom is preferable in the case of the halogen atom. Of these, fluorine or hydrogen atom is preferable as the substituent.

The substituents R¹⁰ and R¹², each independently represents a hydrogen atom, an amino group, a hydroxyl group, a thiol group, a halogenomethyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms, wherein the amino group may have at least one substituent selected from the group consisting of a formyl group, an alkyl group having 1 to 6 carbon atoms and an acyl group having 2 to 5 carbon atoms.

The alkyl group may be either straight- or branched-chain having 1 to 6 carbon atoms and is preferably methyl, ethyl, n-propyl or isopropyl group. The alkenyl group may be either straight- or branched-chain having 2 to 6 carbon atoms and is preferably a vinyl group. The alkynyl group may be either straight- or branched-chain having 2 to 6 carbon atoms and is preferably an ethynyl group. As the halogen of the halogenomethyl group, fluorine is particularly preferred, and its number may be 1 to 3. The alkoxyl group may have 1 to 6 carbon atoms and is preferably methoxyl group.

As the substituents R¹⁰ and R¹², an alkyl group or an amino group is preferred, and a methyl group or an unsubstituted amino group is particularly preferred.

When the substituent R¹⁰ or R¹² is an amino group, a hydroxyl group or a thiol group, these groups may be protected by usually used protective groups.

Illustrative examples of such protective groups include alkoxycarbonyl groups such as tert-butoxycarbonyl, 2,2,2-trichloroethoxycarbonyl and the like, aralkyloxycarbonyl groups such as benzyloxycarbonyl, p-methoxybenzyloxycarbonyl, p-nitrobenzyloxycarbonyl and the like, acyl groups such as acetyl, methoxyacetyl, trifluoroacetyl, chloroacetyl, pivaloyl, formyl, benzoyl and the like, alkyl or aralkyl groups such as tert-butyl, benzyl, p-nitrobenzyl, p-methoxybenzyl, triphenylmethyl and the like, ethers such as methoxymethyl, tert-butoxymethyl, tetrahydropyranyl, 2,2,2-trichloroethoxymethyl and the like, and silyl groups such as trimethylsilyl, isopropyldimethylsilyl, tert-butyldimethylsilyl, tribenzylsilyl, tert-butyldiphenylsilyl and the like. Compounds having substituents which are protected by these protective groups are preferable particularly as production intermediates.

When A¹ is a partial structure represented by formula (III): 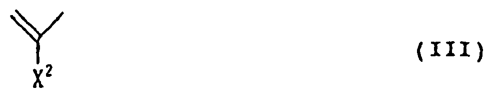 X² is a hydrogen atom, an amino group, a halogen atom, a cyano group, a halogenomethyl group, a halogenomethoxyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms. The amino group may have at least one substituent selected from the group consisting of a formyl group, an alkyl group having 1 to 6 carbon atoms and an acyl group having 2 to 5 carbon atoms.

The alkyl group may be either straight- or branched-chain having 1 to 6 carbon atoms and is preferably methyl or ethyl group. The alkenyl group may be either straight- or branched-chain having 2 to 6 carbon atoms and is preferably vinyl group. The alkynyl group may be either straight- or branched-chain having 2 to 6 carbon atoms and is preferably ethynyl group. As the halogen of the halogenomethyl group, fluorine is particularly preferred, and its number may be 1 to 3. The alkoxyl group may have 1 to 6 carbon atoms and is preferably methoxyl group. As the halogen of the halogenomethoxyl group, fluorine is particularly preferred, and its number may be 1 to 3.

Of these substituents, an alkyl group or an alkoxyl group is preferred. Most preferred is methyl or methoxyl group.

In addition, X² and R⁸ may be combined to form a cyclic structure (the ring may have a ring size of from four-membered to seven-membered and it may be saturated, partially saturated or unsaturated) including a part of the mother nucleus (i.e., including the nitrogen atom to which R⁸ is attached and the carbon atom to which X² is attached). The thus formed ring may contain oxygen, nitrogen or sulfur atom as a ring constituting atom and may further have an alkyl group having 1 to 6 carbon atoms as a substituent.

When A² is a partial structure represented by formula (V): 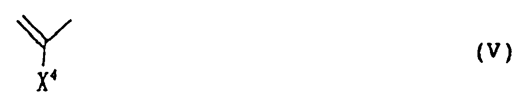 X⁴ is a hydrogen atom, an amino group, a halogen atom, cyano group, a halogenomethyl group, a halogenomethoxyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms. The amino group may have at least one substituent selected from the group consisting of a formyl group, an alkyl group having 1 to 6 carbon atoms and an acyl group having 2 to 5 carbon atoms.

The alkyl group may be either straight- or branched-chain having 1 to 6 carbon atoms and is preferably methyl or ethyl group. The alkenyl group may be either straight- or branched-chain having 2 to 6 carbon atoms and is preferably vinyl group. The alkynyl group may be either straight- or branched-chain having 2 to 6 carbon atoms and is preferably ethynyl group. As the halogen of the halogenomethyl group, fluorine is particularly preferred, and its number may be 1 to 3. The alkoxyl group may have 1 to 6 carbon atoms and is preferably methoxyl group. As the halogen of the halogenomethoxyl group, fluorine is particularly preferred, and its number may be 1 to 3.

In addition, X⁴ and R¹¹ may be combined to form a cyclic structure (the ring may have a ring size of from four-membered to seven-membered and it may be saturated, partially saturated or unsaturated) including a part of the mother nucleus (i.e., including the nitrogen atom to which R¹¹ is attached and the carbon atom to which X⁴ is attached). The thus formed ring may contain oxygen, nitrogen or sulfur atom as a constituent and may further have an alkyl group having 1 to 6 carbon atoms as a substituent.

When A¹ is a partial structure represented by formula (III): 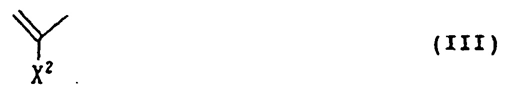 a preferred combination of R¹⁰ and X² is that R¹⁰ is an amino group, a hydrogen atom, a hydroxyl group or an alkyl group having 1 to 6 carbon atoms and X² is an alkyl group having 1 to 6 carbon atoms, an alkoxyl group having 1 to 6 carbon atoms, a halogen atom, a halogenomethoxyl group or a hydrogen atom.

In a more preferred combination, R¹⁰ is amino group, hydrogen atom, hydroxyl group or methyl group and X² is methyl group, methoxyl group, fluorine atom, chlorine atom, difluoromethoxyl group or hydrogen atom.

In a most preferred combination, R¹⁰ is amino group, hydrogen atom, hydroxyl group or methyl group and X² is methyl group or methoxyl group.

For these R¹⁰ and X², X¹ is preferably fluorine atom.

When the substituents X¹ and X², each is independently a halogen atom, fluorine atom is particularly preferred as X¹ and fluorine or chlorine atom is desirable as X².

When A² is a partial structure represented by formula (V): 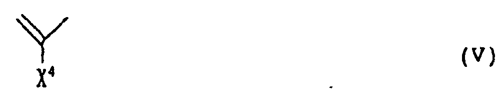 a preferred combination of R¹² and X⁴ is that R¹² is an amino group, a hydrogen atom, a hydroxyl group or an alkyl group having 1 to 6 carbon atoms and X⁴ is an alkyl group having 1 to 6 carbon atoms, an alkoxyl group having 1 to 6 carbon atoms, a halogen atom, a halogenomethoxyl group or a hydrogen atom.

In a more preferred combination, R¹² is amino group, hydrogen atom, hydroxyl group or methyl group and X⁴ is methyl group, methoxyl group, fluorine atom, chlorine atom, difluoromethoxyl group or hydrogen atom.

In a most preferred combination, R¹² is amino group, hydrogen atom, hydroxyl group or methyl group and X⁴ is methyl group or methoxyl group.

When the substituents X³ and X⁴, each is independently a halogen atom, fluorine atom is particularly preferred as X³ and fluorine or chlorine atom is desirable as X⁴.

Next, the halogenocyclopropyl group of R⁸ is described.

As for the halogen atom, a fluorine atom and a chlorine atom can be exemplified, of which fluorine atom is particularly preferred.

With regard to the stereochemical environment of this moiety, it is particularly preferred that the halogen atom and the pyridonecarboxylic acid moiety take cis-configuration in respect of the cyclopropane ring.

Enantiomerical isomers can exist solely due to this cis-2-halogenocyclopropyl moiety of R⁸, and strong antibacterial activity and high safety have been confirmed in both of these isomers.

When the compound of formula (I) of the present invention has a structure in which diastereomers exist, it is desirable to administer the inventive compound to human and animals as a compound comprised of a single diastereomer. The term "comprised of a single diastereomer" as used herein means not only the case in which the other diastereomer is entirely absent but also a case in which the compound is in a chemically pure form. In other words, the other diastereomer may be contained in such an amount that it does not exert influences upon physical constants and physiological activities.

Also, the term "stereochemically pure" as used herein means that, when a compound exists in a plurality of isomer forms due to the presence of asymmetric carbon atoms, the compound is composed of only one of these isomers. The term "pure" of this case can also be understood in the same manner as the aforementioned case.

The pyridonecarboxylic acid derivative of the present invention may be used as its free form or as an acid addition salt or a salt of carboxyl group. Examples of the acid addition salt include inorganic acid salts such as hydrochloride, sulfate, nitrate, hydrobromide, hydroiodide, phosphate and the like and organic acid salts such as acetate, methanesulfonate, benzenesulfonate, toluenesulfonate, citrate, maleate, fumarate, lactate and the like.

The salt of carboxyl group may be either an inorganic or organic salt, which includes lithium salt, sodium salt, potassium salt and the like alkali metal salts, magnesium salt, calcium salt and the like alkaline earth metal salts, ammonium salt, triethylamine salt, N-methylglucamine salt, tris-(hydroxymethyl)aminomethane salt and the like.

Also, these free form, acid addition salts and carboxyl group salts of the pyridonecarboxylic acid derivative may exist as hydrates.

On the other hand, the quinolone derivative whose carboxylic acid moiety is an ester is useful as a synthetic intermediate or prodrug. For example, alkyl esters, benzyl esters, alkoxyalkyl esters, phenylalkyl esters and phenyl esters are useful as synthetic intermediates.

The esters to be used as prodrugs are those which are easily cleaved in the living body to give free form of carboxylic acid, and their illustrative examples include acetoxymethyl ester, pivaloyloxymethyl ester, ethoxycarbonyl ester, choline ester, dimethylaminoethyl ester, 5-indanyl ester and oxoalkyl esters such as phthalidinyl ester, 5-alkyl-2-oxo-1,3-dioxol-4-ylmethyl ester, 3-acetoxy-2-oxobutyl ester and the like.

The compound of the present invention represented by the formula (I) can be produced by various methods, for example by a preferred method in which a compound represented by formula (VI): 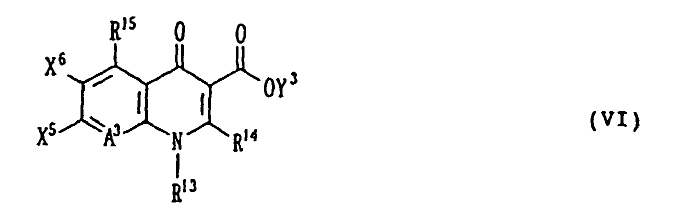 [wherein X⁵ is a substituent which serves as a leaving group, such as a fluorine atom, a chlorine atom, a bromine atom, a substituted or unsubstituted phenylsulfonyl group or a substituted or unsubstituted alkylsulfonyl group having 1 to 3 carbon atoms;
Y³ is the Y¹ as defined in the formula (II) or a group represented by formula (VII): 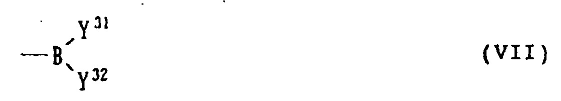 (wherein each of Y³¹ and Y³² is a fluorine atom or an alkylcarbonyloxy group having 2 to 5 carbon atoms), and R¹³, R¹⁴, R¹⁵, A³ and X⁶ are the same groups corresponding to R⁸, R⁹, R¹⁰, A¹ and X¹ as defined in the formula (II)] or a compound represented by formula (VIII): 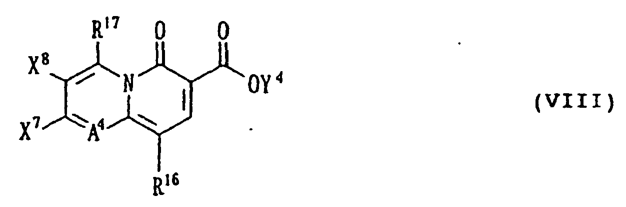 [wherein X⁷ is a substituent which serves as a leaving group, such as a fluorine atom, a chlorine atom, a bromine atom, a substituted or unsubstituted phenylsulfonyl group or a substituted or unsubstituted alkylsulfonyl group having 1 to 3 carbon atoms, and R¹⁶, R¹⁷, A⁴, X⁸ and Y⁴ are the same groups corresponding to R¹¹, R¹², A², X³ and Y² as defined in the formula (IV)] is allowed to react with a compound represented by formula (IX): 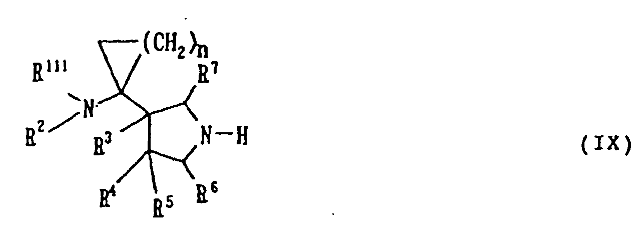 (wherein R¹¹¹ is the R¹ defined in the formula (I) or a protective group of the amino group and R², R³, R⁴, R⁵, R⁶, R⁷ and n are as defined in the formula (I)] or with an acid addition salt thereof.

The reaction can be carried out with or without a solvent. Examples of the solvent to be used in the reaction include those which are inert under the reaction conditions, such as dimethyl sulfoxide, pyridine, acetonitrile, ethanol, chloroform, dimethylformamide, dimethylacetamide, N-methylpyrrolidone, tetrahydrofuran, water, 3-methoxybutanol and mixtures thereof.

It is preferable to carry out the reaction in the presence of an inorganic base, an organic base or the like acid receptor, such as an alkali metal or alkaline earth metal carbonate or bicarbonate, or triethylamine, pyridine, 1,8-diazabicycloundecene or the like.

The reaction temperature may be generally within the range of from room temperature to 200°C, but preferably within the range of approximately from 25 to 150°C. The reaction time may be 15 minutes to 48 hours, and the reaction completes generally within 30 minutes to 2 hours.

Illustrative examples of the amino group-protecting group are those which are generally used in this field, which include alkoxycarbonyl groups such as tert-butoxycarbonyl, 2,2,2-trichloroethoxycarbonyl and the like, aralkyloxycarbonyl groups such as benzyloxycarbonyl, p-methoxybenzyloxycarbonyl, p-nitrobenzyloxycarbonyl and the like, acyl groups such as acetyl, methoxyacetyl, trifluoroacetyl, chloroacetyl, pivaloyl, formyl, benzoyl and the like, alkyl or aralkyl groups such as tert-butyl, benzyl, p-nitrobenzyl, p-methoxybenzyl, triphenylmethyl and the like, ethers such as methoxymethyl, tert-butoxymethyl, tetrahydropyranyl, 2,2,2-trichloroethoxymethyl and the like, and silyl groups such as trimethylsilyl, isopropyldimethylsilyl, tert-butyldimethylsilyl, tribenzylsilyl, tert-butyldiphenylsilyl and the like.

When Y³ and Y⁴ are each an alkyl group having 1 to 6 carbon atoms, an alkoxymethyl group having 2 to 7 carbon atoms or a phenylalkyl group which is composed of an alkylene group having 1 to 6 carbon atoms and phenyl group, conversion into corresponding carboxylic acid can be effected by a treatment under acidic or basic conditions generally used for the hydrolysis of carboxylic acid esters.

When Y³ has a structure represented by formula (VII): 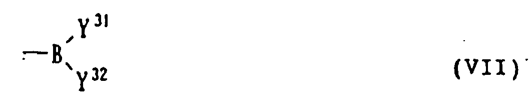 conversion into corresponding carboxylic acid can be effected by carrying out reaction of the compound (VI) with the compound (IX) and then treating under acidic or basic conditions.

In addition, when deprotection is required, the compound of interest represented by the formula (I) can be obtained by removing protective groups under appropriate procedure known in this field corresponding to the protective groups used.

Further, the present invention also relates to the compounds represented by formula (IX) 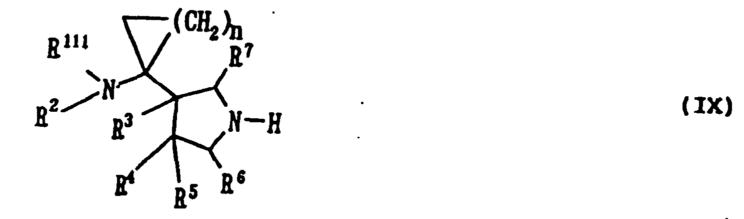 (wherein R¹¹¹ represents a hydrogen atom, an alkyl group having 1 to 6 carbon atoms or a protective group of the amino group; R² represents a hydrogen atom or an alkyl group having 1 to 6 carbon atoms,
wherein the alkyl group may have at least one substituent selected from the group consisting of a hydroxyl group, a halogen atom, an alkylthio group having 1 to 6 carbon atoms and an alkoxyl group having 1 to 6 carbon atoms; R³ represents a hydrogen atom, a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms,
wherein the alkyl group may have at least one substituent selected from the group consisting of a hydroxyl group, a halogen atom and an alkoxyl group having 1 to 6 carbon atoms;
R⁴ represents a hydrogen atom, a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, and R⁵ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms,
wherein the alkyl group may have at least one substituent selected from the group consisting of a hydroxyl group, a halogen atom and an alkoxyl group having 1 to 6 carbon atoms, and
R⁴ and R⁵ may be combined to form a hydroxyimino group, a methylene chain having 3 to 6 carbon atoms (so as to form a spiro cyclic structure together with the pyrrolidine ring) or an alkyloxyimino group having 1 to 6 carbon atoms ; R⁶ and R⁷, each independently represents a hydrogen atom or an alkyl group having 1 to 6 carbon atoms; and n is an integer of 1 to 3]; or an acid addition salt thereof.

In particular, the present invention relates to the aforementioned compounds and acid addition salts thereof, namely 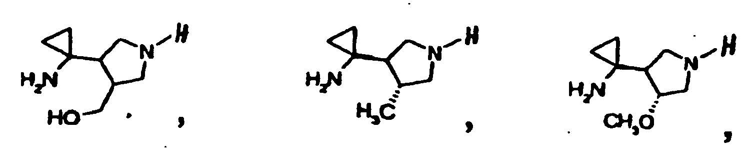 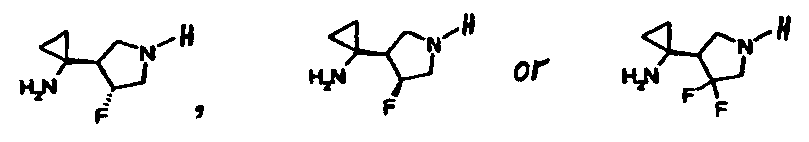 wherein the amino group may be protected, for instance with one of the abovementioned amino group-protecting groups.

Since the compound of the present invention is possessed of strong antibacterial activities, it finds versatile use in such application as pharmaceutical preparations for human, animals and fishes or as agricultural chemicals and food preservatives.

When the compound of the present invention is used as a pharmaceutical preparation in human, its dose may be within the range of from 50 mg to 1 g, preferably from 100 mg to 300 mg, per day per adult.

When used in animals, its dose varies depending on the object of administration (healing or prevention for example), species and size of each animal to be treated, species of the infected pathogenic bacterium and degree of the infection, but its daily dose may be within the range of generally from 1 mg to 200 mg, preferably from 5 mg to 100 mg, per 1 kg body weight.

The daily dose may be administered once a day or by dividing it into 2 to 4 doses. If necessary, the daily dose may be increased by exceeding the above range.

Since the compound of the present invention is active upon a broad range of microorganisms which cause various infectious diseases, it is effective in treating, preventing or alleviating diseases caused by these pathogens.

Examples of bacteria or bacteria-like microorganisms to be treated by the compound of the present invention include the genus *Staphylococcus, Streptococcus pyogenes*, hemolytic streptococci, enterococcus, pneumococcus, the genus *Peptostreptococcus, Neisseria gonorrhoeae, Escherichia coli,* the genus *Citrobacter,* the genus *Shigella, Klebsiella pneumoniae,* the genus *Enterobacter,* the genus *Serratia,* the genus *Proteus, Pseudomonas aeruginosa, Haemophilus influenzae,* the genus *Acinetobacter,* the genus *Campylobacter, Chlamydia trachomatis* and the like.

Examples of diseases induced by these pathogens include folliculitis, furuncle, carbuncle, erysipelas, phlegmon, lymphangitis (lymphadenitis), felon, subcutaneous abscess, hidradenitis, acne conglobata, infectious atheroma, perirectal asscess, mastitis, superficial secondary infections such as of injury, burn injury, operative wound and the like, pharyngitis, laryngitis, acute bronchitis, tonsilitis, chronic bronchitis, bronchiectasis, diffuse bronchiolitis, secondary infection of chronic respiratory disease, pneumonia, pyelonephritis, cystitis, prostatitis, epididymitis, gonococcal urethritis, nonspecific urethritis, cholecystitis, cholangitis, bacillary dystentery, enteritis, uterine adnexitis, intrauterine infection, bartholinitis, blepharitis, hordeolum, dacryocystitis, tarsadenitis, corneal ulcer, otitis media, sinusitis, periodontitis, pericoronitis, jaw inflammation, peritonitis, endocarditis, sepsis, meningitis, skin infection and the like.

The compound of the present invention is also effective against various microorganisms which cause infectious diseases of animals, such as the genera *Escherichia, Salmonella, Pasteurella, Haemophilus, Bordetella, Staphylococcus, Mycoplasma* and the like. Illustrative examples of such diseases include colibacillosis, pullorum, avian paratyphoid, avian cholera, infectious coryza, staphylococcosis, *Mycoplasma* infection and the like in the case of birds, colibacillosis, salmonellosis, pasteurellosis, *Haemophilus* infection, atrophic rhinitis, exudative epidermitis, *Mycoplasma* infection and the like in the case of pigs, colibacillosis, salmonellosis, hemorrhagic sepsis, *Mycoplasma* infection, bovine pleuropneumonia, mastitis and the like in the case of cattle, colisepsis, *Salmonella* infection, hemorrhagic sepsis, uterine empyema, cystitis and the like in the case of dogs, and exudative pleurisy, cystitis, chronic rhinitis, *Haemophilus* infection, kitten diarrhea, *Mycoplasma* infection and the like in the case of cats.

The antibacterial preparation which is comprised of the compound of the present invention can be prepared by selecting an appropriate dosage form corresponding to each administration method and making use of various commonly used medicine preparation methods. Examples of the dosage form of the antibacterial preparation which contains the compound of the present invention as its principal agent include oral preparations such as tablets, powders, granules, capsules, solutions, syrups, elixirs, oily or aqueous suspensions and the like.

When used as injections, stabilizing agents, antiseptics, solubilizing agents and the like may be used in the preparations, and the solution which may contain these auxiliary agents may be packed in containers and freeze-dried to make it into a solid preparation which is re-dissolved prior to its use. Also, one dose may be packed in one container or multiple doses may be put in the same container.

As external preparations, solutions, suspensions, emulsions, ointments, gels, creams, lotions, sprays and the like can be exemplified.

Solid preparations can be prepared by mixing the active compound with pharmaceutically acceptable additive agents optionally selected from fillers and extenders, binders, disintegrators, solubilizing agents, moistening agents, lubricating agents and the like.

Examples of liquid preparations include solutions, suspensions, emulsions and the like which may contain suspending agents, emulsifying agents and the like additives.

Administration of the compound of the present invention to animals may be effected by a method in which the compound is orally administered directly or after mixing it with feed, a method in which the compound is made into a solution and then orally administered directly or by adding the solution to drinking water or feed or a method in which the compound is administered by injection.

With regard to pharmaceutical preparations of the compound of the present invention for use in their administration to animals, the compound may be optionally made into powders, fine subtilaes, soluble powders, syrups, solutions or injections making use of the techniques conventionally used in this field.

The following shows formulation examples of the pharmaceutical preparations.

### Formulation Example 1 [capsules]:

| | |
|---|---|
| Compound of Inventive Example 3 | 100.0 mg |
| Corn starch | 23.0 mg |
| CMC calcium | 22.5 mg |
| Hydroxymethylcellulose | 3.0 mg |
| Magnesium stearate | 1.5 mg |
| Total | 150.0 mg |

### Formulation Example 2 [solutions]:

| | |
|---|---|
| Compound of Inventive Example 5 | 1 - 10 g |
| Acetic acid or sodium hydroxide | 0.5 - 2 g |
| Ethyl paraoxybenzoate | 0.1 g |
| Purified water | 88.9 - 98.4 g |
| Total | 100 g |

### Formulation Example 3 [powders for mixing with feed]:

| | |
|---|---|
| Compound of Inventive Example 7 | 1 - 10 g |
| Corn starch | 98.5 - 89.5 g |
| Soft silicic anhydride | 0.5 g |
| Total | 100 g |

### BEST MODE FOR CARRYING OUT INVENTION

Examples of the present invention are given below by way of illustration and not by way of limitation. In this connection, antibacterial activity of each compound of interest was measured in accordance with the standard method designated by Japan Society of Chemotherapy. The results are shown in Tables 1 to 3 as minimum inhibitory concentration (MIC, µg/ml).

### [Reference Example 1-1]

### (E)-Ethyl 3-(1-tert-butoxycarbonylaminocyclopropyl)propenoate

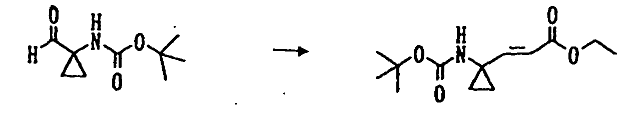

1-Tert-butoxycarbonylaminocyclopropane carbaldehyde (10.99 g, 59.3 mmol) and (carbethoxymethylene) triphenylphosphorane (27.6 g, 75.2 mmol) were dissolved in dichloromethane (300 ml) and heated under reflux for 4 hours. After evaporation of the solvent, the resulting residue was applied to a silica gel column chromatography and eluted with an eluant of n-hexane:ethyl acetate = 9:1, thereby obtaining 9.23 g (61%) of the title compound.
¹H-NMR (400 MHz, CDCl₃) δ: 6.48 (1 H, d, J = 15.62 Hz), 5.84 (1 H, d, J = 15.62 Hz), 5.00 (1 H, brs), 4.18 (2 H, q, J = 7.33 Hz), 1.45 (9 H, s), 1.28 (3 H, t, J = 7.33 Hz), 1.28 (2 H, brs), 1.16 (2 H, brs).

### [Reference Example 1-2]

### Ethyl trans-1-benzyl-4-(1-tert-butoxycarbonylaminocyclopropyl)pyrrolidine-3-carboxylate

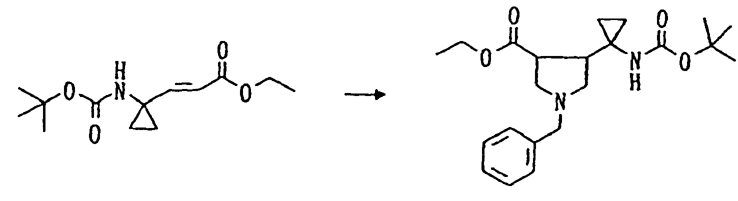

(E)-Ethyl 3-(1-tert-butoxycarbonylaminocyclopropyl)-propenoate (2.91 g, 11.38 mmol) and N-benzyl-N-(n-butoxymethyl) trimethylsilylmethylamine (7.43 g, 26.59 mmol) were dissolved in dichloromethane (40 ml) and, under an atmosphere of nitrogen, the solution was mixed with 1 M dichloromethane solution of trifluoroacetic acid (2.66 ml, 2.66 mmol) and stirred at room temperature for 3 hours. After completion of the reaction, the reaction solution was washed with saturated sodium bicarbonate aqueous solution and saturated sodium chloride aqueous solution in that order and dried over anhydrous sodium sulfate. After evaporation of the solvent, the resulting residue was applied to a silica gel column chromatography and eluted with an eluant of n-hexane:ethyl acetate = 2:1, and the resulting oily material was crystallized from chloroform-n-hexane to obtain 3.06 g (69%) of the title compound as white needle crystals.
¹H-NMR (400 MHz, CDCl₃) δ: 7.34 - 7.21 (5 H, m), 5.14 (1 H, brs), 4.13 (2 H, q, J = 7.33 Hz), 3.60 and 3.56 (2 H, ABd, J = 13.19 Hz), 3.21 - 3.11 (1 H, m), 2.87 - 2.76 (1 H, m), 2.75 - 2.64 (1 H, m), 2.55 - 2.45 (1 H, m), 2.43 - 2.33 (1 H, m), 1.43 (9 H, s), 1.25 (2 H, t, J = 7.33 Hz), 0.98 - 0.88 (1 H, m), 0.86 - 0.73 (2 H, m), 0.72 - 0.63 (1 H, m).

### [Reference Example 1-3]

### Trans-1-benzyl-4-(1-tert-butoxycarbonylaminocyclopropyl)-3-hydroxymethylpyrrolidine

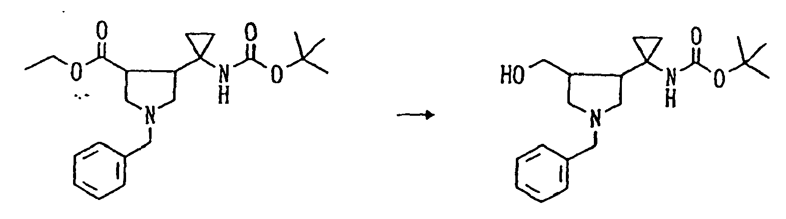

Under an atmosphere of nitrogen, lithium aluminum hydride (381 mg, 9.54 mmol) was suspended in anhydrous tetrahydrofuran (30 ml) to which, while cooling in an ice bath, was subsequently added dropwise anhydrous tetrahydrofuran (10 ml) solution of ethyl trans-1-benzyl-4-(1-tert-butoxycarbonylaminocyclopropyl)pyrrolidine-3-carboxylate (1.24 g, 3.18 mmol) in 15 minutes. After 4 hours of stirring at the same temperature, ice-cooled water was gradually added to the reaction solution. The reaction suspension was subjected to celite filtration (chloroform washing) to separate the organic layer. The aqueous layer was extracted with chloroform (50 ml x 2), and the organic layers were combined and dried over anhydrous sodium sulfate. By evaporating the solvent, 1.12 g (>99%) of the title compound was obtained.
¹H-NMR (400 MHz, CDCl₃) δ: 7.34 - 7.23 (5 H, m), 5.01 (1 H, brs), 3.61 (2 H, brs), 3.59 (2 H, s), 2.95 - 2.87 (1 H, m), 2.63 - 2.49 (2 H, m), 2.37 - 2.27 (1 H, m), 1.98 - 1.88 (1 H, m), 1.43 (9 H, s), 1.25 (2 H, t, J = 7.33 Hz), 0.94 - 0.84 (1 H, m), 0.84 - 0.70 (2 H, m), 0.70 - 0.62 (1 H, m).

### [Reference Example 1]

### 5-Amino-7-[trans-4-(1-aminocyclopropyl)-3-hydroxymethyl-1-pyrrolidinyl]-1-cyclopropyl-6-fluoro-1,4-dihydro-8-methyl-4-oxoquinoline-3-carboxylic acid

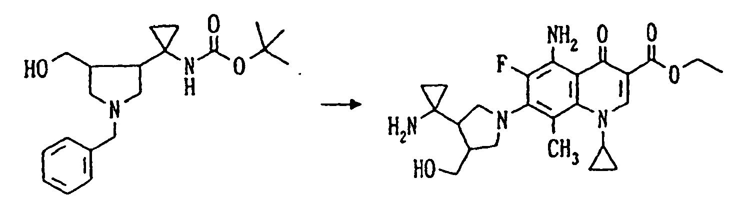

Trans-1-benzyl-4-(1-tert-butoxycarbonylaminocyclopropyl)-3-hydroxymethylpyrrolidine (1.10 g, 3.18 mmol) was dissolved in ethanol (50 ml), and the solution was mixed with palladium hydroxide (500 mg) and stirred for 1.5 hours at room temperature under an atmosphere of hydrogen. The reaction suspension was subjected to celite filtration (ethanol washing) and the solvent was evaporated. The resulting residue and 5-amino-1-cyclopropyl-6,7-difluoro-1,4-dihydro-8-methyl-4-oxoquinoline-3-carboxylic acid (471 mg, 1.60 mmol) were dissolved in dimethyl sulfoxide (20 ml) and, under an atmosphere of nitrogen, the solution was mixed with triethylamine (5 ml) and stirred at 150°C for 19 hours. After evaporation of the solvent, the resulting residue was mixed with 10% citric acid aqueous solution (50 ml) and extracted with chloroform (50 ml x 2), and the extract was dried over anhydrous sodium sulfate. After evaporation of the solvent, the resulting residue was mixed with concentrated hydrochloric acid (5 ml) and stirred for 1 hour. This was mixed with water (50 ml) and washed with chloroform (50 ml x 2). The aqueous layer was adjusted to pH 12.00 with sodium hydroxide aqueous solution and washed with chloroform (50 ml x 2). Finally, the aqueous layer was adjusted to pH 7.40 with 1 N hydrochloric acid and extracted with chloroform (300 ml x 5). The extract was dried over anhydrous sodium sulfate, the solvent was evaporated and then the resulting residue was recrystallized from ethanol to obtain 165 mg (38%) of the title compound.
Melting point: 179 - 182°C
¹H-NMR (400 MHZ, CDCl₃) δ: 8.40 (1 H, s), 4.06 - 3.97 (1 H, m), 3.85 - 3.79 (1 H, m), 3.68 - 3.48 (4 H, m), 3.47 - 3.39 (1 H, m ), 2.50 - 2.40 (1 H, m), 2.42 (3 H, s), 1.79 - 1.70 (1 H, m), 1.17 - 1.03 (2 H, m), 0.82 - 0.67 (2 H, m), 0.67 - 0.46 (4 H, m).

**Elemental analysis data; for C₂₂H₂₇FN₄O₄**

| | |
|---|---|
| calcd.; | C, 61.38; H, 6.32; N, 13.01 |
| found ; | C, 61.15; H, 6.31; N, 12.78 |

### [Reference Example 2-1]

### Ethyl 3-(1-tert-butoxycarbonylaminocyclopropyl)propiolate

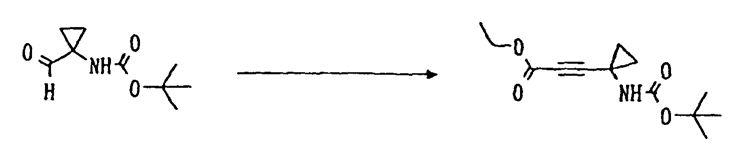

Under an atmosphere of nitrogen, chloromethyltrimethylphosphonium chloride (5.156 g, 14.85 mmol) was suspended in anhydrous tetrahydrofuran (30 ml) to which, after cooling to -55°C, was subsequently added dropwise 1.68 M n-butyl lithium n-hexane solution (8.87 ml, 14.90 mmol) in 5 minutes. The reaction suspension was stirred for 30 minutes in an ice bath and for 3 hours at room temperature and then cooled to -55°C. To the reaction suspension was added dropwise anhydrous tetrahydrofuran (10 ml) solution of 1-tert-butoxycarbonylaminocyclopropane carbaldehyde (2.498 g, 13.50 mmol) in 10 minutes, subsequently stirring the mixture for 1 hour at -50°C and then for 30 minutes in an ice bath. The reaction suspension was cooled to -78°C, n-hexane solution of 1.68 M n-butyl lithium (17.68 ml, 29.70 mmol) was added dropwise thereto in 10 minutes and then the mixture was stirred at -78°C for 20 minutes. Ethyl chloroformate (1.61 ml, 16.88 mmol) was added dropwise to the reaction suspension which was subsequently stirred for 1.5 hours at -78°C and then for 1 hour in an ice bath. While cooling in an ice bath, the reaction suspension was mixed with saturated brine (30 ml), the organic layer was separated and the aqueous layer was extracted with diethyl ether (30 ml x 2). The organic layers were combined, washed with saturated brine (30 ml) and dried over anhydrous magnesium sulfate. After filtration, the filtrate was concentrated under a reduced pressure, and the resulting residue was applied to a flash silica gel column chromatography and eluted with an eluant of n-hexane:ethyl acetate = 5:1, thereby obtaining 2.178 g (63.9%) of the title compound as a colorless oil.
¹H-NMR (400 MHz, CDCl₃) δ: 5.04 (brs, 1 H), 4.27 (q, J = 7.16 Hz, 2 H), 1.44 (s, 9 H), 1.28 (t, J = 7.16 Hz, 3 H), 1.15 (m, 2 H), 1.06 (m, 2 H).

### [Reference Example 2-2]

### Ethyl 1-benzyl-4-(1-tert-butoxycarbonylaminocyclopropyl)-3-pyrroline-3-carboxylate

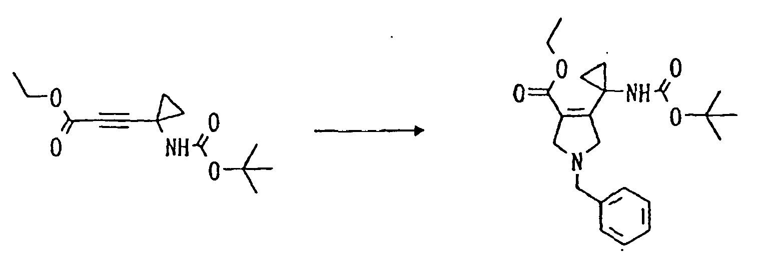

N-Benzyl-N-(n-butoxymethyl)trimethylsilylmethylamine (2.006 g, 7.176 mmol) and ethyl 3-(1-tert-butoxycarbonylaminocyclopropyl)propiolate (1.136 g, 4.485 mmol) were dissolved in dry dichloromethane (9 ml) and, while stirring at room temperature, the solution was mixed with dichloromethane solution of 1.0 M trifluoroacetic acid (0.72 ml, 0.72 mmol). After 3 hours of stirring, the reaction solution was mixed with saturated sodium bicarbonate aqueous solution (20 ml) and extracted with dichloromethane (20 ml x 3). The organic layers were combined, washed with saturated brine (30 ml) and then dried over anhydrous magnesium sulfate. After filtration, the filtrate was concentrated under a reduced pressure, and the resulting residue was applied to a flash silica gel column chromatography and eluted with chloroform, thereby obtaining 1.449 g (83.6%) of the title compound as a colorless oil.
¹H-NMR (400 MHz, CDCl₃) δ: 7.40 - 7.11 (m, 5 H), 5.17 (brs, 1 H), 4.12 (q, J = 6.83 Hz, 2 H), 3.85 (m, 2 H), 3.72 (m, 2 H), 3.67 (s, 2 H), 1.44 (s, 9 H), 1.24 (t, J = 6.83 Hz, 3 H), 1.14 (m, 2 H), 1.01 (m, 2 H).

### [Reference Example 2-3]

### Ethyl cis-1-benzyl-4-(1-tert-butoxycarbonylaminocyclopropyl)-pyrrolidine-3-carboxylate

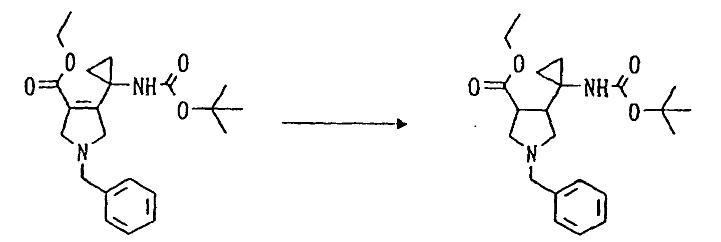

Under a stream of nitrogen, bis(bicyclo[2.2.1]hepta-2,5-diene)rhodium(I)perchlorate (54.5 mg, 0.14 mmol) and 1,2-bis(diphenylphpsphino)ethane (67.4 mg, 0.17 mmol) were dissolved in dried and degassed methanol (25 ml) and stirred at room temperature for 10 minutes. The thus prepared catalyst solution was mixed with dried and degassed methanol (15 ml) in which ethyl 1-benzyl-4-(1-tert-butoxycarbonylaminocyclopropyl)-3-pyrroline-3-carboxylate (1.090 g, 2.820 mmol) has been dissolved, and the reaction solution was stirred at room temperature for 2.5 hours under an atmosphere of hydrogen (1 kg/cm²). The reaction solution was mixed with activated carbon (1 g), and the mixture was stirred at room temperature for 30 minutes and then filtered through celite (methanol washing). After concentration of the filtrate under a reduced pressure, the resulting residue was applied to a flash silica gel column chromatography and eluted with an eluant of n-hexane:ethyl acetate = 5:1, thereby obtaining 1.071 g (97.8%) of the title compound as colorless crystals.
¹H-NMR (400 MHz, CDCl₃) δ: 7.40 - 7.19 (m, 5 H), 5.07 (brs, 1 H), 4.13 (q, J = 7.33 Hz, 2 H), 3.63 (s, 2 H), 2.87 (m, 1 H), 2.67 (m, 1 H), 2.54 (m, 1 H), 2.35 (m, 1 H), 2.15 (m, 1 H), 1.79 (m, 1 H), 1.46 (s, 9 H), 1.23 (t, J = 7.33 Hz, 3 H), 0.85 (m, 2 H), 0.69 (m, 2 H).

### [Reference Example 2-4]

### Cis-1-benzyl-4-(1-tert-butoxycarbonylaminocyclopropyl)-3-hydroxymethylpyrrolidine

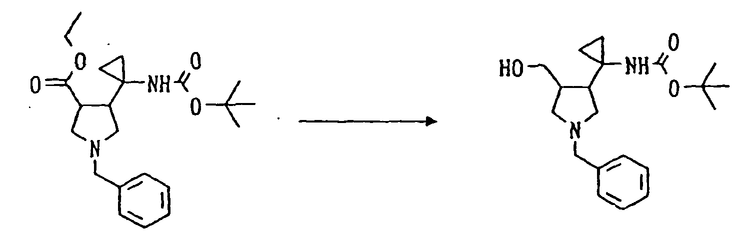

Under an atmosphere of nitrogen, lithium aluminum hydride (195.6 mg, 5.153 mmol) was suspended in anhydrous tetrahydrofuran (40 ml) to which, while stirring at -15°C, was subsequently added dropwise anhydrous tetrahydrofuran (10 ml) solution of ethyl cis-1-benzyl-4-(1-tert-butoxycarbonylaminocyclopropyl)pyrrolidine-3-carboxylate (1.001 g, 2.577 mmol) in 15 minutes. The reaction suspension was stirred for 3.5 hours in an ice bath, gradually mixed with cold water (5 ml) and then stirred for further 15 minutes at room temperature. The reaction suspension was filtered through celite (diethyl ether washing), and the resulting filtrate was concentrated under a reduced pressure and dried, thereby obtaining 833.9 mg (93.4%) of the title compound as a colorless oil.
¹H-NMR (400 MHz, CDCl₃) δ: 7.39 - 7.00 (m, 5 H), 5.10 (brs, 1 H), 3.69 (m, 2 H), 3.58 (s, 2 H), 2.99 (m, 1 H), 2.61 (m, 1 H), 2.51 (m, 1 H), 2.27 (m, 1 H), 2.00 (m, 1 H), 1.94 (brs, 1 H), 1.74 (m, 1 H), 1.42 (s, 9 H), 0.90 (m, 1 H), 0.74 - 0.61 (m, 3 H).

### [Reference Example 2-5]

### Cis-4-(1-tert-butoxycarbonylaminocyclopropyl)-3-hydroxymethylpyrrolidine

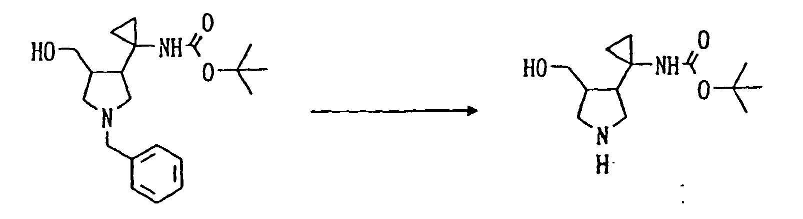

Cis-1-benzyl-4-(1-tert-butoxycarbonylaminocyclopropyl)-3-hydroxymethylpyrrolidine (820.1 mg, 2.376 mmol) was dissolved in methanol (50 ml), and the solution was mixed with 5% palladium-carbon catalyst (water content, 55.6%; 750 mg) and stirred for one day under a pressure of hydrogen (4.5 kg/cm²). After removing the catalyst by celite filtration (methanol washing), the resulting filtrate was concentrated under a reduced pressure to obtain 578.8 mg (91%) of the title compound as a white amorphous substance.
¹H-NMR (400 MHz, CDCl₃) δ: 5.05 (brs, 1 H), 3.72 (m, 2 H), 3.15 (m, 2 H), 2.82 (m, 2 H), 2.29 (m, 1 H), 1.99 (br, 2 H), 1.76 (m, 1 H), 1.42 (s, 9 H), 0.92 (m, 2 H), 0.82 (m, 1 H), 0.61 (m, 1 H).

### [Reference Example 2]

### 5-Amino-7-[cis-4-(1-aminocyclopropyl)-3-hydroxymethyl-1-pyrrolidinyl]-1-cyclopropyl-6-fluoro-1,4-dihydro-8-methyl-4-oxoquinoline-3-carboxylic acid

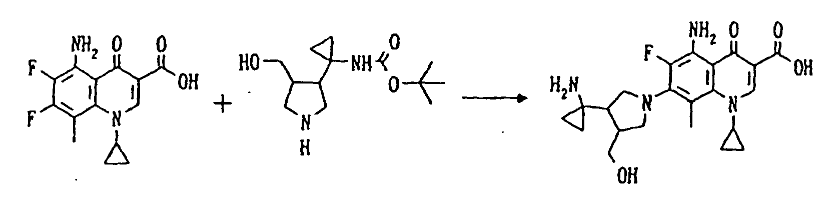

Cis-4-(1-tert-butoxycarbonylaminocyclopropyl)-3-hydroxymentylpyrrolidine (550.1 mg, 2.146 mmol) was dissolved in dimethyl sulfoxide (15 ml), and the solution was mixed with triethylamine (3.5 ml) and 5-amino-1-cyclopropyl-6,8-difluoro-1,4-dihydro-8-methyl-4-oxoquinoline-3-carboxylic acid (300.2 mg, 1.020 mmol) and stirred for 22 hours in an oil bath of 150°C under an atmosphere of nitrogen. After cooling, dimethyl sulfoxide was evaporated under reduced pressure, the resulting residue was dissolved in chloroform (100 ml) and washed with 10% citric acid aqueous solution (100 ml) and saturated brine (50 ml) in that order and then the organic layer was dried over anhydrous magnesium sulfate. After filtration, the filtrate was concentrated under a reduced pressure, concentrated hydrochloric acid (10 ml) was added dropwise to the resulting residue which was cooled in an ice bath, and then the mixture was stirred for 1 hour. The aqueous reaction solution was washed with dichloromethane (20 ml x 4), and the aqueous layer was adjusted to pH 12 with 15% sodium hydroxide aqueous solution and washed with dichloromethane (20 ml x 2). The aqueous solution was adjusted to pH 7.2 with 1 N hydrochloric acid and extracted with chloroform (100 ml x 4). The organic layers were combined, dried over anhydrous magnesium sulfate and filtered, and the resulting filtrate was concentrated under a reduced pressure. The thus obtained crude product was purified by recrystallizing from 2-propanol-diisopropyl ether, and the thus formed crystals were dried at 70°C for 18 hours under a reduced pressure to obtain 112.4 mg (25.6%) of the title compound as yellow crystals.
Melting point: 158.8 - 159.9°C (decomposition)
¹H-NMR (400 MHz, 0.1 N NaOD) δ: 8.39 (s, 1 H), 3.99 (m, 1 H), 3.80 (dd, J = 11.23, 5.37 Hz, 1 H), 3.62 (m, 2 H), 3.51 (d, J = 7.32, 2 H), 3.41 (t, J = 7.81 Hz, 1 H), 2.45 (m, 1 H), 2.37 (s, 3 H), 1.71 (q, J = 7.81, 1 H), 1.18 (m, 2 H), 0.74 (m, 1 H), 0.70 (m, 1 H), 0.55 (m, 4 H).

**Elemental analysis data; for C₂₂H₂₇FN₄O₄**

| | |
|---|---|
| calcd.; | C, 61.38; H, 6.32; N, 13.02 |
| found ; | C, 61.25; H, 6.32; N, 12.74 |

### [Reference Example 3-1]

### (3R,4S)-4-(1-Ethoxycarbonylcyclopropyl)-3-methyl-1-[(S)-1-phenylethyl]-2-pyrrolidone

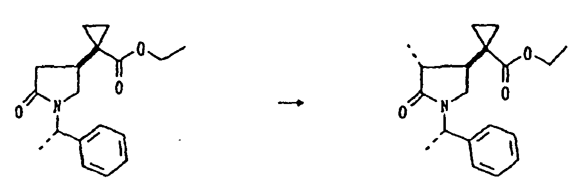

The following reaction was carried out under an atmosphere of nitrogen. At -78°C, n-butyl lithium (5.39 ml, 1.68 N, n-hexane solution, 9.06 mmol) was added dropwise to tetrahydrofuran solution (40 ml) of diisopropylethylamine (1.37 ml, 9.75 mmol), and the mixture was warmed to 0°C and stirred for 30 minutes. At -78°C, to this was further added dropwise tetrahydrofuran solution (20 ml) of (4S)-4-(1-ethoxycarbonylcyclopropyl)-1-[(S)-1-phenylethyl]-2-pyrrolidone (2.10 g, 6.97 mmol). After additional 15 minutes of stirring, methyl iodide (2.17 ml, 34.8 mmol) was added dropwise thereto, and the mixture was stirred for 30 minutes while warming up to 0°C. After completion of the reaction, this was cooled in an ice bath and mixed with saturated ammonium chloride aqueous solution (150 ml) and then tetrahydrofuran was evaporated. The resulting residue was extracted with chloroform (150 ml x 3), and the organic layer was dried over anhydrous sodium sulfate. After evaporation of the solvent, the resulting residue was purified by a silica gel column chromatography (silica gel, 160 ml; ethyl acetate:hexane = 2:3), thereby obtaining 1.90 g (87%) of the title compound.
¹H-NMR (400 MHz, CDCl₃) δ: 0.67 - 0.75 (2 H, m), 1.06 (3 H, t, J = 7.33 Hz), 1.14 - 1.19 (2 H, m), 1.24 (3 H, d, J = 7.33 Hz), 1.52 (3 H, d, J = 7.33 Hz), 1.98 (1 H, q, J = 9.03 Hz), 2.40 (1 H, dq, J = 9.03, 7.33 Hz), 2.84 (1 H, t, J = 9.03 Hz), 3.39 (1 H, t, J = 9.03 Hz), 3.95 - 4.06 (2 H, m), 5.53 (1 H, q, J = 7.33 Hz), 7.28 - 7.35 (5 H, m).

### [Reference Example 3-2]

### (3R,4S)-4-(1-Ethoxycarbonylcyclopropyl)-3-methyl-1-[(S)-1-phenylethyl]-2-pyrrolidinethione

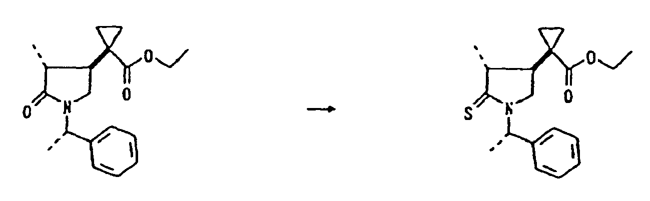

(3R,4S)-4-(1-Ethoxycarbonylcyclopropyl)-3-methyl-1-[(S)-1-phenylethyl]-2-pyrrolidone (1.85 g, 5.87 mmol) was dissolved in benzene (100 ml), and the solution was mixed with Lawesson reagent (1.31 g, 3.24 mmol) and heated under reflux for 20 minutes. After completion of the reaction, the solvent was evaporated and the resulting residue was purified by a silica gel column chromatography (silica gel, 160 ml; ethyl acetate:hexane = 1:4), thereby obtaining 1.80 g (92%) of the title compound.
¹H-NMR (400 MHz, CDCl₃) δ: 0.63 - 0.69 (2 H, m), 1.11 (3 H, t, J = 7.08 Hz), 1.15 - 1.18 (2 H, m), 1.41 (3 H, d, J = 7.32 Hz), 1.58 (3 H, d, J = 6.84 Hz), 2.02 - 2.08 (1 H, m), 2.73 - 2.80 (1 H, m), 3.11 (1 H, dd, J = 7.81, 11.23 Hz), 3.65 (1 H, dd, J = 8.79, 11.23 Hz), 3.95 - 4.06 (2 H, m), 6.44 (1 H, q, J = 6.84 Hz), 7.28 - 7.39 (5 H, m).

### [Reference Example 3-3]

### (3S,4R)-3-(1-Ethoxycarbonylcyclopropyl)-4-methyl-1-[(S)-1-phenylethyl]pyrrolidine

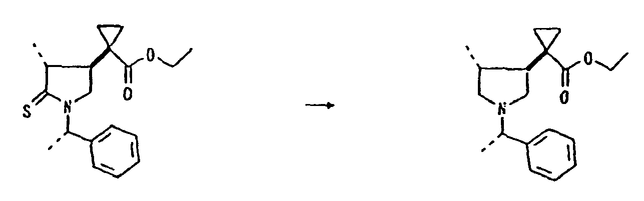

(3S,4R)-3-(1-Ethoxycarbonylcyclopropyl)-4-methyl-1-[(S)-1-phenylethyl]-2-pyrrolidinethion (1.80 g, 5.43 mmol) was dissolved in ethanol (100 ml), and the solution was mixed with Raney nickel (10 ml) and heated under reflux for 1.5 hours. After completion of the reaction, the reaction solution was filtered through celite and the filtrate was concentrated under a reduced pressure. The resulting residue was dissolved in chloroform (100 ml), washed with 10% ammonia water (100 ml), water (100 ml) and saturated brine (100 ml) in that order and then dried over anhydrous sodium sulfate. After evaporation of the solvent, the resulting residue was purified by a silica gel column chromatography (silica gel, 160 ml; ethyl acetate:hexane = 1:1), thereby obtaining 558 mg (34%) of the title compound.
¹H-NMR (400 MHz, CDCl₃) δ: 0.75 - 0.83 (2 H, m), 1.02 (3 H, d, J = 6.84 Hz), 1.11 - 1.14 (2 H, m), 1.21 (3 H, t, J = 7.08 Hz), 1.30 (3 H, d, J = 6.59 Hz), 1.70 - 1.78 (1 H, m), 2.04 - 2.15 (1 H, m), 2.19 (1 H, dd, J = 6.35, 9.03 Hz), 2.42 (1 H, dd, J = 9.03, 6.83 Hz), 2.58 (1 H, t, J = 8.55 Hz), 2.67 (1 H, t, J = 8.55 Hz), 3.13 (1 H, q, J = 6.59 Hz), 4.05 - 4.11 (2 H, m), 7.21 - 7.33 (5 H, m).

### [Reference Example 3-4]

### (3S,4R)-1-Benxyloxycarbonyl-3-(1-ethoxycarbonylcyclopropyl)-4-methylpyrrolidine

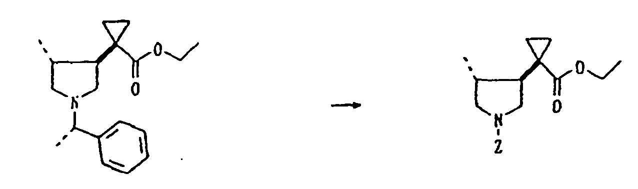

(3S,4R)-3-(1-Ethoxycarbonylcyclopropyl)-4-methyl-1-[(S)-1-phenylethyl]-2-pyrrolidine (1.24 g, 4.13 mmol) was dissolved in dichloromethane (40 ml) to which was subsequently added dropwise benzyl chloroformate (0.766 ml, 5.37 mmol). After completion of the dropwise addition, the reaction solution was heated under reflux for 1.5 hours. After completion of the reaction, the solvent was evaporated and the resulting residue was purified by a silica gel column chromatography (silica gel, 100 ml; ethyl acetate:hexane = 1:4), thereby obtaining 1.17 g (88%) of the title compound.
¹H-NMR (400 MHz, CDCl₃) δ: 0.69 - 0.77 (2 H, m), 1.04 (3 H, dd, J = 6.83, 7.81 Hz), 1.20 - 1.26 (5 H, m), 1.75 - 1.87 (1 H, m), 2.27 - 2.37 (1 H, m), 2.91 (1 H, dt, J = 2.93, 10.25 Hz), 3.32 (1 H, dd, J = 10.74, 21.49 Hz), 3.59 - 3.75 (2 H, m), 4.07 - 4.13 (2 H, m), 5.12 (2 H, s), 7.21 - 7.33 (5 H, m).

### [Reference Example 3-5]

### 1-[(3S,4R)-1-Benzyloxycarbonyl-4-methyl-3-pyrrolidinyl]cyclopropanecarboxylic acid

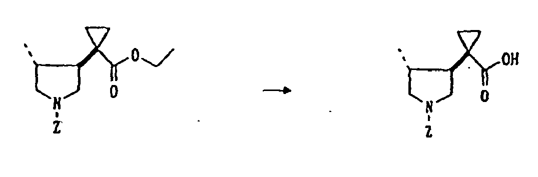

(3S,4R)-1-Benzyloxycarbonyl-3-(1-ethoxycarbonylcyclopropyl)-4-methylpyrrolidine (1:17 g, 3.66 mmol) was dissolved in ethanol (100 ml), and the solution was mixed with 1 N sodium hydroxide aqueous solution (11 ml) and heated under reflux for 8 hours. After completion of the reaction, the solvent was evaporated and the resulting residue was mixed with 0.5 N hydrochloric acid aqueous solution (30 ml). This was extracted with ethyl acetate (50 ml x 3), and the organic layer was washed with water (50 ml) and saturated sodium chloride aqueous solution (50 ml) in that order. This was dried over anhydrous sodium sulfate and then the solvent was evaporated to obtain 1.20 g of the title compound quantitatively.
¹H-NMR (400 MHz, CDCl₃) δ: 0.77 - 0.85 (2 H, m), 1.05 (3 H, t, J = 6.84 Hz), 1.25 - 1.35 (2 H, m), 1.69 (1 H, q, J = 9.57 Hz), 2.34 - 2.46 (1 H, m), 2.90 (1 H, dd, J = 6.35, 9.57 Hz), 3.39 (1 H, t, J = 10.26 Hz), 3.59 - 3.75 (2 H, m), 5.12 (2 H, s), 7.30 - 7.38 (5 H, m).

### [Reference Example 3-6]

### (3R,4R)-1-Benzyloxycarbonyl-3-(1-tert-butoxycarbonylaminocyclopropyl)-4-methylpyrrolidine

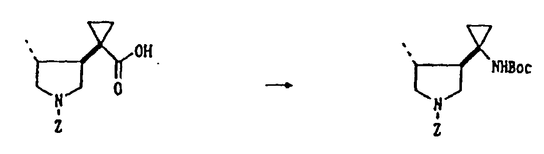

1-[(3S,4R)-1-Benzyloxycarbonyl-4-methyl-3-pyrrolidinyl]cyclopropanecarboxylic acid (1.20 g, 3.66 mmol) was dissolved in tert-butyl alcohol (50 ml), and the solution was mixed with diphenylphosphoryl azide (0.946 ml, 4.39 mmol) and triethylamine (1.02 ml, 7.32 mmol) and heated under reflux for 19 hours. After completion of the reaction, the solvent was evaporated and the resulting residue was purified by a silica gel column chromatography (silica gel, 120 ml; ethyl acetate:hexane = 1:2), thereby obtaining 0.793 g (58%) of the title compound.
¹H-NMR (400 MHz, CDCl₃) δ: 0.52 - 0.60 (1 H, m), 0.70 - 0.82 (2 H, m), 0.90 - 1.01 (1 H, m), 1.15 (3 H, d, J = 5.37 Hz), 1.41 (9 H, s), 1.43 - 1.50 (1 H, m), 2.08 - 2.17 (1 H, m), 2.91 (1 H, dt, J = 5.86, 10.26 Hz), 3.28 (1 H, t, J = 10.26 Hz), 3.57 - 3.73 (2 H, m), 4.80 (1 H, d, J = 7.82 Hz), 5.12 (2 H, s), 7.29 - 7.37 (5 H, m).

### [Inventive Example 3]

### 5-Amino-7-[(3R,4R)-3-(1-aminocyclopropyl)-4-methyl-1-pyrrolidinyl]-6-fluoro-1-[(1R,2S)-2-fluorocyclopropyl]-1,4-dihydro-8-methyl-4-oxoquinoline-3-carboxylic acid

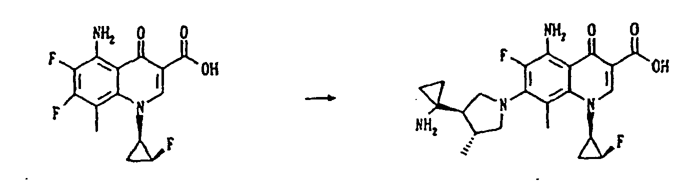

(3R,4R)-1-Benzyloxycarbonyl-3-(1-tert-butoxycarbonylaminocyclopropyl)-4-methylpyrrolidine (793 mg, 2.12 mmol) was dissolved in ethanol (50 ml), and the solution was mixed with 5% palladium-carbon (790 mg) to carry out hydrogenation under 5 atmospheric pressure. After completion of the reaction, 5% palladium carbon was removed by filtration and ethanol was evaporated. The thus obtained residue was dissolved in dimethyl sulfoxide (8 ml), and the solution was mixed with triethylamine (2 ml) and 5-amino-6,7-difluoro-1-[(1R,2S)-2-fluorocyclopropyl]-1,4-dihydro-8-methyl-4-oxoquinoline-3-carboxylic acid (330 mg, 1.06 mmol) and stirred at 150°C for 18 hours. After completion of the reaction, dimethyl sulfoxide was evaporated, and the thus obtained residue was mixed with chloroform (100 ml) and washed with 10% citric acid (100 ml) and saturated brine (100 ml) in that order. The organic layer was dried over anhydrous sodium sulfate and then the solvent was evaporated. To the thus obtained residue, which was cooled in an ice bath, was added dropwise concentrated hydrochloric acid (10 ml), followed by 1 hour of stirring at room temperature. After completion of the reaction, the reaction solution was washed with dichloromethane (20 ml). The aqueous layer was adjusted to pH 12 with sodium hydroxide aqueous solution and then to pH 7.4 with hydrochloric acid, subsequently carrying out extraction with chloroform (100 ml x 4). The organic layers were combined and dried over anhydrous sodium sulfate and then the solvent was evaporated. The thus obtained residue was applied to a silica gel thin layer chromatography and developed with the bottom layer of a mixture solvent of chloroform:methanol = 3:1, and then the resulting silica gel was scratched off and extracted with the same solvent system. The solvent was evaporated and the thus obtained residue was dissolved in 1 N hydrochloric acid aqueous solution (6 ml) and stirred at room temperature for 10 minutes. After evaporation of the solvent, the resulting crude product was recrystallized from isopropyl alcohol to obtain 20.1 mg (4%) of the title compound.
Melting point: 203 - 205°C (decomposition)
[α]_{D}²⁴ = -162.93 (c = 0.205, 0.1 N sodium hydroxide aqueous solution)
¹H-NMR (400 MHz, 0.1 N NaOD) δ: 0.35 - 0.41 (1 H, m), 0.48 - 0.60 (3 H, m), 1.10 - 1.15 (1 H, m), 1.12 (3 H, d, J = 6.35 Hz), 1.40 - 1.55 (2 H, m), 2.26 (3 H, s), 2.18 - 2.24 (1 H, m), 3.30 (1 H, t, J = 8.55 Hz), 3.29 - 3.51 (2 H, m), 3.76 - 3.78 (1 H, m), 3.89 - 3.94 (1 H, m), 4.96 (1 H, dm, J = 65.91 Hz), 8.25 (1 H, d, J = 2.93 Hz).

**Elemental analysis data; for C₂₂H₁₆F₂N₄O₃•HCl•1.25H₂O:**

| | |
|---|---|
| calcd.; | C, 53.77; H, 6.05; N, 11.40 |
| found ; | C, 53.68; H, 6.05; N, 11.12 |

### [Reference Example 4-1]

### 4-(S)-(1-Ethoxycarbonylcyclopropyl)-3-(R)-hydroxy-1-[1-(S)-phenylethyl]-2-pyrrolidone

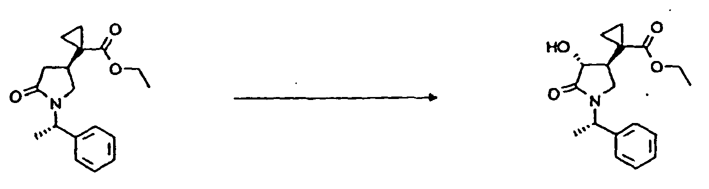

Under an atmosphere of nitrogen, diisopropylamine (3.93 ml, 28.0 mmol) was dissolved in anhydrous tetrahydrofuran (200 ml) to which, after cooling to -78°C, was subsequently added dropwise n-hexane solution of 1.69 M n-butyl lithium (15.9 ml, 26.9 mmol) in 10 minutes. After 20 minutes of stirring at 0°C and subsequent cooling to -78°C, to the resulting reaction solution was added dropwise anhydrous tetrahydrofuran solution (40 ml) of 4-(S)-(1-ethoxycarbonylcyclopropyl)-1-[1-(S)-phenylethyl]-2-pyrrolidone (6.74 g, 22.4 mmol) in 15 minutes. The reaction solution was stirred at -78°C for 10 minutes and then the reaction vessel was charged with dried oxygen at the same temperature. The reaction solution was stirred at -78°C for 20 minutes and then mixed with saturated ammonium chloride aqueous solution (200 ml). This was warmed up to room temperature and the organic layer was separated. The aqueous layer was extracted with diethyl ether (200 ml x 2), and the organic layers were combined and dried over anhydrous magnesium sulfate. After filtration, the filtrate was concentrated under a reduced pressure, and the resulting residue was applied to a flash silica gel chromatography and eluted with an eluant of n-hexane:ethyl acetate = 2:1, thereby obtaining 5.21 g (73%) of the title compound as a colorless oil.
¹H-NMR (400 MHz, CDCl₃) δ: 0.86 - 0.96 (2 H, m), 1.13 (3 H, t, J = 7.08 Hz), 1.18 - 1.30 (2 H, m), 1.56 (3 H, d, J = 6.92 Hz), 2.38 (1 H, dd, J = 18.06, 9.28 Hz), 2.81 (1 H, t, J = 9.28 Hz), 3.50 (2 H, t, J = 9.28 Hz), 3.99 - 4.07 (2 H, m), 4.11 (1 H, d, J = 9.28 Hz), 5.48 (1 H, q, J = 6.92 Hz), 7.26 - 7.36 (5 H, m).

### [Reference Example 4-2]

### 3-(R)-Tert-butyldimethylsilyloxy-4-(S)-(1-ethoxycarbonylcyclopropyl)-1-[1-(S)-1-phenylethyl]-2-pyrrolidone

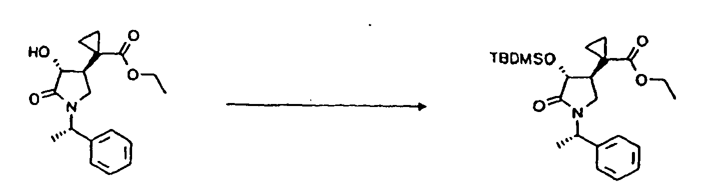

4-(S)-(1-Ethoxycarbonylcyclopropyl)-3-(R)-hydroxy-1-[1-(S)-phenylethyl]-2-pyrrolidone (7.26 g, 22.87 mmol) was dissolved in anhydrous dimethylformamide (75 ml), and the solution was mixed with imidazole (3.90 g, 57.3 mmol) and stirred at room temperature for 10 minutes. This was mixed with tert-butylchlorodimethylsilane (4.32 g, 28.7 mmol) and stirred for 4 hours. After concentration of the mixture under a reduced pressure, the thus obtained residue was dissolved in ethyl acetate (300 ml), washed with water (150 ml), saturated sodium bicarbonate aqueous solution (150 x 5) and saturated brine (150 ml) in that order and then dried over anhydrous sodium sulfate. After filtration, the filtrate was concentrated under a reduced pressure and the resulting residue was applied to a flash silica gel chromatography and eluted with an eluant of n-hexane:ethyl acetate = 6:1, thereby obtaining 8.74 g (88%) of the title compound as a colorless oil.
¹H-NMR (400 MHz, CDCl₃) δ: 0.043 (3 H, s), 0.122 (3 H, s), 0.54 - 0.63 (1 H, m), 0.79 (9 H, s), 0.95 (3 H, t, J = 7.08 Hz), 1.03 - 1.15 (3 H, m), 1.38 (3 H, d, J = 6.98 Hz), 1.61 - 1.90 (1 H, m), 2.83 (1 H, t, J = 9.28 Hz), 3.13 (1 H, t, J = 9.28 Hz), 3.81 - 3.90 (2 H, m), 4.48 (1 H, d, J = 9.28 Hz), 5.36 (1 H, q, J = 6.96 Hz), 7.14 - 7.19 (5 H, m).

### [Reference Example 4-3]

### 3-(R)-Tert-butyldimethylsilyloxy-4-(S)-(1-ethoxycarbonylcyclopropyl)-1-[1-(S)-phenylethyl]-2-pyrrolidinethione

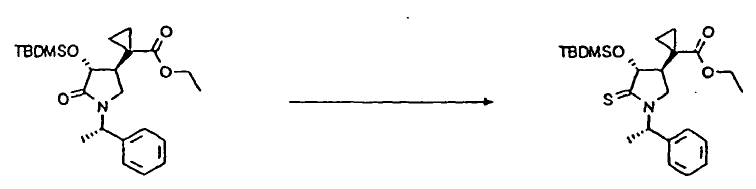

3-(R)-Tert-butyldimethylsilyloxy-4-(S)-(1-ethoxycarbonylcyclopropyl)-1-[1-(S)-phenylethyl]-2-pyrrolidone was dissolved in dry benzene (200 ml), and the solution was mixed with Lawesson reagent (4.49 g, 11.1 mmol) and heated under reflux for 3 hours. After cooling, benzene was evaporated under a reduced pressure, and the resulting residue was applied to a flash silica gel chromatography and eluted with an eluant of n-hexane:ethyl acetate = 4:1, thereby obtaining 7.96 g (88%) of the title compound as a light yellow oil.
¹H-NMR (400 MHz, CDCl₃) δ: 0.176 (3 H, s), 0.327 (3 H, s), 0.63 - 0.68 (1 H, m), 0.92 - 0.95 (1 H, m), 0.95 (9 H, s), 1.11 (3 H, t, J = 7.08 Hz), 1.15 - 1.20 (1 H, m), 1.29 - 1.34 (1 H, m), 1.58 (3 H, d, J = 6.84 Hz), 1.68 - 1.79 (1 H, m), 3.27 (1 H, t, J = 10.74 Hz), 3.44 (1 H, dd, J = 10.74, 8.79 Hz), 3.99 - 4.01 (2 H, m), 4.93 (1 H, d, J = 8.30 Hz), 6.38 (1 H, q, J = 6.84 Hz), 7.44 - 7.46 (5 H, m).

### [Reference Example 4-4]

### 3-(S)-Tert-butyldimethylsilyloxy-4-(R)-(1-ethoxycarbonylcyclopropyl)-1-[1-(S)-phenylethyl]pyrrolidine

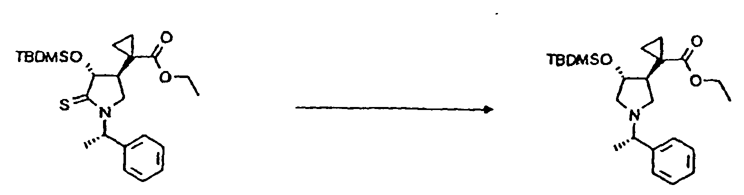

3-(R)-Tert-butyldimethylsilyloxy-4-(S)-(1-ethoxycarbonylcyclopropyl)-1-[1-(S)-phenylethyl]-2-pyrrolidinethione (7.96 g, 17.74 mmol) was dissolved in anhydrous ethanol (490 ml), and the solution was mixed with Raney nickel (25 ml) and heated under reflux for 40 minutes. After removing the catalyst by celite filtration (ethanol washing), the resulting filtrate was concentrated under a reduced pressure. The thus obtained residue was dissolved in chloroform (400 ml), washed with 10% ammonia water (300 ml), water (300 ml) and saturated brine (300 ml) in that order and then dried over anhydrous sodium sulfate. After filtration, the filtrate was concentrated under a reduced pressure and the resulting residue was applied to a flash silica gel chromatography and eluted with an eluant of n-hexane:ethyl acetate = 6:1, thereby obtaining 5.48 g (74%) of the title compound as a colorless oil.
¹H-NMR (400 MHz, CDCl₃) δ: 0.023 (3 H, s), 0.038 (3 H, s), 0.61 - 0.64 (1 H, m), 0.83 - 0.85 (1 H, m), 0.84 (9 H, s), 1.11 - 1.13 (2 H, m), 1.17 (3 H, t, J = 7.33 Hz), 1.29 (3 H, d, J = 6.83 Hz), 1.74 - 1.79 (1 H, m), 2.35 (1 H, t, J = 9.27 Hz), 2.62 - 2.67 (1 H, m), 2.74 - 2.77 (1 H, m), 3.16 (1 H, q, J = 6.51 Hz), 4.00 - 4.06 (2 H, m), 4.33 - 4.37 (1 H, m), 7.23 - 7.30 (5 H, m).

### [Reference Example 4-5]

### 1-Benzyloxycarbonyl-3-(S)-tert-butyldimethylsilyloxy-4-(R)-(1-ethoxycarbonylcyclopropyl)pyrrolidine

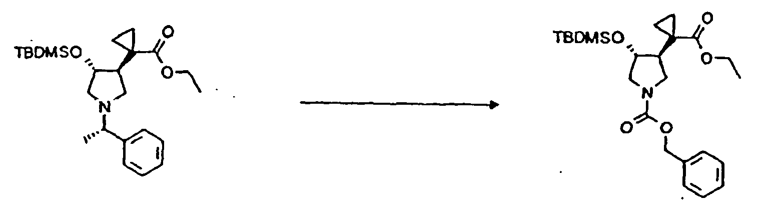

3-(S)-Tert-butyldimethylsilyloxy-4-(R)-(1-ethoxycarbonylcyclopropyl)-1-[1-(S)-phenylethyl]pyrrolidine (5.48 g, 13.15 mmol) was dissolved in dry dichloromethane (120 ml), and benzyl chloroformate (3.76 ml, 26.3 mmol) was added dropwise to the thus prepared solution which was cooled in an ice bath. After 2 hours of heating of the reaction solution under reflux, dichloromethane was evaporated under a reduced pressure. Thereafter, the resulting residue was applied to a flash silica gel chromatography and eluted with an eluant of n-hexane:ethyl acetate = 5:1, thereby obtaining 4.52 g (77%) of the title compound as a colorless oil.
¹H-NMR (400 MHz, CDCl₃) δ: 0.049 (6 H, s), 0.66 - 0.71 (1 H, m), 0.87 (9 H, s), 0.93 - 0.97 (1 H, m), 1.04 - 1.08 (1 H, m), 1.22 (3 H, t, J = 3.42 Hz), 1.36 - 1.39 (1 H, m), 1.77 - 1.87 (1 H, m), 3.08 (1 H, t, J = 8.29 Hz), 3.43 (1 H, q, J = 10.42 Hz), 3.60 - 3.82 (2 H, m), 4.08 - 4.16 (2 H, m), 4.54 - 4.63 (1 H, m), 5.10 - 5.18 (2 H, m), 7.29 - 7.35 (5 H, m).

### [Reference Example 4-6]

### 1-Benzyloxycarbonyl-3-(S)-hydroxy-4-(R)-(1-ethoxycarbonylcyclopropyl)pyrrolidine

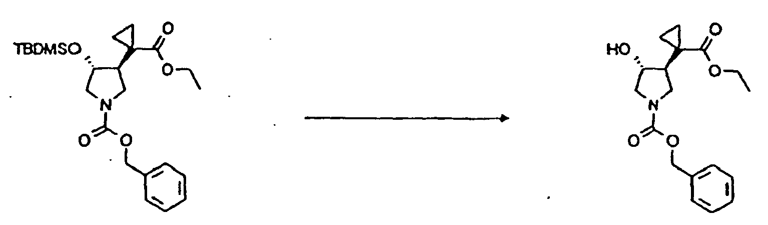

1-Benzyloxycarbonyl-3-(S)-tert-butyldimethylsilyloxy-4-(R)-(1-ethoxycarbonylcyclopropyl)pyrrolidine (1.79 g, 4.00 mmol) was dissolved in tetrahydrofuran (40 ml) to which, cooled in an ice bath, was subsequently added dropwise tetrahydrofuran solution of 1.0 M tetrabutylammonium fluoride (5.33 ml, 5.33 mmol). The reaction solution was stirred at room temperature for 30 minutes and then tetrahydrofuran was evaporated under a reduced pressure. Thereafter, the resulting residue was applied to a flash silica gel chromatography and eluted with an eluant of n-hexane:ethyl acetate = 1:1, thereby obtaining 1.04 g (76%) of the title compound as a colorless oil.
¹H-NMR (400 MHz, CDCl₃) δ: 0.80 - 0.89 (2 H, m), 1.21 - 1.28 (5 H, m), 2.57 - 2.73 (1 H, m), 2.85 - 2.98 (1 H, m), 3.23 - 3.33 (2 H, m), 3.62 - 3.67 (1 H, m), 3.82 - 3.99 (1 H, m), 4.10 - 4.25 (3 H, m), 5.12 (2 H, s), 7.28 - 7.39 (5 H, m).

### [Reference Example 4-7]

### 1-[1-Benzyloxycarbonyl-4-(R)-methoxy-3-(S)-pyrrolidinyl]cyclopropanecarboxylic acid

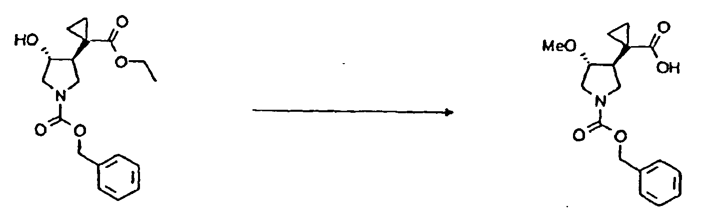

Under an atmosphere of nitrogen, 60% sodium hydride (0.149 g, 3.73 mmol) was suspended in anhydrous tetrahydrofuran (20 ml) to which, after cooling to 0°C, was subsequently added dropwise dry tetrahydrofuran (20 ml) solution of 1-benzyloxycarbonyl-3-(S)-hydroxy-4-(R)-(1-ethoxycarbonylcyclopropyl)pyrrolidine (0.98 g, 2.92 mmol) in 5 minutes. After 15 minutes of stirring in an ice bath, dimethyl sulfate (0.441 ml, 4.66 mmol) was added dropwise to the reaction solution which was cooled in the ice bath. The reaction solution was stirred at room temperature for 4 hours and then mixed with water (0.5 ml), followed by evaporation of tetrahydrofuran under a reduced pressure. The thus obtained residue was dissolved in ethanol (40 ml), and 1 M sodium hydroxide aqueous solution (8.76 ml) was added dropwise to the resulting solution at room temperature. The reaction solution was heated under reflux for 2 hours and then ethanol was evaporated under a reduced pressure. The resulting residue was cooled in an ice bath, acidified by adding dropwise 1 M hydrochloric acid aqueous solution (15 ml) and then extracted with ethyl acetate (50 ml x 3). All of the organic layers were combined, washed with 1 N hydrochloric acid aqueous solution (50 ml) and saturated brine (50 ml) and then dried over anhydrous sodium sulfate. After filtration, the filtrate was concentrated under a reduced pressure to obtain 0.935 g (quantitative) of the title compound as a colorless amorphous substance.
¹H-NMR (400 MHz, CDCl₃) δ: 0.84 - 0.89 (1 H, m), 0.89 - 1.01 (1 H, m), 1.25 - 1.35 (2 H, m), 2.33 - 2.40 (1 H, m), 3.22 - 3.30 (2 H, m), 3.35 (3 H, s), 3.74 - 3.91 (3 H, m), 5.12 (2 H, s), 7.32 - 7.38 (5 H, m).

### [Reference Example 4-8]

### 1-Benzyloxycarbonyl-4-(R)-(1-tert-butoxycarbonylaminocyclopropyl)-3-(R)-inethoxypyrrolidine

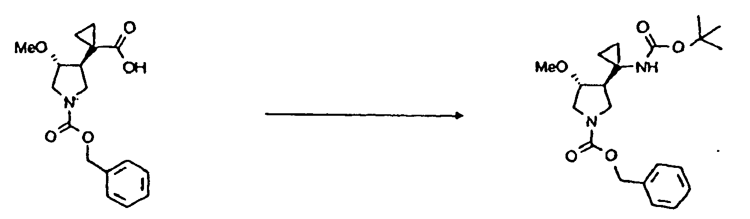

1-[1-Benzyloxycarbonyl-4-(R)-methoxy-3-(S)-pyrrolidinyl]cyclopropanecarboxylic acid (713 mg, 2.22 mmol) was dissolved in tert-butyl alcohol (20 ml), and the solution was mixed with diphenylphosphoryl azide (623 µl, 2.89 mmol) and triethylamine (775 µl, 5.56 mmol), stirred at room temperature for 20 minutes and then heated under reflux for 19 hours. The reaction solution was cooled and then concentrated under a reduced pressure, and the resulting residue was applied to a flash silica gel chromatography and eluted with an eluant of n-hexane:ethyl acetate = 3:2, thereby obtaining the title compound (431 mg, 50%) as a colorless oil.
¹H-NMR (400 MHz, CDCl₃) δ: 0.65 - 0.97 (4 H, m), 1.39, 1.41 (total 9 H, each s), 2.03 - 2.07 (1 H, m), 3.32, 3.34 (total 3 H, each s), 3.28 - 3.46 (2 H, m), 3.95 - 4.06 (1 H, m), 5.13 (2 H, s), 7.29 - 7.38 (5 H, m).

### [Inventive Example 4]

### 5-Amino-7-[4-(R)-(1-aminocyclopropyl)-3-(R)-methoxy-1-pyrrolidinyl]-6-fluoro-1-[2-(S)-fluoro-1-(R)-cyclopropyl]-1,4-dihydro-8-methyl-4-oxoquinoline-3-carboxylic acid

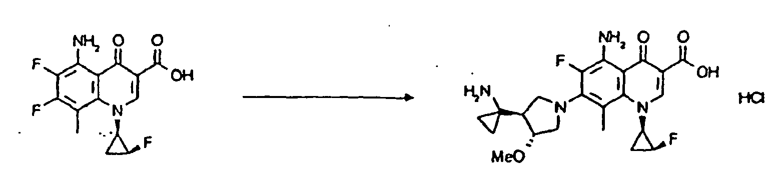

1-Benzyloxycarbonyl-4-(R)-(1-tert-butoxycarbonylaminocyclopropyl)-3-(R)-methoxypyrrolidine (550 mg, 1.41 mmol) was dissolved in ethanol (40 ml), and the solution was mixed with 5% palladium-carbon catalyst (water content, 55.6%; 550 mg) and stirred for 4 hours under a pressured hydrogen atmosphere (4.5 kg/cm²). The catalyst was removed by celite filtration (methanol washing), and the filtrate was concentrated under a reduced pressure. The thus obtained residue was dissolved in dimethyl sulfoxide (5 ml), and the solution was mixed with 5-amino-6,7-difluoro-1-[2-(S)-fluoro-1-(R)-cyclopropyl]-1,4-dihydro-8-methyl-4-oxoquinoline-3-carboxylic acid (249.8 mg, 0.80 mmol) and triethylamine (3 ml) and stirred for 2 days in an oil bath of 120°C under an atmosphere of nitrogen. After cooling, dimethyl sulfoxide was evaporated under a reduced pressure, the thus obtained residue was dissolved in chloroform (100 ml) and washed with 10% citric acid aqueous solution (50 ml x 2) and saturated brine (100 ml) in that order and then the organic layer was dried over anhydrous magnesium sulfate. After filtration, the filtrate was concentrated under a reduced pressure. To the thus obtained residue, which was cooled in an ice bath, was added dropwise concentrated hydrochloric acid (10 ml), followed by 1 hour of stirring at room temperature. After adding water (20 ml) to the reaction solution, the aqueous solution was washed with dichloromethane (50 ml x 3), adjusted to pH 11 with sodium hydroxide aqueous solution and then washed with dichloromethane (50 ml x 2). This was adjusted to pH 7.4 with concentrated hydrochloric acid and extracted with chloroform (150 ml x 3). The organic layers were combined, dried over anhydrous sodium sulfate and then filtered, and the filtrate was concentrated under a reduced pressure. The thus obtained residue was applied to a preparative silica gel thin layer chromatography (development with the bottom layer of a mixture of chloroform:methanol:water = 7:3:1), and the resulting crude product was purified by recrystallizing from an ethanol-diisopropyl ether and then dried under a reduced pressure to obtain 57 mg (16%) of the title compound as a yellow powder.
Melting point: 202.4 - 204.3°C
[α]_{D}²⁴ = -154.03° (c = 0.335, 0.1 N NaOH)
IR (KBr disk): 3464, 3344, 2892, 2832, 1722, 1628, 1584, 1504, 1428, 1342, 1292, 1228 cm⁻¹
¹H-NMR (400 MHz, 0.1 N NaOD) δ: 0.60 - 0.65 (4 H, m), 1.14 - 1.24 (1 H, m), 1.49 - 1.59 (1 H, m), 2.07 - 2.13 (1 H, m), 2.37 (3 H, s), 3.41 - 3.66 (4 H, m), 3.44 (3 H, s), 3.96 - 4.09 (2 H, m), 4.95 (1 H, dm, J = 64.94 Hz), 8.31 (1 H, d, J = 2.44 Hz).

**Elemental analysis data; for C₂₂H₂₆F₂N₄O₄•0.75H₂O:**

| | |
|---|---|
| calcd.; | C, 57.20; H, 6.00; N, 12.13 |
| found ; | C, 57.43; H, 5.80; N, 11.90 |

### [Reference Example 5-1]

### 4-(S)-(1-Ethoxycarbonylcyclopropyl)-3-(R)-fluoro-1-[1-(S)-phenylethyl]-2-pyrrolidone

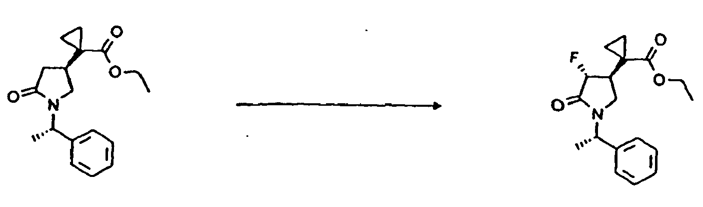

Under an atmosphere of nitrogen, diisopropylamine (3.99 ml, 30.4 mmol) was dissolved in anhydrous tetrahydrofuran (50 ml) to which, after cooling to -78°C, was subsequently added dropwise n-hexane solution of 1.68 M n-butyl lithium (18.1 ml, 30.4 mmol) in 10 minutes. After 20 minutes of stirring at -10°C and subsequent cooling to -78°C, to the resulting reaction solution was added dropwise anhydrous tetrahydrofuran solution (30 ml) of 4-(S)-(1-ethoxycarbonylcyclopropyl)-1-[1-(S)-phenylethyl]-2-pyrrolidone (7.052 g, 23.40 mmol) in 15 minutes. The reaction solution was stirred at -78°C for 1 hour and then anhydrous tetrahydrofuran solution (60 ml) of N-fluorobenzene disulfonimide (11.81 g, 37.44 mmol) was added dropwise thereto at the same temperature in 25 minutes. The reaction solution was stirred at -78°C for 2 hours and then at room temperature for 20 minutes. While cooling in an ice bath, the reaction solution was mixed with saturated ammonium chloride aqueous solution (200 ml), the organic layer was separated and then the aqueous layer was extracted with diethyl ether (200 ml x 2). The organic layers were combined, washed with water (200 ml x 3) and then dried over anhydrous magnesium sulfate. After filtration, the filtrate was concentrated under a reduced pressure, and the resulting residue was applied to a flash silica gel chromatography and eluted with an eluant of n-hexane:ethyl acetate = 3:1, thereby obtaining 5.276 g (70.6%) of the title compound as a colorless oil.
¹H-NMR (400 MHz, CDCl₃) δ: 0.76 - 0.81 (1 H, m), 0.89 - 0.93 (1 H, m), 1.09 (3 H, t, J = 6.84 Hz), 1.24 - 1.34 (2 H, m), 1.58 (3 H, d, J = 7.33 Hz), 2.23 (1 H, dq, J = 28.32, 8.30 Hz), 2.88 - 2.93 (1 H, m), 3.48 (1 H, t, J = 9.28 Hz), 3.92 - 4.08 (2 H, m), 5.14 (1 H, dd, J = 53.71, 7.81 Hz), 5.54 (1 H, q, J = 7.33 Hz), 7.27 - 7.34 (5 H, m).

### [Reference Example 5-2]

### 4-(S)-(1-Ethoxycarbonylcyclopropyl)-3-(R)-fluoro-1-[1-(S)-phenylethyl]-2-eyrrolidinethione

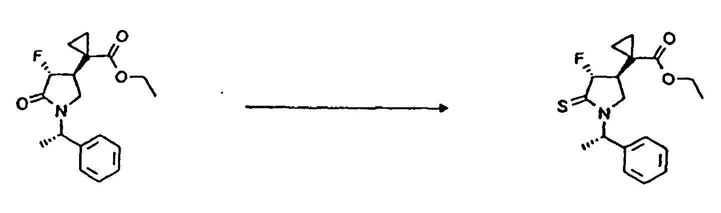

4-(S)-(1-Ethoxycarbonylcyclopropyl)-3-(R)-fluoro-1-[1-(S)-phenylethyl]-2-pyrrolidone (4.825 g, 15.11 mmol) was dissolved in dry benzene (150 ml), and the solution was mixed with Lawesson reagent (3.085 g, 7.625 mmol) and heated under reflux for 30 minutes. After cooling, benzene was evaporated under a reduced pressure, and the resulting residue was applied to a flash silica gel chromatography and eluted with an eluant of n-hexane:ethyl acetate = 5:1, thereby obtaining 4.494 g (88.7%) of the title compound as a light yellow oil.
¹H-NMR (400 MHz, CDCl₃) δ: 0.75 - 0.82 (1 H, m), 0.88 - 0.93 (1 H, m), 1.11 (3 H, t, J = 7.33 Hz), 1.25 - 1.34 (2 H, m), 1.64 (3 H, d, J = 7.33 Hz), 2.28 (1 H, dq, J = 26.86, 8.30 Hz), 3.12 - 3.18 (1 H, m), 3.72 (1 H, dd, J = 11.23, 9.28 Hz), 3.92 - 4.08 (2 H, m), 5.22 (1 H, dd, J = 53.22, 7.81 Hz), 6.33 (1 H, q, J = 7.33 Hz), 7.28 - 7.38 (5 H, m).

### [Reference Example 5-3]

### 4-(R)-(1-Ethoxycarbonylcyclopropyl)-3-(S)-fluoro-1-[1-(S)-phenylethyl]pyrrolidine

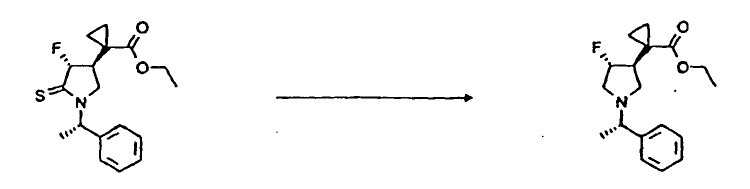

4-(S)-(1-Ethoxycarbonylcyclopropyl)-3-(R)-fluoro-1-[1-(S)-phenylethyl]-2-pyrrolidinethione (4.401 g, 13.12 mmol) was dissolved in anhydrous ethanol (150 ml), and the solution was mixed with Raney nickel (13 ml) and stirred at room temperature for 1 hour. After removing the catalyst by celite filtration (ethanol washing), the resulting filtrate was concentrated under a reduced pressure. The thus obtained residue was dissolved in diethyl ether (250 ml), washed with 10% ammonia water (100 ml x 5) and saturated brine (100 ml) in that order and then dried over anhydrous magnesium sulfate. After filtration, the filtrate was concentrated under a reduced pressure and the resulting residue was applied to a flash silica gel chromatography and eluted with an eluant of n-hexane:ethyl acetate = 4:1, thereby obtaining 3.794 g (94.7%) of the title compound as a colorless oil.
¹H-NMR (400 MHz, CDCl₃) δ: 0.66 - 0.71 (1 H, m), 0.83 - 0.88 (1 H, m), 1.19 (3 H, t, J = 7.33 Hz), 1.28 - 1.44 (2 H, m), 1.37 (3 H, d, J = 6.84 Hz), 2.02 (1 H, dm, J = 29.30 Hz), 2.10 (1 H, q, J = 9.28 Hz), 2.67 (1 H, ddd, J = 33.20, 11.23, 5.37 Hz), 2.80 (1 H, t, J = 7.82 Hz), 3.17 (1 H, q, J = 6.84 Hz), 3.33 (1 H, dd, J = 22.95, 11.23 Hz), 4.06 (2 H, q, J = 7.33 Hz), 5.16 (1 H, dd, J = 56.65, 3.41 Hz), 7.21 - 7.34 (5 H, m).

### [Reference Example 5-4]

### 1-Benzyloxycarbonyl-4-(R)-(1-ethoxycarbonylcyclopropyl)-3-(S)-fluoropyrrolidine

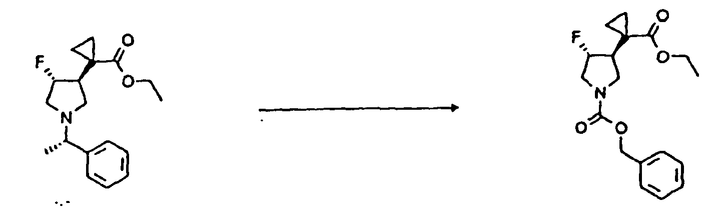

4-(R)-(1-Ethoxycarbonylcyclopropyl)-3-(S)-fluoro-1-[1-(S)-phenylethyl]pyrrolidine (3.786 g, 12.40 mmol) was dissolved in dry dichloromethane (120 ml), and benzyl chloroformate (3.37 ml, 25.0 mmol) was added dropwise to the thus prepared solution which was cooled in an ice bath. After 25 hours of stirring of the reaction solution at room temperature, dichloromethane was evaporated under a reduced pressure. Thereafter, the resulting residue was applied to a flash silica gel chromatography and eluted with an eluant of n-hexane:ethyl acetate = 4:1, thereby obtaining 3.718 g (89.4%) of the title compound as a colorless oil.
¹H-NMR (400 MHz, CDCl₃) δ: 0.71 - 0.78 (1 H, m), 0.90 - 0.95 (1 H, m), 1.23 (3 H, t, J = 6.83 Hz), 1.19 - 1.25 (1 H, m), 1.28 - 1.32 (1 H, m), 2.48 (1 H, dm, J = 28.32 Hz), 3.27 (1 H, t, J = 10.25 Hz), 3.67 (1 H, dd, J = 23.93, 13.19 Hz), 3.80 - 3.92 (2 H, m), 4.11 (2 H, q, J = 6.83 Hz), 5.14 (2 H, s), 5.17 (1 H, brd, J = 55.17 Hz), 7.29 - 7.35 (5 H, m).

### [Reference Example 5-5]

### 1-[1-Benzyloxycarbonyl-4-(R)-fluoro-3-(S)-pyrrolidinyl]cyclopropanecarboxylic acid

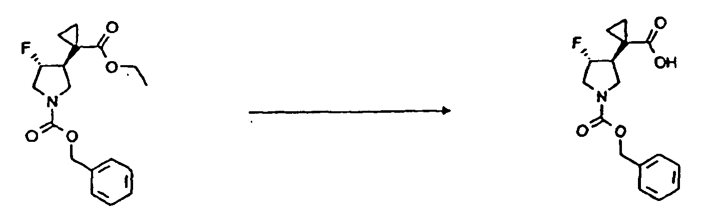

1-Benzyloxycarbonyl-4-(R)-(1-ethoxycarbonylcyclopropyl)-3-(S)-fluoropyrrolidine (3.715 g, 11.08 mmol) was dissolved in ethanol (110 ml), and 10 N sodium hydroxide aqueous solution (11 ml) was added dropwise to the thus prepared solution which was cooled in an ice bath. The reaction solution was stirred at room temperature for 18 hours and then ethanol was evaporated under a reduced pressure. The thus obtained residue was mixed with water (50 ml) and washed with dichloromethane (50 ml x 2). The thus separated aqueous layer was cooled in an ice bath, acidified by adding dropwise concentrated hydrochloric acid, extracted with diethyl ether (100 ml x 5) and then dried over anhydrous magnesium sulfate. After filtration, the filtrate was concentrated under a reduced pressure, and the residue was dissolved in benzene (100 ml) and again concentrated under a reduced pressure. This azeotropic step with benzene was repeated 3 times to obtain 3.346 g (98.3%) of the title compound as a colorless amorphous substance.
¹H-NMR (400 MHz, CDCl₃) δ: 0.84 - 0.89 (1 H, m), 0.99 - 1.07 (1 H, m), 1.32 - 1.42 (2 H, m), 2.37 - 2.56 (1 H, m), 3.26 - 3.31 (1 H, m), 3.58 - 3.67 (1 H, m), 3.82 - 3.88 (2 H, m), 5.13 (1 H, s), 5.20 (1 H, brd, J = 54.96 Hz), 7.30 - 7.34 (5 H, m).

### [Reference Example 5-6]

### 1-Benzyloxycarbonyl-4-(R)-(1-tert-butoxycarbonylaminocyclopropyl)-3-(R)-fluoropyrrolidine

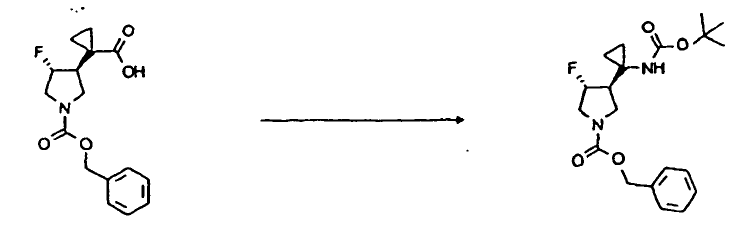

1-[1-Benzyloxycarbonyl-4-(R)-fluoro-3-(S)-pyrrolidinyl]cyclopropanecarboxylic acid (3.342 g, 10.87 mmol) was dissolved in tert-butyl alcohol (100 ml), and the solution was mixed with diphenylphosphoryl acid azide (2,398 µl, 11.11 mmol) and triethylamine (2,273 µl, 16.31 mmol), stirred at room temperature for 2 hours and then heated under reflux for 14 hours. The reaction solution was cooled and then concentrated under a reduced pressure, and the resulting residue was applied to a flash silica gel chromatography and eluted with an eluant of n-hexane:ethyl acetate = 3:1, thereby obtaining 2.682 g (65.2%) of the title compound as a colorless oil.
¹H-NMR (400 MHz, CDCl₃) δ: 0.64 - 0.70 (1 H, m), 0.79 - 0.83 (1 H, m), 0.86 - 1.09 (2 H, m), 1.39 (9 H, s), 2.21 (1 H, dm, J = 21.48 Hz), 3.44 (1 H, dd, J = 11.23, 2.93 Hz); 3.59 - 3.76 (3 H, m), 4.91 (1 H, brs), 5.14 (2 H, s), 5.40 (1 H, brd, J = 52.74 Hz), 7.28 - 7.33 (5 H, m).

### [Inventive Example 5]

### 5-Amino-7-[4-(R)-(1-aminocyclopropyl)-3-(R)-fluoro-1-pyrrolidinyl]-6-fluoro-1-[2-(S)-fluoro-1-(R)-cyclopropyl]-1,4-dihydro-8-methyl-4-oxoquinoline-3-carboxylic acid hydrochloride

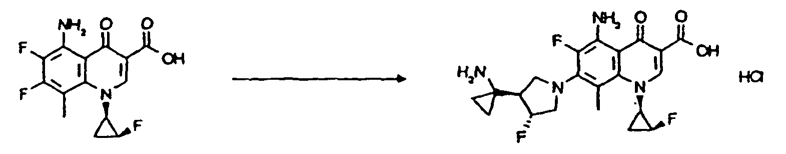

1-Benzyloxycarbonyl-4-(R)-(1-tert-butoxycarbonylaminocyclopropyl)-3-(R)-fluoropyrrolidine (757.8 mg, 2.002 mmol) was dissolved in methanol (80 ml), and the solution was mixed with 5% palladium-carbon catalyst (water content, 55.6%; 800 mg) and stirred for 7 hours under a pressured hydrogen atomosphere (4.5 kg/cm²). The catalyst was removed by celite filtration (methanol washing), and the filtrate was concentrated under a reduced pressure. The thus obtained residue was dissolved in dimethyl sulfoxide (8 ml), and the solution was mixed with 5-amino-6,7-difluoro-1-[2-(S)-fluoro-1-(R)-cyclopropyl]-1,4-dihydro-8-methyl-4-oxoquinoline-3-carboxylic acid (404.7 mg, 1.296 mmol) and triethylamine (3 ml) and stirred for 4 days in an oil bath of 120°C under an atmosphere of nitrogen. After cooling, dimethyl sulfoxide was evaporated under a reduced pressure, the thus obtained residue was dissolved in chloroform (150 ml) and washed with 10% citric acid aqueous solution (100 ml x 2) and saturated brine (100 ml) in that order and then the organic layer was dried over anhydrous magnesium sulfate. After filtration, the filtrate was concentrated under a reduced pressure. To the thus obtained residue, which was cooled in an ice bath, was added dropwise concentrated hydrochloric acid (10 ml), followed by 15 minutes of stirring at room temperature. After adding water (10 ml) to the reaction solution, the aqueous solution was washed with dichloromethane (30 ml x 4), adjusted to pH 7.4 with sodium hydroxide aqueous solution and then extracted with chloroform (100 ml x 4). The organic layers were combined, dried over anhydrous magnesium sulfate and then filtered, and the filtrate was concentrated under a reduced pressure. The thus obtained residue was applied to a preparative silica gel thin layer chromatography (development with the bottom layer of a mixture solvent of chloroform:methanol:water = 7:3:1), and then the thus obtained crude product was dissolved in ethanol (20 ml). 1 N hydrochloric acid (1.5 ml) was added dropwise thereto under ice-cooling, and the resulting reaction solution was stirred for 5 minutes at the same temperature and then concentrated under a reduced pressure (3 times of ethanol azeotropic treatment). Thereafter, the resulting residue was purified by recrystallization from ethanol-diisopropyl ether and then dried under a reduced pressure to obtain 141.8 mg (22.3%) of the title compound as a yellow powder.
Melting point: 220.2 - 224.9°C (decomposition)
¹H-NMR (400 MHz, 0.1. N NaOD) δ: 0.58 - 0.68 (4 H, m), 1.11 - 1.25 (1 H, m), 1.52 - 1.59 (1 H, m), 2.41 (3 H, s), 2.39 - 2.49 (1 H, m), 3.39 (1 H, t, J = 9.27 Hz), 3.58 - 3.67 (1 H, m), 3.71 - 3.83 (2 H, m), 3.88 - 3.99 (1 H, m), 4.96 (1 H, dm, J = 65.86 Hz), 5.49 (1 H, brd, J = 54.69 Hz), 8.27 (1 H, d, J = 3.41 Hz).

**Elemental analysis data; for C₂₁H₂₃F₃N₄O₃•HCl•H₂O:**

| | |
|---|---|
| calcd.; | C, 51.38; H, 5.34; N, 11.41 |
| found ; | C, 51.21; H, 5.38; N, 11.22 |

### [Inventive Example 6]

### 10-[4-(R)-(1-Aminocyclopropyl)-3-(R)-fluoro-1-pyrrolidinyl]-9-fluoro-2,3-dihydro-3-(S)-methyl-7-oxo-7H-pyrido[1.2.3-de][1.4]benzoxazine-6-carboxylic acid

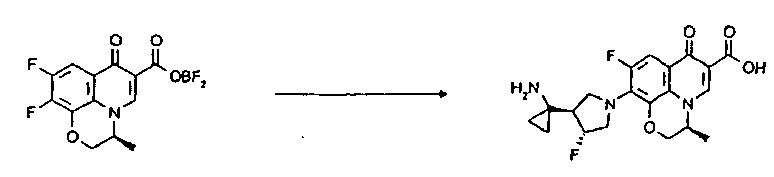

1-Benzyloxycarbonyl-4-(R)-(1-tert-butoxycarbonylaminocyclopropyl)-3-(R)-fluoropyrrolidine (759.9 mg, 2.008 mmol) was dissolved in methanol (80 ml), and the solution was mixed with 5% palladium-carbon catalyst (water content, 55.6%; 800 mg) and stirred for 7 hours under a pressured hydrogen atmosphere (4.5 kg/cm²). The catalyst was removed by celite filtration (methanol washing), and the filtrate was concentrated under a reduced pressure. The thus obtained residue was dissolved in dimethyl sulfoxide (8 ml), and the solution was mixed with 9,10-difluoro-2,3-dihydro-3-(S)-methyl-7-oxo-7H-pyrido[1.2.3-de][1.4]benzoxazine-6-carboxylic acid-BF₂ chelate (440.9 mg, 1.340 mmol) and triethylamine (374 µl, 2.68 mmol) and stirred at room temperature for 20 hours. After concentration of the reaction solution under a reduced pressure, the resulting residue was mixed with water, and the thus precipitated yellow crystals were collected by filtration and washed with water. The thus obtained crystals were suspended in a solution of methanol:water = 9:1 (20 ml), and the suspension was mixed with triethylamine (1 ml) and heated under reflux for 4 hours. After cooling, the reaction solution was concentrated under a reduced pressure; and the thus obtained residue was dissolved in chloroform (100 ml) and washed with 10% citric acid aqueous solution (100 ml x 2) and saturated brine (100 ml) in that order and then the organic layer was dried over anhydrous magnesium sulfate. After filtration, the filtrate was concentrated under a reduced pressure. To the thus obtained residue, which was cooled in an ice bath, was added dropwise concentrated hydrochloric acid (10 ml), followed by 15 minutes of stirring at room temperature. After adding water (10 ml) to the reaction solution, the aqueous solution was washed with dichloromethane (30 ml x 2), adjusted to pH 7.2 with sodium hydroxide aqueous solution and then extracted with chloroform (100 ml x 4). The organic layers were combined, dried over anhydrous magnesium sulfate and then filtered, and the filtrate was concentrated under a reduced pressure. Thereafter, the resulting residue was purified by recrystallization from a mixture of ethanol and 28% ammonia water, and then dried under a reduced pressure to obtain 370.8 mg (67.5%) of the title compound as light yellow crystals.
Melting point: 240.6 - 243.4°C (decomposition)
¹H-NMR (400 MHz, 0.1 N NaOD) δ: 0.59 - 0.68 (4 H, m), 1.52 (3 H, d, J = 6.84 Hz), 2.39 (1 H, dt, J = 29.30, 7.81 Hz), 3.37 (1 H, t, J = 7.81 Hz), 3.74 - 3.90 (3 H, m), 3.95 (1 H, t, J = 9.76 Hz), 4.36 (1 H, d, J = 10.26 Hz), 4.53 (1 H, d, J = 11.23 Hz), 4.62 (1 H, q, J = 6.84 Hz), 5.34 (1 H, brd, J = 54.20 Hz), 7.57 (1 H, d, J = 13.67 Hz), 8.35 (1 H, s).

**Elemental analysis data; for C₂₀H₂₁F₂N₃O₄•0.25H₂O:**

| | | | |
|---|---|---|---|
| calcd.; | C, 58.60; | H, 5.29; | N, 10.25 |
| found ; | C, 58.42; | H, 5.35; | N, 10.01 |

### [Reference Example 6-1]

### 4-(S)-(1-Ethoxycarbonylcyclopropyl)-3,3-difluoro-1-[1-(S)-phenylethyl]-2-pyrrolidone

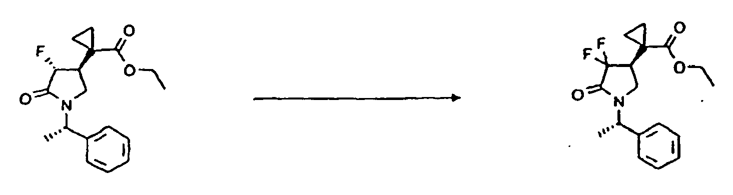

Under an atmosphere of nitrogen, diisopropylamine (2.49 ml, 19.0 mmol) was dissolved in anhydrous tetrahydrofuran (25 ml) to which, after cooling to -78°C, was subsequently added dropwise n-hexane solution of 1.68 M n-butyl lithium (11.2 ml, 18.8 mmol) in 10 minutes. After 20 minutes of stirring at -10°C and subsequent cooling to -78°C, to the resulting reaction solution was added dropwise anhydrous tetrahydrofuran solution (15 ml) of 4-(S)-(1-ethoxycarbonylcyclopropyl)-3-(R)-fluoro-1-[1-(S)-phenylethyl]-2-pyrrolidone (5.011 g, 15.69 mmol) in 15 minutes. After 30 minutes of stirring at -78°C, to the reaction solution cooled at the same temperature was added dropwise anhydrous tetrahydrofuran solution (35 ml) of N-fluorobenzene disulfonimide (7.421 g, 23.54 mmol) in 20 minutes. The reaction solution was stirred at -78°C for 2 hours and then at room temperature for 1 hour. While cooling in an ice bath, the reaction solution was mixed with saturated ammonium chloride aqueous solution (100 ml), the organic layer was separated and then the water layer was extracted with diethyl ether (100 ml x 3). The organic layers were combined, washed with water (100 ml x 2) and then dried over anhydrous magnesium sulfate. After filtration, the filtrate was concentrated under a reduced pressure, and the resulting residue was applied to a flash silica gel chromatography and eluted with an eluant of n-hexane:ethyl acetate = 4:1, thereby obtaining 3.637 g (68.7%) of the title compound as a colorless oil.
¹H-NMR (400 MHz, CDCl₃) δ: 0.76 - 0.82 (1 H, m), 0.87 - 0.94 (1 H, m), 1.09 (3 H, t, J = 6.83 Hz), 1.23 - 1.36 (2 H, m), 1.58 (3 H, d, J = 7.33 Hz), 2.56 - 2.69 (1 H, m), 2.92 - 2.98 (1 H, m), 3.53 (1 H, td, J = 10.93, 2.91 Hz), 3.84 - 3.92 (1 H, m), 4.02 - 4.10 (1 H, m), 5.53 (1 H, q, J = 7.33 Hz), 7.28 - 7.35 (5 H, m).

### [Reference Example 6-2]

### 4-(S)-(1-Ethoxycarbonylcyclopropyl)-3,3-difluoro-1-[1-(S)-phenylethyl]-2-pyrrolidinethione

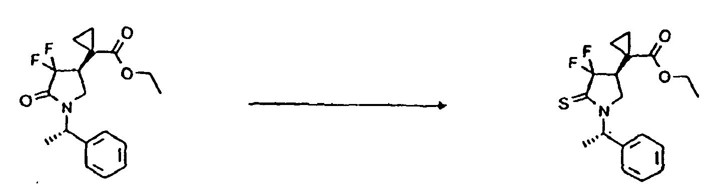

4-(S)-(1-Ethoxycarbonylcyclopropyl)-3,3-difluoro-1-[1-(S)-phenylethyl]-2-pyrrolidone (3.621 g, 10.73 mmol) was dissolved in dry benzene (100 ml), and the solution was mixed with Lawesson reagent (2.192 g, 5.420 mmol) and heated under reflux for 1 hour. After cooling, benzene was evaporated under a reduced pressure, and the resulting residue was applied to a flash silica gel chromatography and eluted with an eluant of n-hexane:ethyl acetate = 5:1, thereby obtaining 2.886 g (76.1%) of the title compound as a light yellow oil.
¹H-NMR (400 MHz, CDCl₃) δ: 0.85 - 0.95 (2 H, m), 1.10 (3 H, t, J = 6.84 Hz), 1.24 - 1.32 (2 H, m), 1.64 (3 H, d, J = 7.33 Hz), 2.69 - 2.81 (1 H, m), 3.20 (1 H, ddd, J = 11.72, 6.84, 2.93 Hz), 3.73 (1 H, td, J = 10.26, 2.54 Hz), 3.84 - 3.92 (1 H, m), 4.02 - 4.11 (1 H, m), 6.31 (1 H, q, J = 7.33 Hz), 7.32 - 7.38 (5 H, m).

### [Reference Example 6-3]

### 4-(R)-(1-Ethoxycarbonylcyclopropyl)-3,3-difluoro-1-[1-(S)-phenylethyl]pyrrolidine

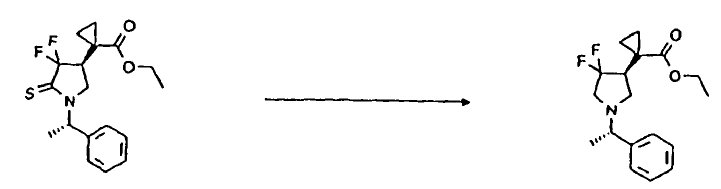

4-(S)-(1-Ethoxycarbonylcyclopropyl)-3,3-difluoro-1-[1-(S)-phenylethyl]-2-pyrrolidinethione (2.883 g, 8.157 mmol) was dissolved in anhydrous ethanol (80 ml), and the solution was mixed with Raney nickel (8 ml) and stirred at room temperature for 30 minutes. After removing the catalyst by celite filtration (ethanol washing), the resulting filtrate was concentrated under a reduced pressure. The thus obtained residue was dissolved in diethyl ether (150 ml), washed with 10% ammonia water (100 ml x 4) and saturated brine (100 ml) in that order and then dried over anhydrous magnesium sulfate. After filtration, the filtrate was concentrated under a reduced pressure and the resulting residue was applied to a flash silica gel chromatography and eluted with an eluant of n-hexane:ethyl acetate = 4:1, thereby obtaining 2.540 g (96.3%) of the title compound as a colorless oil.
¹H-NMR (400 MHz, CDCl₃) δ: 0.67 - 0.89 (2 H, m), 1.19 (3 H, t, J = 7.33 Hz), 1.27 - 1.46 (2 H, m), 1.38 (3 H, d, J = 7.33 Hz), 2.34 - 2.62 (2 H, m), 2.68 - 2.96 (2 H, m), 3.20 (1 H, q, J = 7.33 Hz), 3.52 - 3.48 (1 H, m), 3.94 - 4.09 (2 H, m), 7.28 - 7.34 (5 H, m).

### [Reference Example 6-4]

### 1-Benzyloxycarbonyl-4-(R)-(1-ethoxycarbonylcyclopropyl)-3,3-difluoropyrrolidine

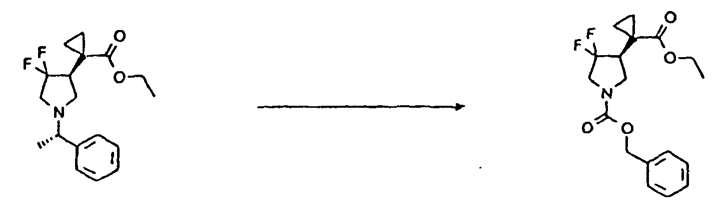

4-(R)-(1-Ethoxycarbonylcyclopropyl)-3,3-difluoro-1-[1-(S)-phenylethyl]pyrrolidine (2.536 g, 7.842 mmol) was dissolved in dry dichloromethane (80 ml), and benzyl chloroformate (2.80 ml, 19.6 mmol) was added dropwise to the thus prepared solution which was cooled in an ice bath. The reaction solution was stirred at room temperature for 44 hours and then dichloromethane was evaporated under a reduced pressure. Thereafter, the resulting residue was applied to a flash silica gel chromatography and eluted with an eluant of n-hexane:ethyl acetate = 4:1, thereby obtaining 2.294 g (82.8%) of the title compound as a colorless oil.
¹H-NMR (400 MHz, CDCl₃) δ: 0.97 - 1.05 (1 H, m), 1.07 - 1.16 (1 H, m), 1.22 (3 H, t, J = 7.33 Hz), 1.20 - 1.30 (1 H, m), 1.32 - 1.42 (1 H, m), 2.93 - 3.07 (1 H, m), 3.36 - 3.44 (1 H, m), 3.77 - 3.84 (2 H, m), 3.93 (1 H, t, J = 10.74 Hz), 4.12 (2 H, qd, J = 7.33, 1.47 Hz), 5.14 (2 H, s), 7.28 - 7.35 (5 H, m).

### [Reference Example 6-5]

### 1-(1-Benzyloxycarbonyl-4,4-difluoro-3-(S)-pyrrolidinyl]cyclopropanecarboxylic acid

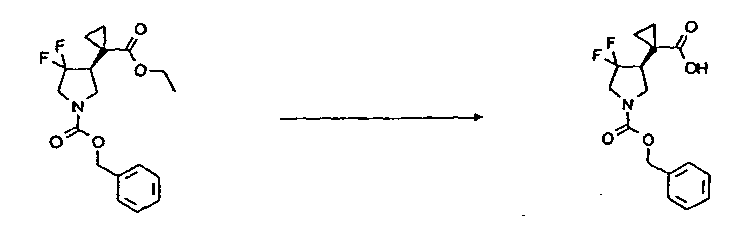

1-Benzyloxycarbonyl-4-(R)-(1-ethoxycarbonylcyclopropyl)-3,3-difluoropyrrolidine (2.287 g, 6.472 mmol) was dissolved in ethanol (65 ml) to which, while cooling in an ice bath, was added dropwise 10 N sodium hydroxide aqueous solution (6.5 ml). The reaction solution was stirred at room temperature for 16 hours and then ethanol was evaporated under a reduced pressure. The thus obtained residue was mixed with water (50 ml) and washed with dichloromethane (50 ml x 2), and the thus separated aqueous layer was cooled in an ice bath, acidified by adding dropwise concentrated hydrochloric acid and then extracted with diethyl ether (100 ml x 5), subsequently drying the extract over anhydrous magnesium sulfate. After filtration, the filtrate was concentrated under a reduced pressure, and the thus obtained residue was dissolved in benzene (100 ml) and again concentrated under a reduced pressure. This azeotropic treatment with benzene was repeated 3 times to obtain 1.956 g (92.9%) of the title compound as a colorless oil.
¹H-NMR (400 MHz, CDCl₃) δ: 1.08 - 1.14 (1 H, m), 1.19 - 1.28 (1 H, m), 1.37 - 1.42 (1 H, m), 1.44 - 1.49 (1 H, m), 2.93 - 3.09 (1 H, m), 3.37 - 3.46 (1 H, m), 3.76 - 3.85 (2 H, m), 3.92 - 4.00 (1 H, m), 5.14 (2 H, s), 7.29 - 7.34 (5 H, m).

### [Reference Example 6-6]

### 1-Benzyloxycarbonyl-4-(R)-(1-tert-butoxycarbonylaminocyclopropyl)-3,3-difluoropyrrolidine

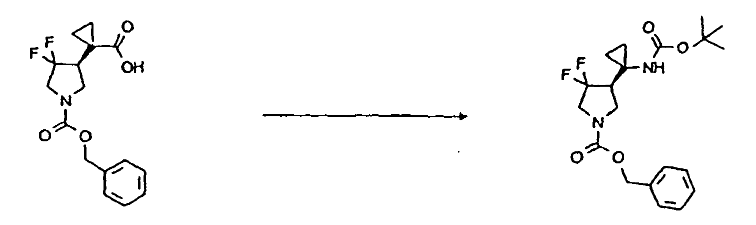

1-[1-Benzyloxycarbonyl-4,4-difluoro-3-(S)-pyrrolidinyl]cyclopropanecarboxylic acid (1.953 g, 6.004 mmol) was dissolved in tert-butyl alcohol (50 ml), and the solution was mixed with diphenylphosphoryl azide (1,426 µl, 6.604 mmol) and triethylamine (1,381 µl, 9.906 mmol), stirred at room temperature for 2 hours and then heated under reflux for 16 hours. The reaction solution was cooled and then concentrated under a reduced pressure, and the resulting residue was applied to a flash silica gel chromatography and eluted with an eluant of n-hexane:ethyl acetate = 4:1, thereby obtaining 1.430 g (60.1%) of the title compound as a colorless oil.
¹H-NMR (400 MHz, CDCl₃) δ: 0.83 - 0.92 (2 H, m), 1.40 (9 H, s), 1.34 - 1.55 (2 H, m), 2.38 - 2.51 (1 H, m), 3.47 (1 H, t, J = 9.28 Hz), 3.67 - 3.84 (2 H, m), 4.99 (1 H, brs), 5.13 (2 H, s), 7.29 - 7.35 (5 H, m).

### [Inventive Example 7]

### 5-Amino-7-[4-(R)-(1-aminocyclopropyl)-3,3-difluoro-1-pyrrolidinyl]-6-fluoro-1-[2-(S)-fluoro-1-(R)-cyclopropyl]-1,4-dihydro-8-methyl-4-oxoquinoline-3-carboxylic acid hydrochloride

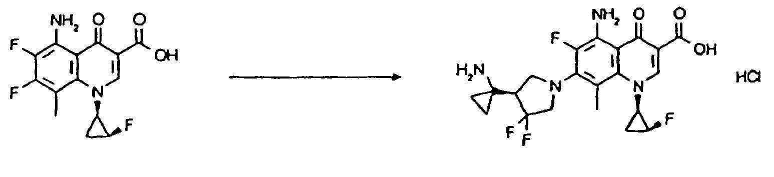

1-Benzyloxycarbonyl-4-(R)-(1-tert-butoxycarbonylaminocyclopropyl)-3,3-difluoropyrrolidine (792.4 mg, 1.999 mmol) was dissolved in methanol (80 ml), and the solution was mixed with 5% palladium-carbon catalyst (water content, 55.6%; 800 mg) and stirred for 6 hours under a pressured hydrogen atmosphere (4.5 kg/cm²). The catalyst was removed by celite filtration (methanol washing), and the filtrate was concentrated under a reduced pressure. The thus obtained residue was dissolved in dimethyl sulfoxide (8 ml), and the solution was mixed with 5-amino-6,7-difluoro-1-[2-(S)-fluoro-1-(R)-cyclopropyl]-1,4-dihydro-8-methyl-4-oxoquinoline-3-carboxylic acid (416.2 mg, 1.333 mmol) and triethylamine (3 ml) and stirred for 5 days in an oil bath of 120°C under an atmosphere of nitrogen. After cooling, dimethyl sulfoxide was evaporated under a reduced pressure, the thus obtained residue was dissolved in chloroform (150 ml) and washed with 10% citric acid aqueous solution (100 ml x 2) and saturated brine (100 ml) in that order and then the organic layer was dried over anhydrous magnesium sulfate. After filtration, the filtrate was concentrated under a reduced pressure. To the thus obtained residue, which was cooled in an ice bath, was added dropwise concentrated hydrochloric acid (10 ml), followed by 15 minutes of stirring at room temperature. After adding water (10 ml) to the reaction solution, the aqueous solution was washed with dichloromethane (30 ml x 5), adjusted to pH 7.4 with sodium hydroxide aqueous solution and then extracted with chloroform (100 ml x 4). The organic layers were combined, dried over anhydrous magnesium sulfate and then filtered, and the filtrate was concentrated under a reduced pressure. The thus obtained residue was applied to a preparative silica gel thin layer chromatography (development with the bottom layer of a mixture of chloroform:methanol:water = 7:3:1), and the thus obtained crude product was dissolved in ethanol (20 ml). While cooling in an ice bath, 1 N hydrochloric acid (1.5 ml) was added dropwise thereto, and the reaction solution was stirred at the same temperature for 5 minutes and then concentrated under a reduced pressure (three times of ethanol azeotropic treatment). Thereafter, the resulting residue was purified by recrystallization from an ethanol-diisopropyl ether and then dried under a reduced pressure to obtain 137.4 mg (19.9%) of the title compound as a yellow powder.
Melting point: 211.2 - 215.4°C (decomposition)
¹H-NMR (400 MHz, 0.1 N NaOD) δ: 0.59 - 0.71 (4 H, m), 1.08 - 1.20 (1 H, m), 1.48 - 1.57 (1 H, m), 2.30 (3 H, s), 2.25 - 2.33 (1 H, m), 3.37 - 2.54 (1 H, m), 3.88 (1 H, t, J = 9.28 Hz), 3.90 - 3.95 (1 H, m), 3.97 - 4.04 (1 H, m), 4.96 (1 H, dm, J = 65.92 Hz), 8.25 (1 H, d, J = 2.93 Hz).

**Elemental analysis data; for C₂₁H₂₂F₄N₄O₃•HCl•1.5H₂O:**

| | | | |
|---|---|---|---|
| calcd.; | C, 48.70; H, 5.05; N, 10.82 | | |
| found ; | C, 48.58; H, 5.11; N, 10.66 | | |

### [Inventive Example 8]

### 10-[4-(R)-(1-Aminocyclopropyl)-3,3-difluoro-1-pyrrolidinyl]-9-fluoro-2,3-dihydro-3-(S)-methyl-7-oxo-7H-pyrido[1.2.3-de][1.4]benzoxazine-6-carboxylic acid

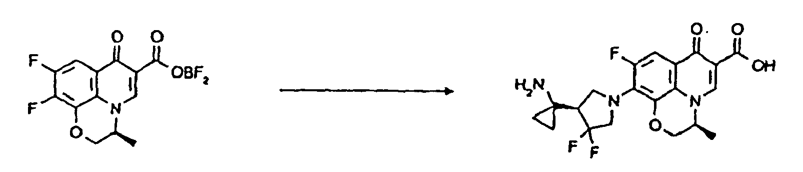

1-Benzyloxycarbonyl-4-(R)-(1-tert-butoxycarbonylaminocyclopropyl)-3,3-difluoropyrrolidine (628.8 mg, 1.586 mmol) was dissolved in methanol (60 ml), and the solution was mixed with 5% palladium-carbon catalyst (water content, 55.6%; 650 mg) and stirred for 7 hours under a pressured hydrogen atomosphere (4.5 kg/cm²). The catalyst was removed by celite filtration (methanol washing), and the filtrate was concentrated under a reduced pressure. The thus obtained residue was dissolved in dimethyl sulfoxide (8 ml), and the solution was mixed with 9,10-difluoro-2,3-dihydro-3-(S)-methyl-7-oxo-7H-pyrido[1.2.3-de][1.4]benzoxazine-6-carboxylic acid-BF₂ chelate (347.9 mg, 1.057 mmol) and triethylamine (294 µl, 2.11 mmol) and stirred at room temperature for 41 hours. After concentration of the reaction solution under a reduced pressure, the resulting residue was mixed with water, and the thus precipitated yellow crystals were collected by filtration and washed with water. The thus obtained crystals were suspended in a solution of methanol:water = 9:1 (20 ml), and the suspension was mixed with triethylamine (1 ml) and heated under reflux for 5 hours. After cooling, the reaction solution was concentrated under a reduced pressure, and the thus obtained residue was dissolved in chloroform (100 ml) and washed with 10% citric acid aqueous solution (100 ml x 2) and saturated brine (100 ml) in that order and then the organic layer was dried over anhydrous magnesium sulfate. After filtration, the filtrate was concentrated under a reduced pressure. To the thus obtained residue, which was cooled in an ice bath, was added dropwise concentrated hydrochloric acid (10 ml), followed by 15 minutes of stirring at room temperature. After adding water (10 ml) to the reaction solution, the aqueous solution was washed with dichloromethane (30 ml x 3), adjusted to pH 7.2 with sodium hydroxide aqueous solution and then extracted with chloroform (100 ml x 4). The organic layers were combined, dried over anhydrous magnesium sulfate and then filtered, and the filtrate was concentrated under a reduced pressure. Thereafter, the resulting residue was purified by recrystallization from a mixture of ethanol and 28% ammonia water, and then dried under a reduced pressure to obtain 183.8 mg (41.1%) of the title compound as light yellow crystals.
Melting point: 246.7 - 248.0°C (decomposition)
¹H-NMR (400 MHz, 0.1 N NaOD) δ: 0.61 - 0.72 (4 H, m), 1.53 (3 H, d, J = 6.83 Hz), 2.36 - 2.45 (1 H, m), 3.74 - 3.94 (3 H, m), 4.08 - 4.14 (1 H, m), 4.37 (1 H, d, J = 10.74 Hz), 4.53 (1 H, d, J = 10.74 Hz), 4.61 - 4.64 (1 H, m), 7.60 (1 H, d, J = 13.68 Hz), 8.36 (1 H, s).

**Elemental analysis data; for C₂₀H₂₀F₃N₃O₄:**

| | | | |
|---|---|---|---|
| calcd.; | C, 56.74; H, 4.76; N, 9.92 | | |
| found ; | C, 56.72; H, 4.66; N, 9.74 | | |

### [Reference Example 7-1]

### 4-(S)-(1-Ethoxycarbonylcyclopropyl)-3-(S)-fluoro-1-[1-(S)-phenylethyl]-2-pyrrolidone

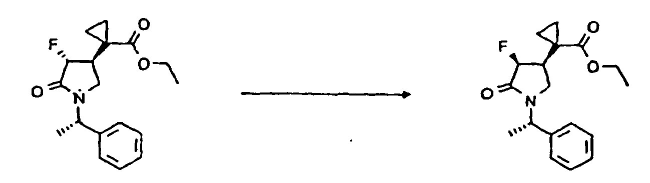

Under an atmosphere of nitrogen, diisopropylamine (7.22 ml, 51.52 mmol) was dissolved in anhydrous tetrahydrofuran (100 ml) to which, after cooling to -78°C, was subsequently added dropwise n-hexane solution of 1.68 M n-butyl lithium (28.1 ml, 47.21 mmol) in 15 minutes. After 10 minutes of stirring at 0°C and subsequent cooling to - 78°C, to the resulting reaction solution was added dropwise anhydrous tetrahydrofuran solution (40 ml) of 4-(S)-(1-ethoxycarbonylcyclopropyl)-3-(R)-fluoro-1-[1-(S)-phenylethyl]-2-pyrrolidone (13.72 g, 42.96 mmol) in 20 minutes. After additional 20 minutes of stirring at -78°C, to the reaction solution was added dropwise 2,6-di-tertbutylphenol (10.63 g, 51.52 mmol) dissolved in anhydrous tetrahydrofuran (40 ml), in 20 minutes. The reaction solution was stirred at -78°C for 10 minutes and then warmed up to room temperature. While cooling in an ice bath, the resulting reaction solution was mixed with saturated ammonium chloride aqueous solution (200 ml), the organic layer was separated and then the aqueous layer was extracted with diethyl ether (200 ml x 2). The organic layers were combined, washed with water (400 ml x 2) and then dried over anhydrous sodium sulfate. After filtration, the filtrate was concentrated under a reduced pressure, and the resulting residue was applied to a flash silica gel chromatography and eluted with an eluant of n-hexane:ethyl acetate = 3:1, thereby obtaining 10.19 g (74.2%) of the title compound as a colorless oil.
¹H-NMR (400 MHz, CDCl₃) δ: 0.57 - 0.63 (1 H, m), 0.78 - 0.84 (1 H, m), 1.07 - 1.13 (1 H, m), 1.26 (3 H, t, J = 7.09 Hz), 1.23 - 1.29 (1 H, m), 1.54 (3 H, d, J = 7.32 Hz), 2.59 (1 H, t, J = 9.77 Hz), 3.05 (1 H, dq, J = 28.81, 8.30 Hz), 3.25 (1 H, t, J = 9.77 Hz), 4.00 - 4.16 (2 H, m), 5.15 (1 H, dd, J = 52.73, 6.35 Hz), 5.53 (1 H, q, J = 7.32 Hz), 7.27 - 7.38 (5 H, m).

### [Reference Example 7-2]

### 4-(S)-(1-Ethoxycarbonylcyclopropyl)-3-(S)-fluoro-1-[1-(S)-phenylethyl]-2-pyrrolidinethione

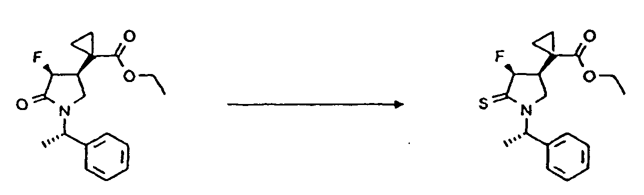

4-(S)-(1-Ethoxycarbonylcyclopropyl)-3-(S)-fluoro-1-[1-(S)-phenylethyl]-2-pyrrolidone (6.86 g, 21.48 mmol) was dissolved in dry toluene (100 ml), and the solution was mixed with Lawesson reagent (5.21 g, 12.89 mmol) and heated at 60°C for 30 minutes. After cooling, toluene was evaporated under a reduced pressure, and the resulting residue was applied to a flash silica gel chromatography and eluted with an eluant of n-hexane:ethyl acetate = 4:1, thereby obtaining 6.49 g (90.1%) of the title compound as a light yellow oil.
¹H-NMR (400 MHz, CDCl₃) δ: 0.59 - 0.66 (1 H, m), 0.86 - 0.92 (1 H, m), 1.08 - 1.15 (1 H, m), 1.20 (3 H, t, J = 7.33 Hz), 1.24 - 1.31 (1 H, m), 1.60 (3 H, d, J = 7.32 Hz), 2.85 (1 H, dd, J = 11.23, 9.28 Hz), 3.16 (1 H, dq, J = 30.27, 8.30 Hz), 3.50 (1 H, dd, J = 11.23, 9.28 Hz), 4.04 - 4.15 (2 H, m), 5.32 (1 H, dd, J = 52.73, 5.38 Hz), 6.28 - 6.34 (1 H, m), 7.30 - 7.41 (5 H, m).

### [Reference Example 7-3]

### 4-(S)-(1-Ethoxycarbonylcyclopropyl)-3-(S)-fluoro-1-[1-(S)-phenylethyl]pyrrolidine

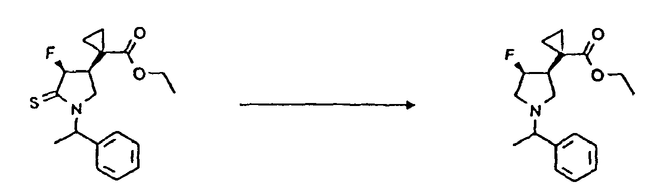

4-(S)-(1-Ethoxycarbonylcyclopropyl)-3-(S)-fluoro-1-[1-(S)-phenylethyl]-2-pyrrolidinethione (6.49 g, 19.35 mmol) was dissolved in anhydrous tetrahydrofuran (150 ml), and the solution was mixed with Raney nickel (15 ml) and stirred at room temperature for 30 minutes. After removing the catalyst by celite filtration (tetrahydrofuran washing), the resulting filtrate was concentrated under a reduced pressure. The thus obtained residue was dissolved in diethyl ether (200 ml), washed with 10% ammonia water (200 ml x 2) and saturated brine (150 ml) in that order and then dried over anhydrous sodium sulfate. After filtration, the filtrate was concentrated under a reduced pressure to obtain 5.08 g (86.0%) of the title compound as a colorless oil.
¹H-NMR (400 MHz, CDCl₃) δ: 0.54 - 0.60 (1 H, m), 0.95 - 1.08 (2 H, m), 1.22 (3 H, t, J = 7.33 Hz), 1.25 - 1.32 (1H, m), 1.35 (3 H, d, J = 6.35 Hz), 1.99 (1 H, t, J = 9.28 Hz), 2.42 (1 H, t, J = 8.30 Hz), 2.63 (1 H, ddd, J = 33.21, 11.72, 1.95 Hz), 2.99 (1 H, dm, J = 28.32 Hz), 3.25 - 3.37 (2 H, m), 4.03 - 4.16 (2 H, m), 5.33 (1 H, dm, J = 55.67 Hz), 7.21 - 7.36 (5 H, m).

### [Reference Example 7-4]

### 1-Benzyloxycarbonyl-4-(S)-(1-ethoxycarbonylcyclopropyl)-3-(S)-fluoropyrrolidine

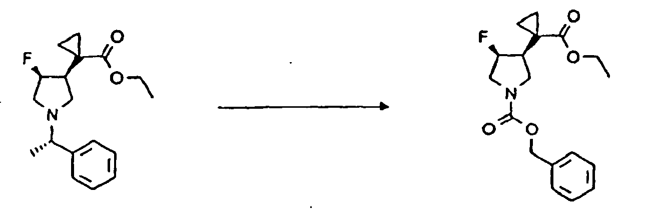

4-(S)-(1-Ethoxycarbonylcyclopropyl)-3-(S)-fluoro-1-[1-(S)-phenylethyl]pyrrolidine (5.08 g, 16.63 mmol) was dissolved in dry dichloromethane (50 ml), and benzyl chloroformate (3.56 ml, 25.0 mmol) was added dropwise to the thus prepared solution which was cooled in an ice bath. After 1 hour of heating of the reaction solution under reflux, dichloromethane was evaporated under a reduced pressure. Thereafter, the resulting residue was applied to a flash silica gel chromatography and eluted with an eluant of n-hexane:ethyl acetate = 3:1, thereby obtaining 4.67 g (83.7%) of the title compound as a colorless oil.
¹H-NMR (400 MHz, CDCl₃) δ: 0.71 - 0.78 (1 H, m), 1.11 - 1.23 (2 H, m), 1.24 (3 H, t, J = 6.84 Hz), 1.29 - 1.37 (1 H, m), 2.93 - 3.00 (1 H, m), 3.10 (1 H, dm, J = 34.67 Hz), 3.54 - 3.84 (2 H, m), 4.09 - 4.18 (2 H, m), 5.14 (2 H, s), 5.34 (1 H, ddm, J = 53.71, 16.6 Hz), 7.29 - 7.38 (5 H, m).

### [Reference Example 7-5]

### 1-[1-Benzyloxycarbonyl-4-(S)-fluoro-3-(S)-pyrrolidinyl]cyclopropanecarboxylic acid

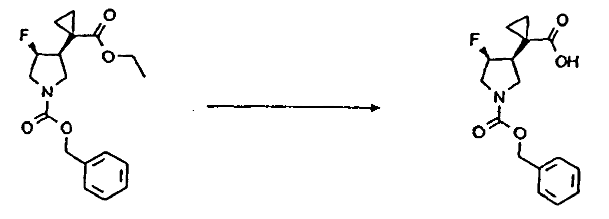

1-Benzyloxycarbonyl-4-(S)-(1-ethoxycarbonylcyclopropyl)-3-(S)-fluoropyrrolidine (4.67 g, 13.92 mmol) was dissolved in ethanol (50 ml), and 1 N sodium hydroxide aqueous solution (50 ml) was added dropwise to the resulting solution. The reaction solution was stirred at 40°C for 1.5 hours and then ethanol was evaporated under a reduced pressure. The resulting residue was mixed with water (50 ml) and washed with chloroform (100 ml), and the thus separated aqueous layer was acidified by adding dropwise 1 N hydrochloric acid and extracted with chloroform (200 ml x 2) and then with diethyl ether (100 ml). The organic layers were combined and dried over anhydrous sodium sulfate. After filtration, the filtrate was concentrated under a reduced pressure to obtain 3.94 g (92.1%) of the title compound as a colorless amorphous substance.
¹H-NMR (400 MHz, CDCl₃) δ: 0.79 - 0.89 (1 H, m), 1.18 - 1.35 (2 H, m), 1.37 - 1.47 (1 H, m), 2.90 - 3.18 (2 H, m), 3.50 - 3.84 (3 H, m), 5.13 (2 H, s), 5.31 (1 H, ddm, J = 53.22, 15.13 Hz), 7.26 - 7.42 (5 H, m).

### [Reference Example 7-6]

### 1-Benzyloxycarbonyl-4-(R)-(1-tert-butoxycarbonylaminocyclopropyl)-3-(S)-fluoropyrrolidine

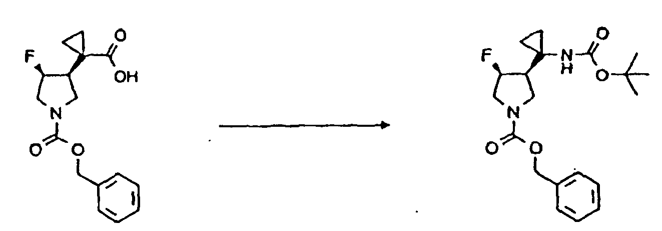

1-[1-Benzyloxycarbonyl-4-(S)-fluoro-3-(S)-pyrrolidinyl]cyclopropanecarboxylic acid (3.22 g, 10.48 mmol) was dissolved in anhydrous acetonitrile (80 ml), and the solution was mixed with N-N'-carbonyldiimidazole (2.55 g, 15.73 mmol) and stirred at room temperature for 30 minutes. Ammonia was bubbled into the reaction solution for 30 minutes at the same temperature. The reaction solution was concentrated under a reduced pressure. The thus obtained residue was mixed with water (80 ml) and extracted with chloroform (80 ml x 2), and the organic layers were combined and dried over anhydrous sodium sulfate. After filtration, the filtrate was concentrated under a reduced pressure. The resulting residue was dissolved in tert-butyl alcohol (100 ml), and the solution was mixed with lead tetraacetate (7.93 g, 15.70 mmol) and heated under reflux for 30 minutes. The reaction solution was cooled, mixed with diethyl ether (50 ml) and sodium bicarbonate (10 g) and then stirred at room temperature for 10 minutes. After filtration, the filtrate was concentrated under a reduced pressure. The thus obtained residue was mixed with ethyl acetate (150 ml), washed with saturated sodium bicarbonate aqueous solution and then dried over anhydrous sodium sulfate. After filtration, the filtrate was concentrated under a reduced pressure, and the resulting residue was applied to a flash silica gel chromatography and eluted with an eluant of n-hexane:ethyl acetate = 3:2, thereby obtaining 3.216 g (81.2%) of the title compound as a colorless oil.
¹H-NMR (400 MHz, CDCl₃) δ: 0.65 - 0.74 (1 H, m), 0.77 - 0.84 (1 H, m), 0.85 - 1.00 (2 H, m), 1.42 (9 H, s), 2.21 (1 H, ddm, J = 80.57, 36.14 Hz), 3.08 - 3.24 (2 H, m), 3.48 - 3.84 (3 H, m), 5.02 (1 H, brs), 5.13 (2 H, s), 5.15 (1 H, brd, J = 53.72 Hz), 7.28 - 7.38 (5 H, m).

### [Inventive Example 9]

### 5-Amino-7-[4-(R)-(1-aminocyclopropyl)-3-(S)-fluoro-1-pyrrolidinyl]-6-fluoro-1-[2-(S)-fluoro-1-(R)-cyclopropyl]-1,4-dihydro-8-methyl-4-oxoquinoline-3-carboxylic acid hydrochloride

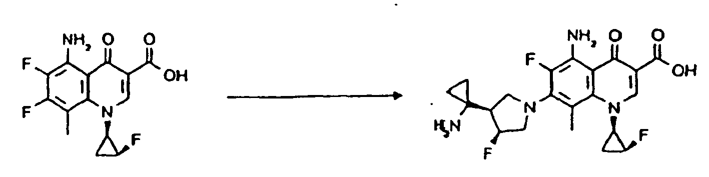

1-Benzyloxycarbonyl-4-(R)-(1-tert-butoxycarbonylaminocyclopropyl)-3-(S)-fluoropyrrolidine (1.43 g, 3.78 mmol) was dissolved in ethanol (60 ml), and the solution was mixed with 5% palladium-carbon catalyst (water content, 55.6%; 1.5 g) and stirred for 3 hours under an atmosphere of hydrogen. The catalyst was removed by celite filtration (methanol washing), and the filtrate was concentrated under a reduced pressure. The thus obtained residue was dissolved in dimethyl sulfoxide (12 ml), and the solution was mixed with 5-amino-6,7-difluoro-1-[2-(S)-fluoro-1-(R)-cyclopropyl]-1,4-dihydro-8-methyl-4-oxoquinoline-3-carboxylic acid (1.18 g, 3.78 mmol) and triethylamine (3 ml) and stirred for 3 days at 130°C under an atmosphere of nitrogen. After cooling, dimethyl sulfoxide was evaporated under a reduced pressure, the thus obtained residue was dissolved in chloroform (80 ml) and washed with 10% citric acid aqueous solution (80 ml) and saturated brine (100 ml) in that order and then the organic layer was dried over anhydrous sodium sulfate. After filtration, the filtrate was concentrated under a reduced pressure. The thus obtained residue was applied to a flash silica gel chromatography and eluted with a mixture of chloroform:methanol = 9:1, and the eluate was concentrated under a reduced pressure. To the thus obtained residue, which was cooled in an ice bath, was added dropwise concentrated hydrochloric acid (10 ml), followed by 50 minutes of stirring at room temperature. After adding 1 N hydrochloric acid (30 ml) to the reaction solution, the aqueous solution was washed with chloroform (50 ml x 2) and adjusted to pH 12.0 with sodium hydroxide aqueous solution. The aqueous solution was washed with chloroform (100 ml), adjusted to pH 7.4 with 1 N hydrochloric acid and then extracted with chloroform (150 ml x 3). The organic layers were combined, dried over anhydrous sodium sulfate and then filtered, and the filtrate was concentrated under a reduced pressure. To the thus obtained residue which was cooled in an ice bath was added dropwise 1 N hydrochloric acid (2.0 ml), followed by 5 minutes of stirring at the same temperature and subsequent concentration of the reaction solution under a reduced pressure (three times of ethanol azeotropic treatment). Thereafter, the resulting residue was purified by recrystallization from ethanol and then dried under a reduced pressure to obtain 230 mg (12.1%) of the title compound as a yellow powder.
¹H-NMR (400 MHz, 0.1 N NaOD) δ: 0.55 - 0.71 (4 H, m), 1.10 - 1.21 (1 H, m), 1.46 - 1.58 (1 H, m), 2.30 (3 H, s), 2.21 - 2.35 (1 H, m), 3.32 (1 H, t, J = 8.79 Hz), 3.49 (1 H, dd, J = 25.88, 12.21 Hz), 3.85 - 3.97 (2 H, m), 4.11 (1 H, ddm, J = 40.77, 12.45 Hz), 4.97 (1 H, dm, J = 70.31 Hz), 5.49 (1 H, brd, J = 55.18 Hz), 8.27 (1 H, d, J = 3.42 Hz).

**Elemental analysis data; for C₂₁H₂₃F₃N₄O₃•HCl•1.25H₂O:**

| | | | |
|---|---|---|---|
| calcd.; | C, 50.40; H, 5.33; N, 10.87 | | |
| found ; | C, 50.45; H, 5.44; N, 11.21 | | |

### [Inventive Example 10]

### 5-Amino-7-[4-(R)-(1-aminocyclopropyl)-3-(S)-fluoro-1-pyrrolidinyl]-6-fluoro-1-[2-(S)-fluoro-1-(R)-cycloaropyl]-1,4-dihydro-8-methoxy-4-oxoquinoline-3-carboxylic acid

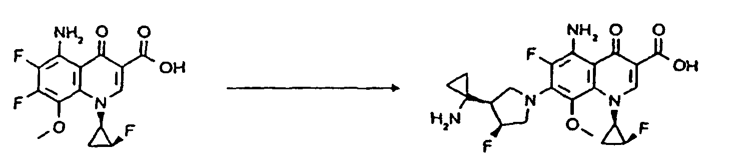

1-Benzyloxycarbonyl-4-(R)-(1-tert-butoxycarbonylaminocyclopropyl)-3-(S)-fluoropyrrolidine (400 mg, 1.06 mmol) was dissolved in ethanol (20 ml), and the solution was mixed with 5% palladium-carbon catalyst (water content, 55.6%; 500 mg) and stirred for 18 hours under an atmosphere of hydrogen. The catalyst was removed by celite filtration (methanol washing), and the filtrate was concentrated under a reduced pressure. The thus obtained residue was dissolved in dimethyl sulfoxide (8 ml), and the solution was mixed with 5-amino-6,7-difluoro-1-[2-(S)-fluoro-1-(R)-cyclopropyl]-1,4-dihydro-8-methoxy-4-oxoquinoline-3-carboxylic acid (289 mg, 0.88 mmol) and triethylamine (2 ml) and stirred for 26 hours at 100°C in an atmosphere of nitrogen. After cooling, dimethyl sulfoxide was evaporated under a reduced pressure, the thus obtained residue was dissolved in chloroform (80 ml) and washed with 10% citric acid aqueous solution (80 ml) and then the organic layer was dried over anhydrous sodium sulfate. After filtration, the filtrate was concentrated under a reduced pressure. The thus obtained residue was applied to a flash silica gel chromatography and eluted with a mixture of chloroform:methanol = 9:1, and the eluate was concentrated under a reduced pressure. To the thus obtained residue, which was cooled in an ice bath, was added dropwise concentrated hydrochloric acid (5 ml), followed by 20 minutes of stirring at room temperature. After adding 1 N hydrochloric acid (30 ml) to the reaction solution, the aqueous solution was washed with chloroform (50 ml x 2) and adjusted to pH 12.0 with sodium hydroxide aqueous solution. The aqueous solution was washed with chloroform (100 ml x 2), adjusted to pH 7.4 with 1 N hydrochloric acid and then extracted with chloroform (200 ml x 3). The organic layers were combined, dried over anhydrous sodium sulfate and then filtered, and the filtrate was concentrated under a reduced pressure. Thereafter, the resulting residue was purified by recrystallization from ethanol and then dried under a reduced pressure to obtain 170 mg (42.6%) of the title compound as a yellow powder.
¹H-NMR (400 MHz, 0.1 N NaOD) δ: 0.57 - 0.74 (4 H, m), 1.12 - 1.27 (1 H, m), 1.36 - 1.48 (1 H, m), 2.24 (1 H, dm, J = 37.60 Hz), 3.46 (3 H, s), 3.53 (1 H, t, J = 8.79 Hz), 3.69 (1 H, dd, J = 25.40, 12.21 Hz), 3.86 - 3.94 (2 H, m), 4.10 (1 H, ddm, J = 42.48, 12.70 Hz), 5.00 (1 H, dm, J = 63.97 Hz), 5.49 (1 H, brd, J = 54.69 Hz), 8.19 (1 H, d, J = 3.91 Hz)

**Elemental analysis data; for C₂₁H₂₃F₃N₄O₄:**

| | | | |
|---|---|---|---|
| calcd.; | C, 55.75; H, 5.12; N, 12.38 | | |
| found ; | C, 55.78; H, 5.20; N, 12.28 | | |

### [Inventive Example 11]

### 10-[4-(R)-(1-Aminocyclopropyl)-3-(S)-fluoro-1-pyrrolidinyl]-9-fluoro-2,3-dihydro-3-(S)-methyl-7-oxo-7H-pyrido[1.2.3-de][1.4]benzoxazine-6-carboxylic acid

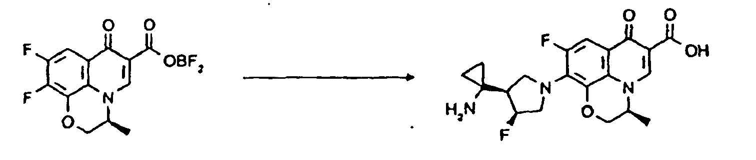

1-Benzyloxycarbonyl-4-(R)-(1-tert-butoxycarbonylaminocyclopropyl)-3-(S)-fluoropyrrolidine (913 mg, 2.41 mmol) was dissolved in methanol (50 ml), and the solution was mixed with 5% palladium-carbon catalyst (water content, 55.6%; 1.0 g) and stirred for 3 hours under an atmosphere of hydrogen. The catalyst was removed by celite filtration (methanol washing), and the filtrate was concentrated under a reduced pressure. The thus obtained residue was dissolved in dimethyl sulfoxide (15 ml), and the solution was mixed with 9,10-difluoro-2,3-dihydro-3-(S)-methyl-7-oxo-7H-pyrido(1.2.3-de)[1.4]benzoxazine-6-carboxylic acid-BF₂ chelate (661 mg, 2.01 mmol) and triethylamine (336 µl, 2.41 mmol) and stirred for 3 days at room temperature. The reaction solution was concentrated under a reduced pressure, the resulting residue was mixed with water and then the thus precipitated yellow crystals were collected by filtration and washed with water. The thus obtained crystals were suspended in a mixture of methanol:water = 1:1 (200 ml), and the suspension was mixed with triethylamine (4 ml) and heated under reflux for 4 hours. After cooling, the reaction solution was concentrated under a reduced pressure, the thus obtained residue was dissolved in chloroform (200 ml) and washed with 10% citric acid aqueous solution (200 ml) and then the organic layer was dried over anhydrous sodium sulfate. After filtration, the filtrate was concentrated under a reduced pressure. To the thus obtained residue, which was cooled in an ice bath, was added dropwise concentrated hydrochloric acid (10 ml), followed by 10 minutes of stirring at room temperature. After adding 1 N hydrochloric acid (30 ml) to the reaction solution, the aqueous solution was washed with chloroform (50 ml x 2) and adjusted to pH 12.0 with sodium hydroxide aqueous solution and then to pH 7.4 with 1 N hydrochloric acid, followed by extraction with chloroform (500 ml x 3). The organic layers were combined, dried over anhydrous sodium sulfate and then filtered, and the filtrate was concentrated under a reduced pressure. Thereafter, the resulting residue was purified by recrystallization from ethanol and then dried under a reduced pressure to obtain 459 mg (56.4%) of the title compound as light yellow crystals.
¹H-NMR (400 MHz, 0.1 N NaOD) δ: 0.55 - 0.75 (4 H, m), 1.52 (3 H, d, J = 6.84 Hz), 2.25 (1 H, dm, J = 36.62 Hz), 3.49 (1 H, t, J = 8.79 Hz), 3.70 (1 H, dd, J = 26.37, 11.72 Hz), 3.88 (1 H, t, J = 8.79 Hz), 4.10 (1 H, dd, J = 40.53, 12.70 Hz), 4.30 (1 H, d, J = 9.27 Hz), 4.50 (1 H, d, J = 9.28 Hz), 4.55 - 4.65 (1 H, m), 5.47 (1 H, dt, J = 55.17, 3.42 Hz), 7.53 (1 H, d, J = 14.16 Hz), 8.33 (1 H, s).

### [Inventive Example 12]

### 7-[4-(R)-(1-Aminocyclopropyl)-3-(S)-fluoro-1-pyrrolidinyl]-6-fluoro-1-[2-(S)-fluoro-1-(R)-cyclopropyl]-1,4-dihydro-8-methoxy-4-oxoquinoline-3-carboxylic acid

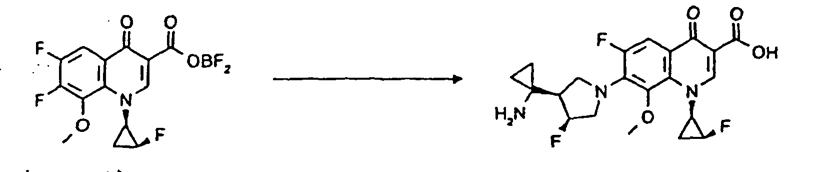

1-Benzyloxycarbonyl-4-(R)-(1-tert-butoxycarbonylaminocyclopropyl)-3-(S)-fluoropyrrolidine (1.07 g, 2.84 mmol) was dissolved in ethanol (50 ml), and the solution was mixed with 10% palladium-carbon catalyst (water content, 50.5%; 1.0 g) and stirred for 16 hours under an atmosphere of hydrogen. The catalyst was removed by celite filtration (methanol washing), and the filtrate was concentrated under a reduced pressure. The thus obtained residue was dissolved in dimethyl sulfoxide (10 ml), and the solution was mixed with 6,7-difluoro-1-[2-(S)-fluoro-1-(R)-cyclopropyl]-1,4-dihydro-8-methoxy-4-oxoquinoline-3-carboxylic acid-BF₂ chelate (853 mg, 2.36 mmol) and triethylamine (395 µl, 2.83 mmol) and stirred for 24 hours at room temperature. The reaction solution was concentrated under a reduced pressure, the resulting residue was mixed with water and then the thus precipitated solid matter was collected by filtration and washed with water. The thus obtained solid matter was suspended in a mixture of methanol:water = 9:1 (100 ml), and the suspension was mixed with triethylamine (5 ml) and heated under reflux for 3 hours. After cooling, the reaction solution was concentrated under a reduced pressure, the thus obtained residue was dissolved in chloroform (300 ml) and washed with 10% citric acid aqueous solution (300 ml) and then the organic layer was dried over anhydrous sodium sulfate. After filtration, the filtrate was concentrated under a reduced pressure. To the thus obtained residue, which was cooled in an ice bath, was added dropwise concentrated hydrochloric acid (10 ml), followed by 5 minutes of stirring at room temperature. After adding 1 N hydrochloric acid (30 ml) to the reaction solution, the aqueous solution was washed with chloroform (50 ml x 2) and adjusted to pH 12.0 with sodium hydroxide aqueous solution. The aqueous solution was washed with chloroform (50 ml x 2), adjusted to pH 7.4 with 1 N hydrochloric acid and then extracted with chloroform (500 ml x 3). The organic layers were combined, dried over anhydrous sodium sulfate and then filtered, and the filtrate was concentrated under a reduced pressure. Thereafter, the resulting residue was purified by recrystallization from ethanol and then dried under a reduced pressure to obtain 715 mg (69.3%) of the title compound as light yellow crystals.
Melting point: 218.5 - 219.8°C (decomposition)
¹H-NMR (400 MHz, 0.1 N NaOD) δ: 0.57 - 0.74 (4 H, m), 1.32 - 1.45 (1 H, m), 1.48 - 1.60 (1 H, m), 2.20 - 2.38 (1 H, m), 3.53 - 3.58 (1 H, m), 3.58 (3 H, s), 3.72 (1 H, dd, J = 25.88, 13.19 Hz), 3.86 - 3.93 (1 H, m), 4.00 - 4.18 (2 H, m), 5.05 (1 H, dm, J = 63.96 Hz), 5.51 (1 H, brd, J = 54.68 Hz), 7.68 (1 H, d, J = 14.16 Hz), 8.19 (1 H, d, J = 3.91 Hz).

**Elemental analysis data; for C₂₁H₂₂F₃N₃O₄:**

| | | | |
|---|---|---|---|
| calcd.; | C, 57.66; H, 5.07; N, 9.61 | | |
| found ; | C, 57.96; H, 5.13; N, 9.48 | | |

### [Reference Example 8-1]

### Ethyl 1-acetylcyclobutanecarboxylate

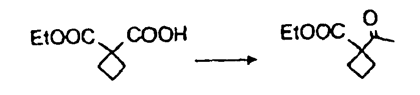

Ethyl hydrogen 1,1-cyclobutanecarboxylate (64.43 g, 374 mmol) was dissolved in methylene chloride (500 ml) to which, while cooling in an ice bath, were subsequently added oxalyl chloride (65.29 ml, 748 mmol) and a catalytical amount of N,N-dimethylformamide in that order. After 1.5 hours of stirring at room temperature, the solvent was evaporated and the resulting residue was subjected twice to azeotropic treatment with toluene, thereby preparing an acid chloride.

Separately from this, under a stream of nitrogen, copper(I) iodide (85.52 g, 449 mmol) was suspended in 1 liter of tetrahydrofuran to which was then added dropwise 1.4 M methyl lithium diethyl ether solution (294 ml) at -20°C, followed by 1 hour of stirring at the same temperature. To this was added dropwise a solution (300 ml) of the aforementioned acid chloride at the same temperature, followed by 1.5 hours of stirring. After completion of the reaction, the reaction solution was warmed up to room temperature and mixed with 10% citric acid aqueous solution (500 ml). Tetrahydrofuran was evaporated, and the resulting residue was mixed with ethyl acetate (1 liter), washed, after removing insoluble material by filtration, with 5% sodium thiosulfate aqueous solution (300 ml) and saturated brine (300 ml) in that order and then dried over anhydrous sodium sulfate. After evaporation of the solvent, the resulting residue was applied to a silica gel column chromatography and eluted with an eluant of n-hexane:ethyl acetate = 4:1, thereby obtaining 56.70 g (89%) of the title compound in an oily form.
¹H-NMR (400 MHz, CDCl₃) δ: 1.27 (3 H, t, J = 7.33 Hz), 1.82 - 2.01 (2 H, m), 2.12 (3 H, s), 2.45 - 2.55 (4 H, m), 4.20 - 4.24 (2 H, m).

### [Reference Example 8-2]

### Ethyl 1-ethoxycarbonyl-β-hydroxy-β-methylcyclobutylpropanoate

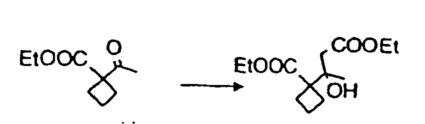

Ethyl 1-acetylcyclobutanecarboxylate (13.79 g, 81 mmol) was dissolved in tetrahydrofuran (50 ml), and the solution was mixed with zinc powder (10.59 g) and a catalytical amount of iodine. While heating under reflux, tetrahydrofuran solution (100 ml) of ethyl bromoacetate (13.48 ml, 121 mmol) was added dropwise thereto. The reaction solution was heated under reflux for additional 1 hour, cooled and then mixed with 1 N hydrochloric acid (100 ml). After evaporation of the solvent, the resulting residue was mixed with ethyl acetate (500 ml), washed, after removing insoluble material by filtration, with saturated brine (300 ml) and then dried over anhydrous sodium sulfate. By evaporating the solvent, the title compound was obtained quantitatively in an oily form.
¹H-NMR (400 MHz, CDCl₃) δ: 1.24 - 1.32 (9 H, m), 1.73 - 1.87 (2 H, m), 2.21 - 2.34 (2 H, m), 2.41 - 2.57 (5 H, m), 4.16 - 4.21 (4 H, m).

### [Reference Example 8-3]

### (E)-Ethyl 3-(1-ethoxycarbonylcyclobutyl)-2-butenoate

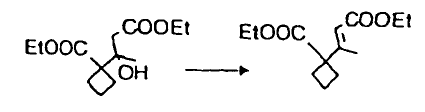

Ethyl 1-ethoxycarbonyl-β-hydroxy-β-methylcyclobutylpropanoate (22.27 g, 86 mmol) was dissolved in pyridine (42 ml), and thionyl chloride (8.18 ml, 112 mmol) was added dropwise to the thus prepared solution which was cooled at -10°C. After completion of the reaction, the reaction solution was poured into ice water (250 ml) and extracted with ethyl acetate (100 ml x 3). The organic layers were combined, washed with 1 N hydrochloric acid (100 ml) and saturated brine (100 ml) in that order and then dried over anhydrous sodium sulfate. The solvent was evaporated and the thus obtained residue was dissolved in methylene chloride (250 ml). At 0°C, to this was added dropwise 1,8-diazabicyclo[5,4,0]-7-undecene (12.89 ml), followed by 18 hours of stirring at room temperature. After completion of the reaction, the solvent was evaporated and the thus obtained residue was mixed with ice water (100 ml) and extracted with ethyl acetate (200 ml x 3). The organic layers were combined, washed with 1 N hydrochloric acid (100 ml) and saturated brine (100 ml) and then dried over anhydrous sodium sulfate. After evaporation of the solvent, the resulting residue was applied to a silica gel column chromatography and eluted with an eluant of n-hexane:ethyl acetate = 4:1, thereby obtaining 16.91 g (82%) of the title compound in an oily form.
¹H-NMR (400 MHz, CDCl₃) δ: 1.24 (3 H, t, J = 6.83 Hz), 1.29 (3 H, t, J = 7.32 Hz), 1.74 - 1.80 (2 H, m), 1.94 - 2.04 (1 H, m), 2.07 (3 H, d, J = 1.47 Hz), 2.12 - 2.30 (2 H, m), 2.12 - 2.30 (2 H, m), 2.50 - 2.57 (2 H, m), 4.13 - 4.20 (4 H, m).

### [Reference Example 8-4]

### 4-(1-Ethoxycarbonylcyclobutyl)-1-[(S)-1-phenylethyl]-3-pyrrolin-2-one

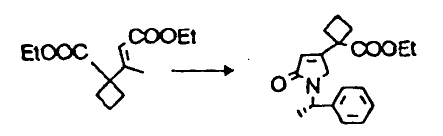

(E)-Ethyl 3-(1-ethoxycarbonylcyclobutyl)-2-butenoate (16.91 g, 70 mmol) was dissolved in chloroform (180 ml), and the solution was mixed with N-bromosuccinimide (12.53 g, 70 mmol) and a catalytical amount of azobisisobutyronitrile and heated under reflux for 18 hours. After completion of the reaction, the solvent was evaporated, the thus obtained residue was mixed with carbon tetrachloride (100 ml), insoluble material was removed by filtration and then the resulting filtrate was concentrated. The thus obtained residue was dissolved in ethanol (100 ml) and mixed with sodium bicarbonate (11.82 g, 140 mmol). At room temperature, thereto was added dropwise (S)-phenylethylamine (9.87 ml, 77 mmol). After completion of the dropwise addition, the mixture was heated under reflux for 3 hours. After completion of the reaction, the solvent was evaporated, and the thus obtained residue was mixed with methylene chloride (300 ml). After removing insoluble material by filtration, the solvent was evaporated and the resulting residue was applied to a silica gel column chromatography and eluted with an eluant of n-hexane:ethyl acetate = 1:1, thereby obtaining 19.57 g (43%) of the title compound in an oily form.
¹H-NMR (400 MHz, CDCl₃) δ: 1.17 (3 H, t, J = 7.33 Hz), 1.74 - 1.80 (2 H, m), 1.59 (3 H, d, J = 6.84 Hz), 1.84 - 2.01 (2 H, m), 2.15 - 2.28 (2 H, m), 2.60 - 2.69 (2 H, m), 3.56 (2 H, d, J = 9.04 Hz), 3.88 (2 H, d, J = 9.04 Hz), 4.13 (2 H, q, J = 7.32 Hz), 5.50 - 5.59 (1 H, m), 6.03 (1 H, s), 7.26 - 7.35 (5 H, m).

### [Reference Example 8-5]

### 4-(1-Ethoxycarbonylcyclobutyl)-1-[(S)-1-phenylethyl]-2-pyrrolidone

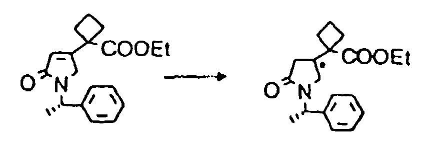

4-(1-Ethoxycarbonylcyclobutyl)-1-[(S)-1-phenylethyl]-3-pyrrolin-2-one (9.57 g, 31 mmol) was dissolved in ethanol (150 ml), and the solution was mixed with platinum oxide (230 mg) and stirred for 18 hours in an atmosphere of hydrogen. After completion of the reaction, the reaction solution was filtered and concentrated, and the resulting residue was applied three times to a silica gel column chromatography and eluted with an eluant of n-hexane:ethyl acetate = 1:1, thereby obtaining optical isomer A (2.3 g, 24%) and optical isomer B (7.1 g, 74%) of the title compound each in an oily form.

### Optical isomer A

¹H-NMR (400 MHz, CDCl₃) δ: 1.26 (3 H, t, J = 6.83 Hz), 1.49 (2 H, d, J = 7.32 Hz), 1.83 - 1.95 (4 H, m), 2.38 - 2.54 (4 H, m), 2.66 - 2.74 (1 H, m), 3.01 (1 H, t, J = 8.30 Hz), 3.14 (1 H, d, J = 5.86, 9.77 Hz), 4.09 - 4.18 (2 H, m), 5.48 (1 H, dd, J = 7.32, 14.16 Hz), 7.27 - 7.35 (5 H, m).

### Optical isomer B

¹H-NMR (400 MHz, CDCl₃) δ: 1.17 (3 H, t, J = 7.32 Hz), 1.52 (2 H, d, J = 7.33 Hz), 1.68 - 1.92 (4 H, m), 2.23 - 2.43 (3 H, m), 2.50 - 2.57 (1 H, m), 2.73 - 2.86 (2 H, m), 3.37 (1 H, t, J = 8.30 Hz), 4.05 (2 H, q, J = 7.32 Hz), 5.50 (1 H, dd, J = 7.32, 14.16 Hz), 7.24 - 7.35 (5 H, m).

### [Reference Example 8-6]

### Trans 4-(1-ethoxycarbonylcyclobutyl)-3-fluoro-1-[1-(S)-phenylethyl]-2-pyrrolidone (optical isomer B)

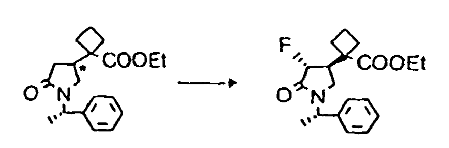

Under an atmosphere of nitrogen, diisopropylamine (2.55 ml, 18.2 mmol) was dissolved in anhydrous tetrahydrofuran (120 ml) to which, after cooling to -78°C, was subsequently added dropwise n-hexane solution of 1.63 M n-butyl lithium (11.2 ml, 18.2 mmol) in 10 minutes. After 15 minutes of stirring at 0°C, the reaction solution was cooled to -78°C and 4-(1-ethoxycarbonylcyclopropyl)-1-[1-(S)-phenylethyl]-2-pyrrolidone (optical isomer B; 4.42 g, 14.01 mmol) dissolved in anhydrous tetrahydrofuran (30 ml) was added dropwise thereto in 15 minutes. The reaction solution was stirred at -78°C for 1 hour, and N-fluorobenzene disulfonimide (7.07 g, 22.42 mmol) dissolved in anhydrous tetrahydrofuran (25 ml) was added dropwise thereto at the same temperature in 5 minutes. The reaction solution was stirred at -78°C for 30 minutes and then at room temperature for 20 minutes. Saturated ammonium chloride aqueous solution (200 ml) was added to the reaction solution which was cooled in an ice bath, tetrahydrofuran was evaporated and then the aqueous layer was extracted with ethyl acetate (200 ml x 2). The organic layers were combined, washed with water (200 ml x 3) and then dried over anhydrous sodium sulfate. After filtration, the filtrate was concentrated under a reduced pressure, and the resulting residue was applied to a silica gel column chromatography and eluted with an eluant of n-hexane:ethyl acetate = 1:1, thereby obtaining 3.88 g (83%) of the title compound in an oily form.
¹H-NMR (400 MHz, CDCl₃) δ: 1.14 (3 H, t, J = 6.83 Hz), 1.57 (2 H, d, J = 6.83 Hz), 1.88 - 2.08 (4 H, m), 2.33 - 2.58 (3 H, m), 2.81 - 2.92 (1 H, m), 3.42 (1 H, t, J = 9.77 Hz), 3.93 - 4.07 (2 H, m), 5.18 (1 H, dd, J = 6.83, 53.22 Hz), 5.51 (1 H, dd, J = 7.32, 14.16 Hz), 7.25 - 7.34 (5 H, m).

### [Reference Example 8-7]

### Cis 4-(1-ethoxycarbonylcyclobutyl)-3-fluoro-1-[1-(S)-phenylethyl]-2-pyrrolidone (optical isomer B)

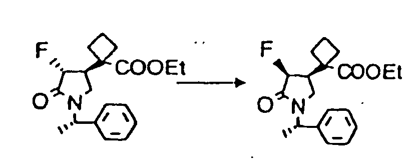

Under an atmosphere of nitrogen, diisopropylamine (2.97 ml, 21.19 mmol) was dissolved in anhydrous tetrahydrofuran (30 ml) to which, after cooling to -78°C, was subsequently added dropwise n-hexane solution of 1.63 M n-butyl lithium (10.8 ml, 17.60 mmol) in 5 minutes. After 15 minutes of stirring at 0°C, the reaction solution was cooled to -78°C and trans 4-(1-ethoxycarbonylcyclopropyl)-3-fluoro-1-[1-(S)-phenylethyl]-2-pyrrolidone (optical isomer B; 4.71 g, 14.13 mmol) dissolved in anhydrous tetrahydrofuran (30 ml) was added dropwise thereto in 5 minutes. The reaction solution was stirred at -78°C for 3 minutes, and 2,6-di-tertbutylphenol (4.37 g, 21.18 mmol) dissolved in anhydrous tetrahydrofuran (40 ml) was added dropwise thereto in 5 minutes. The reaction solution was stirred at -78°C for 10 minutes, mixed with saturated ammonium chloride aqueous solution (200 ml) and then warmed up to room temperature. The organic layer was separated and then the aqueous layer was extracted with chloroform (100 ml x 2). The organic layers were combined, washed with water (100 ml x 2) and then dried over anhydrous sodium sulfate. After filtration, the filtrate was concentrated under a reduced pressure, and the resulting residue was applied to a silica gel column chromatography and eluted with an eluant of n-hexane:ethyl acetate = 2:1 to recover 1.96 g (42%) of the starting material and then with n-hexane:ethyl acetate = 3:2 to obtain 1.79 g (38%) of the title compound in an oily form.
¹H-NMR (400 MHz, CDCl₃) δ: 1.22 (3 H, t, J = 6.83 Hz), 1.56 - 1.58 (3 H, d, J = 6.83 Hz), 1.84 - 2.42 (6 H, m), 2.83 - 2.97 (1 H, m), 3.15 - 3.24 (1 H, m), 3.36 - 3.43 (1 H, m), 4.11 - 4.17 (2 H, m), 5.07 (1 H, dd, J = 6.83, 52.24 Hz), 5.56 (1 H, q, J = 7.33 Hz), 7.26 - 7.36 (5 H, m).

### [Reference Example 8-8]

### Cis 4-(1-carboxycyclobutyl)-3-fluoro-1-[1-(S)-phenylethyl]-2-pyrrolidone (optical isomer B)

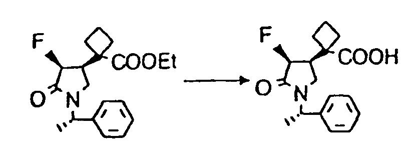

Cis 4-(1-ethoxycarbonylcyclobutyl)-3-fluoro-1-[1-(S)-phenylethyl]-2-pyrrolidone (optical isomer B; 1.79 g, 5.37 mmol) was dissolved in methanol (10 ml) to which was subsequently added dropwise 1 N sodium hydroxide aqueous solution (10 ml). The reaction solution was stirred at 40°C for 18 hours and then methanol was evaporated under a reduced pressure. The thus obtained residue was mixed with water (50 ml) and washed with chloroform (100 ml). The thus separated aqueous layer was acidified by dropwise addition of 1 N hydrochloric acid and extracted with chloroform (100 ml x 2). The organic layers were combined and dried over anhydrous sodium sulfate. After filtration, the filtrate was concentrated under a reduced pressure to obtain the title compound quantitatively as a crude product.

### [Reference Example 8-9]

### Cis 4-(1-tert-butoxycarbonylaminocyclobutyl)-3-fluoro-1-[1-(S)-pbenylethyl]-2-pyrrolidone (optical isomer B)

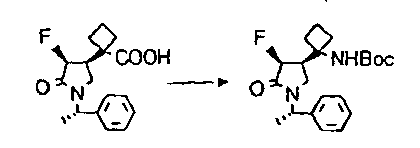

Cis 4-(1-carboxycyclobutyl)-3-fluoro-1-[1-(S)-phenylethyl]-2-pyrrolidone (optical isomer B; 1.92 g, 6.29 mmol) was dissolved in anhydrous acetonitrile (30 ml), and the solution was mixed with N,N'-carbonyl diimidazole (1.33 g, 8.20 mmol) and stirred at 60°C for 1 hour. At room temperature and for 10 minutes, ammonia was bubbled into the reaction solution which was subsequently concentrated under a reduced pressure. The thus obtained residue was mixed with water (100 ml) and extracted with chloroform (100 ml x 2), and the organic layers were combined and dried over anhydrous sodium sulfate. After filtration, the filtrate was concentrated under a reduced pressure, and the resulting residue was dissolved in tert-butyl alcohol (50 ml), mixed with lead tetraacetate (6.32 g, 14.25 mmol) and then heated under reflux for 1 hour. After cooling, the reaction solution was mixed with diethyl ether (50 ml) and sodium bicarbonate (6 g), and the mixture was stirred at room temperature for 10 minutes. After filtration, the filtrate was concentrated under a reduced pressure. The thus obtained residue was mixed with 100 ml of ethyl acetate, washed with saturated sodium bicarbonate and then dried over anhydrous sodium sulfate. Thereafter, this was filtered and the resulting filtrate was concentrated under a reduced pressure to obtain 1.74 g (65%) of the title compound in an oily form.
¹H-NMR (400 MHz, CDCl₃) δ: 1.40 (9 H, s), 1.92 - 2.21 (6 H, m), 3.04 - 3.12 (1 H, m), 3.31 - 3.38 (1 H, m), 4.87 (1 H, brs), 5.01 (1 H, dd, J = 5.86, 52.73 Hz), 5.52 (1 H, dd, J = 7.32, 14.16 Hz), 7.30 - 7.38 (5 H, m).

### [Reference Example 8-10]

### Cis 1-[1-(S)-phenylethyl]-4-(1-tert-butoxycarbonylaminocyclobutyl)-3-fluoropyrrolidone (optical isomer B)

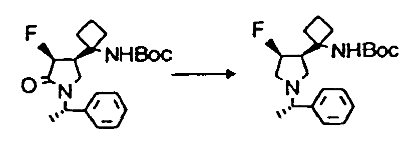

Cis 4-(1-tert-butoxycarbonylaminocyclobutyl)-3-fluoro-1-[1-(S)-phenylethyl]-2-pyrrolidone (optical isomer B; 1.74 g, 4.62 mmol) was dissolved in tetrahydrofuran (30 ml), and the solution was mixed with 1 M boran-tetrahydrofuran complex (13.86 ml) at 0°C and then stirred at room temperature for 2 days. After completion of the reaction, the solvent was evaporated and the thus obtained residue was mixed with water (50 ml) and extracted with chloroform (100 ml x 2). The organic layers were combined and dried over anhydrous sodium sulfate. After filtration, the filtrate was concentrated under a reduced pressure, and the resulting residue was dissolved in 80% aqueous ethanol (40 ml), mixed with triethylamine (10 ml) and then heated under reflux for 2 hours. Thereafter, the solvent was evaporated and the resulting residue was applied to a silica gel column chromatography and eluted with an eluant of n-hexane:ethyl acetate = 2:1, thereby obtaining 1.13 g (67%) of the title compound in an oily form.
¹H-NMR (400 MHz, CDCl₃) δ: 1.37 (3 H, d, J = 6.35 Hz), 1.44 (9 H, s), 1.65 - 2.58 (7 H, m), 2.70 - 2.92 (4 H, m), 3.27 - 3.32 (1 H, m), 5.14 (1 H, brd), 5.53 (1 H, brs), 7.22 - 7.33 (5 H, m).

### [Inventive Example 13]

### 5-Amino-7-[cis 4-(1-aminocyclobutyl)-3-fluoro-1-Pyrrolidinyl]-6-fluoro-1-[2-(S)-fluoro-1-(R)-cyclopropyl]-1,4-dihydro-8-methyl-4-oxoquinoline-3-carboxylic acid (optical isomer B)

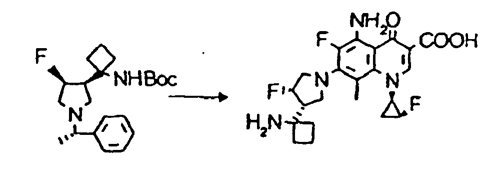

Cis 1-[1-(S)-phenylethyl]-4-(1-tert-butoxycarbonylaminocyclobutyl)-3-fluoropyrrolidine (optical isomer B; 1.13 g, 3.12 mmol) was dissolved in ethanol (20 ml), and the solution was mixed with 10% palladium-carbon catalyst (water content, 55.6%; 1.0 g) and stirred at 50°C for 18 hours under an atmosphere of hydrogen. The catalyst was removed by celite filtration (methanol washing), and the filtrate was concentrated under a reduced pressure. The thus obtained residue was dissolved in dimethyl sulfoxide (10 ml), and the solution was mixed with 5-amino-6,7-difluoro-1-[2-(S)-fluoro-1-(R)-cyclopropyl]-1,4-dihydro-8-methyl-4-oxoquinoline-3-carboxylic acid (1.18 g, 3.78 mmol) and triethylamine (5 ml) and stirred at 140°C for 4 days under an atmosphere of nitrogen. After cooling, dimethyl sulfoxide was evaporated under a reduced pressure, the thus obtained residue was dissolved in chloroform (50 ml) and washed with 10% citric acid aqueous solution (50 ml) and saturated brine (100 ml) in that order and then the organic layer was dried over anhydrous sodium sulfate. After filtration, the filtrate was concentrated under a reduced pressure. The thus obtained residue was applied to a flash silica gel chromatography and eluted with an eluant of chloroform:methanol = 9:1. The eluate was concentrated under a reduced pressure. To the thus obtained residue, which was cooled in an ice bath, was added dropwise concentrated hydrochloric acid (5 ml), followed by 30 minutes of stirring at room temperature. After adding 1 N hydrochloric acid (30 ml) to the reaction solution, the aqueous solution was washed with chloroform (50 ml x 2) and adjusted to pH 12.0 with sodium hydroxide aqueous solution. The aqueous solution was washed with chloroform (100 ml), adjusted to pH 7.4 with 1 N hydrochloric acid and then extracted with chloroform (150 ml x 3). The organic layers were combined, dried over anhydrous sodium sulfate and then filtered, and the filtrate was concentrated under a reduced pressure. The thus obtained residue was applied to a preparative TLC (development with the bottom layer of a mixture of chloroform:methanol:water = 7:3:1) to give a crude title compound, and this was recrystallized from a mixture of ethanol and ether to obtain 157 mg (17%) of the title compound.
Melting point: 177 - 184°C
¹H-NMR (400 MHz, CDCl₃) δ: 1.16 - 2.34 (13 H, m), 2.47 - 2.60 (1 H, m), 3.35 (1 H, t, J = 8.79 Hz), 3.53 (1 H, q, J = 12.21 Hz), 3.78 - 3.83 (1 H, m), 4.09 - 4.21 (2 H, m), 4.76 - 4.95 (1 H, m), 5.42 (1 H, dt, J = 3.41, 55.18 Hz), 6.53 (2 H, brs), 8.60 (1 H, d, J = 3.41 Hz)

**Elemental analysis data; for C₂₂H₂₅F₃N₄O₃•0.5H₂O:**

| | | | |
|---|---|---|---|
| calcd.; | C, 57.51; H, 5.70; N, 12.19 | | |
| found ; | C, 57.59; H, 5.52; N, 11.89 | | |

**Table 1**

| Strain/Compound | Inventive Example No. | | |
|---|---|---|---|
| | 3 | 4 | 5 |
| *E. coli,* NIHJ | ≤ 0.003 | 0.013 | ≤ 0.003 |
| *S. flexneli,* 2A 5503 | ≤ 0.003 | 0.013 | ≤ 0.003 |
| *Pr. vulgaris,* 08601 | 0.025 | 0.10 | 0.013 |
| *Pr. mirabilis*, IFO-3849 | 0.05 | 0.20 | 0.025 |
| *Ser. marcescens,* 10100 | 0.10 | 0.20 | 0.05 |
| *Ps. aeruginosa,* 32104 | 0.20 | 0.78 | 0.10 |
| *Ps. aeruginosa,* 32121 | 0.10 | 0.39 | 0.05 |
| *Ps. maltophilia*, IID-1275 | 0.10 | 0.20 | 0.05 |
| *S. aureus,* 209P | ≤ 0.003 | ≤ 0.003 | ≤ 0.003 |
| *S. epidermidis,* 56500 | ≤ 0.003 | 0.013 | 5.0.003 |
| *Str. pyogenes,* G-36 | ≤ 0.003 | 0.025 | ≤ 0.003 |
| *Str. faecalis,* ATCC-19433 | 0.025 | 0.10 | 0.013 |
| *S. aureus,* 870307 | 0.025 | 0.10 | 0.006 |

**Table 2**

| Strain/Compound | Inventive Example No. | | |
|---|---|---|---|
| | 6 | 7 | 8 |
| *E. coli,* NIHJ | 0.013 | ≤ 0.003 | 0.025 |
| *S. flexneli,* 2A 5503 | 0.025 | ≤ 0.003 | 0.05 |
| *Pr. vulgaris,* 08601 | 0.05 | 0.05 | 0.10 |
| *Pr. mirabilis,* IFO-3849 | 0.20 | 0.025 | 0.78 |
| *Ser. marcescens,* 10100 | 0.10 | 0.05 | 0.39 |
| *Ps. aeruginosa,* 32104 | 0.78 | 0.10 | 1.56 |
| *Ps. aeruginosa,* 32121 | 0.20 | 0.05 | 0.39 |
| *Ps. maltophilia*, IID-1275 | 0.39 | 0.05 | 0.39 |
| *S. aureus,* 209P | 0.006 | ≤ 0.003 | 0.025 |
| *S. epidermidis,* 56500 | 0.025 | ≤ 0.003 | 0.05 |
| *Str. pyogenes*, G-36 | 0.025 | ≤ 0.003 | 0.10 |
| *Str. faecalis,* ATCC-19433 | 0.10 | 0.013 | 0.20 |
| *S. aureus,* 870307 | 0.39 | 0.013 | 0.78 |

**Table 3**

| Strain/Compound | Inventive | Example No. |
|---|---|---|
| | 12 | 13 |
| *E. coli,* NIHJ | ≤ 0.003 | ≤ 0.003 |
| *S. flexneli,* 2A 5503 | 0.013 | 0.006 |
| *Pr. vulgaris,* 08601 | 0.013 | 0.025 |
| *Pr. mirabilis*, IFO-3849 | 0.05 | 0.05 |
| *Ser. marcescens*, 10100 | 0.10 | 0.20 |
| *Ps. aeruginosa,* 32104 | 0.39 | 0.20 |
| *Ps. aeruginosa,* 32121 | 0.10 | 0.10 |
| *Ps. maltophilia,* IID-1275 | 0.20 | 0.20 |
| *S. aureus,* 209P | ≤ 0.003 | ≤ 0.003 |
| *S. epidermidis,* 56500 | 0.013 | 0.006 |
| *Str. pyogenes,* G-36 | 0.006 | 0.006 |
| *Str. faecalis,* ATCC-19433 | 0.025 | 0.025 |
| *S. aureus,* 870307 | 0.025 | 0.05 |

### INDUSTRIAL APPLICABILITY

Thus, as has been described in the foregoing, it is evident that the compound of the present invention has excellent antibacterial activity and safety and is useful as pharmaceutical drugs.

## Claims

1. A compound represented by formula (I): 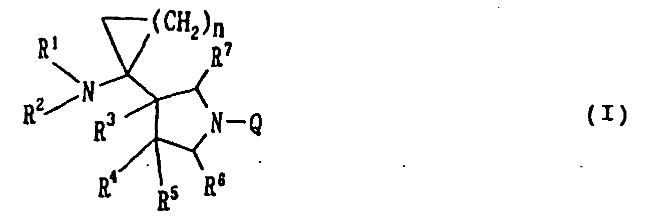 {wherein R¹ represents a hydrogen atom or an alkyl group having 1 to 6 carbon atoms;
R² represents a hydrogen atom or an alkyl group having 1 to 6 carbon atoms,
wherein the alkyl group may have at least one substituent selected from the group consisting of a hydroxyl group, a halogen atom, an alkylthio group having 1 to 6 carbon atoms and an alkoxyl group having 1 to 6 carbon atoms; R³ represents a hydrogen atom, a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms,
wherein the alkyl group may have at least one substituent selected from the group consisting of a hydroxyl group, a halogen atom and an alkoxyl group having 1 to 6 carbon atoms;
R⁴ represents a hydrogen atom, a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, and R⁵ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms,
wherein the alkyl group may have at least one substituent selected from the group consisting of a hydroxyl group, a halogen atom and an alkoxyl group having 1 to 6 carbon atoms, and
R⁴ and R³ may be combined to form a hydroxyimino group, a methylene chain having 3 to 6 carbon atoms (so as to form a spiro cyclic structure together with the pyrrolidine ring) or an alkyloxyimino group having 1 to 6 carbon atoms;
R⁶ and R⁷, each independently represents a hydrogen atom or an alkyl group having 1 to 6 carbon atoms;
n is an integer of 1 to 3; and
Q is a partial structure represented by formula (II): 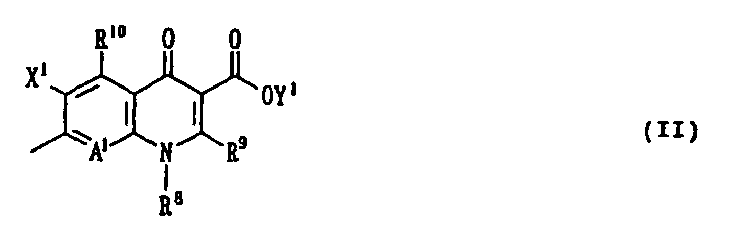 [wherein R⁸ represents a 2-halogenocyclopropyl group;
R⁹ represents a hydrogen atom or an alkylthio group having 1 to 6 carbon atoms,
or wherein R⁸ and R⁹ may be combined to form a cyclic structure including a part of the mother nucleus, and the ring may contain a sulfur atom as a ring constituting atom and may further have an alkyl group having 1 to 6 carbon atoms as a substituent,
X¹ represents a halogen atom or a hydrogen atom;
R¹⁰ represents a hydrogen atom, an amino group, a hydroxyl group, a thiol group, a halogenomethyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms,
wherein the amino group may have at least one substituent selected from the group consisting of a formyl group, an alkyl group having 1 to 6 carbon atoms and an acyl group having 2 to 5 carbon atoms;
A¹ represents a nitrogen atom, or a partial structure represented by formula (III): 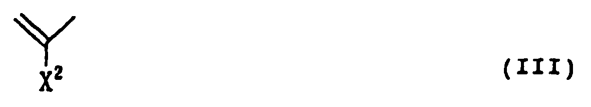 (wherein X² represents a hydrogen atom, an amino group, a halogen atom, a cyano group, a halogenomethyl group, a halogenomethoxyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms,
wherein the amino group may have at least one substituent selected from the group consisting of a formyl group, an alkyl group having 1 to 6 carbon atoms and an acyl group having 2 to 5 carbon atoms, and
X² and R³ may be combined to form a cyclic structure including a part of the mother nucleus, and the ring may contain an oxygen atom, a nitrogen atom or a sulfur atom as a ring constituting atom and may further have an alkyl group having 1 to 6 carbon atoms as a substituent); and
Y¹ represents a hydrogen atom, a phenyl group, an acetoxymethyl group, a pivaloyloxymethyl group, an ethoxycarbonyl group, a choline group, a dimethylaminoethyl group, a 5-indanyl group, a phthalidinyl group, a 5-alkyl-2-oxo-1,3-dioxol-4-ylmethyl group, a 3-acetoxy-2-oxobutyl group, an alkyl group having 1 to 6 carbon atoms, an alkoxymethyl group having 2 to 7 carbon atoms or a phenylalkyl group composed of an alkylene group having 1 to 6 carbon atoms and a phenyl group] or Q is a partial structure represented by formula (IV): 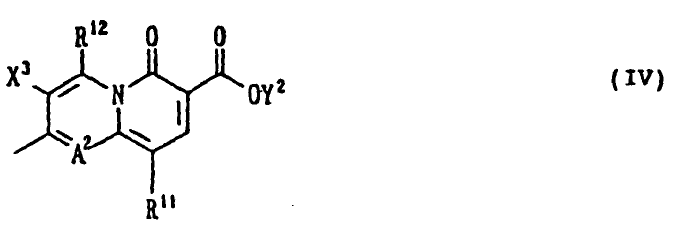 [wherein R¹¹ represents a 2-halogenocyclopropyl group;
R¹² represents a hydrogen atom, an amino group, a hydroxyl group, a thiol group, a halogenomethyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms,
wherein the amino group may have at least one substituent selected from the group consisting of a formyl group, an alkyl group having 1 to 6 carbon atoms and an acyl group having 2 to 5 carbon atoms;
X³ represents a halogen atom or a hydrogen atom;
A² represents a nitrogen atom or a partial structure represented by formula (V): 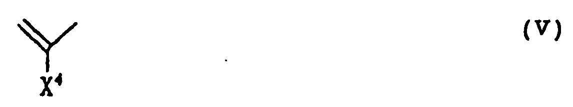
(wherein X⁴ represents a hydrogen atom, an amino group, a halogen atom, a cyano group, a halogenomethyl group, a halogenomethoxyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms,
wherein the amino group may have at least one substituent selected from the group consisting of a formyl group, an alkyl group having 1 to 6 carbon atoms and an acyl group having 2 to 5 carbon atoms, and
X⁴ and R¹¹ may be combined to form a cyclic structure including a part of the mother nucleus, and the ring may contain an oxygen atom, a nitrogen atom or a sulfur atom as a ring constituting atom and may further have an alkyl group having 1 to 6 carbon atoms as a substituent); and Y² represents a hydrogen atom, a phenyl group, an acetoxymethyl group, a pivaloyloxymethyl group, an ethoxycarbonyl group, a choline group, a dimethylaminoethyl group, a 5-indanyl group, a phthalidinyl group, a 5-alkyl-2-oxo-1,3-dioxol-4-ylmethyl group, a 3-acetoxy-2-oxobutyl group, an alkyl group having 1 to 6 carbon atoms, an alkoxymethyl group having 2 to 7 carbon atoms or a phenylalkyl group composed of an alkylene group having 1 to 6 carbon atoms and a phenyl group)}, and its salts and hydrates thereof.

2. The compound, its salts and hydrates thereof according to claim 1, wherein Q in the formula (I) has a structure represented by the formula (II).

3. The compound, its salts and hydrates thereof according to claim 2, wherein R⁸ is a 1,2-cis-2-halogenocyclopropyl group.

4. The compound, its salts and hydrates thereof according to claim 3, wherein the 1,2-cis-2-halogenocyclopropyl group in the formula (I) is a stereochemically pure substituent.

5. The compound, its salts and hydrates thereof according to claim 4, wherein the halogenocyclopropyl group in the formula (I) is a (1R,2S)-2-halogenocyclopropyl group.

6. The compound, its salts and hydrates thereof according to claim 3, 4 or 5, wherein the halogen atom of the halogenocyclopropyl group in the formula (I) is a fluorine atom.

7. The compound, its salts and hydrates thereof according to any one of the preceding claims, wherein the compound of the formula (I) is a stereochemically pure compound.

8. The compound, its salts and hydrates thereof according to claim 1, namely 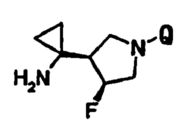
wherein Q is a partial structure selected from the group consisting of:

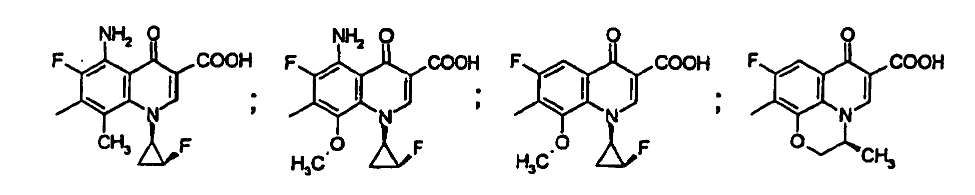

9. The compound, its salts and hydrates thereof according to claim 1, namely 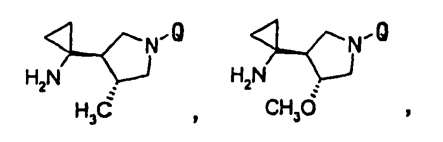 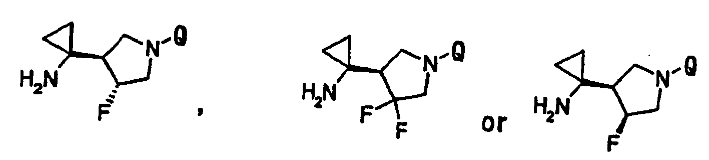 wherein Q is the partial structure:

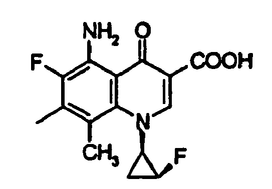

10. The compound, its salts and hydrates thereof according to claim 1, namely
5-Amino-7- [(3R,4R)-3-(1-aminocyclopropyl)-4-methyl-1-pyrrolidinyl]-6-fluoro-1-[(1R,2S)-2-fluorocyclopropyl]-1,4-dihydro-8-methyl-4-oxoquinoline-3-carboxylic acid;
5-Amino-7-[4-(R)-(1-aminocyclopropyl)-3-(R)-methoxy-1-pyrrolidinyl]-6-fluoro-1-[2-(S)-fluoro-1-(R)-cyclopropyl]-1,4-dihydro-8-methyl-4-oxoquinoline-3-carboxylic acid;
5-Amino-7-[4-(R)-(1-aminocyclopropyl)-3-(R)-fluoro-1-pyrrolidinyl]-6-fluoro-1-[2-(S)-fluoro-1-(R)-cyclopropyl]-1,4-dihydro-8-methyl-4-oxoquinoline-3-carboxylic acid hydrochloride;
5-Amino-7-[4-(R)-(1-aminocyclopropyl)-3,3-difluoro-1-pyrrolidinyl]-6-fluoro-1-[2-(S)-fluoro-1- (R)-cyclopropyl]-1,4-dihydro-8-methyl-4-oxoquinoline-3-carboxylic acid hydrochloride;
5-Amino-7-[4-(R)-(1-aminocyclopropyl)-3-(S)-fluoro-1-pyrrolidinyl]-6-fluoro-1-[2-(S)-fluoro-1-(R)-cyclopropyl]-1,4-dihydro-8-methyl-4-oxoquinoline-3-carboxylic acid hydrochloride;
5-Amino-7-[4-(R)-(1-aminocyclopropyl)-3- (S)-fluoro-1-pyrrolidinyl] -6-fluoro-1-[2-(S) -fluoro-1-(R) -cyclopropyl]-1, 4-dihydro-8-methoxy-4-oxoquinoline-3-carboxylic acid;
7-[4-(R)-(1-Aminocyclopropyl)-3-(S)-fluoro-1-pyrrolidinyl]-6-fluoro-1-[2- (S) -fluoro-1-(R) -cyclopropyl]-1,4-dihydro-8-methoxy-4-oxoquinoline-3-carboxylic acid;
5-Amino-7-[cis-4-(1-aminocyclobutyl)-3-fluoro-1-pyrrolidinyl]-6-fluoro-1-[2-(S)-fluoro-1-(R)-cyclopropyl]-1,4-dihydro-8-methyl-4-oxoquinoline-3-carboxylic acid.

11. The compound, its salts and hydrates thereof according to claim 1, namely
10-[4-(R)-(1-Aminocyclopropyl)-3-(R)-fluoro-1-pyrrolidinyl]-9-fluoro-2,3-dihydro-3-(S)-methyl-7-oxo-7H-pyrido[1.2.3-de][1.4]benzoxazine-6-carboxylic acid;
10-[4-(R)-(1-Aminocyclopropyl)-3,3-difluoro-1-pyrrolidinyl]-9-fluoro-2,3-dihydro-3-(S)-methyl-7-oxo-7H-pyrido[1.2.3-de][1.4]benzoxazine-6-carboxylic acid;
10-[4-(R)-(1-Aminocyclopropyl)-3-(S)-fluoro-1-pyrrolidinyl]-9-fluoro-2,3-dihydro-3-(S)-methyl-7-oxo-7H-pyrido[1.2.3-de][1.4]benzoxazine-6-carboxylic acid.

12. A process for producing a compound, its salts and hydrates thereof according to any one of the preceding claims, in which process a compound represented by formula (VI): 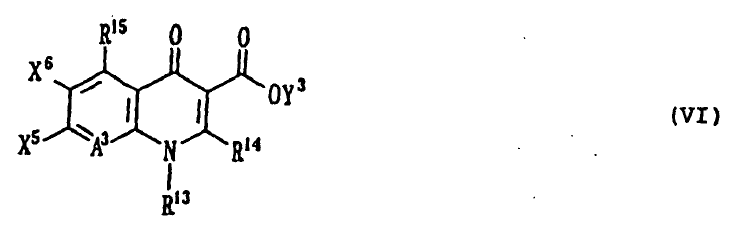 [wherein X⁵ is a leaving group; Y³ is the Y¹ as defined in claim 1 or a group represented by formula (VII): 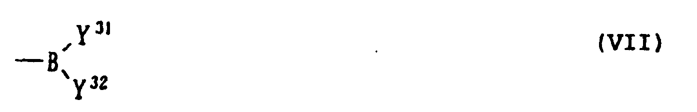 (wherein each of Y³¹ and Y³² is a fluorine atom or an alkylcarbonyloxy group having 2 to 5 carbon atoms), and R¹³, R¹⁴, R¹⁵, A³ and X⁶ correspond to R⁸, R⁹, R¹⁰, A¹ and X¹ as defined in claim 1],
or a compound represented by formula (VIII): 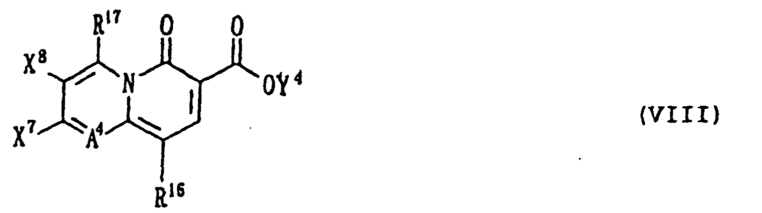 [wherein X⁷ is a leaving group; and R¹⁶, R¹⁷, A⁴, X⁸ and Y⁴ correspond to R¹¹, R¹², A², X³ and Y² as defined in claim 1], is allowed to react with a compound represented by formula (IX): 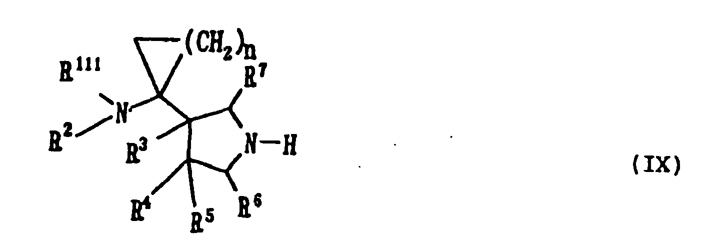 [wherein R¹¹¹ is the R¹ as defined in claim 1 or a protective group of the amino group and R², R³, R⁴, R⁵, R⁶, R⁷ and n are as defined in claim 1] or with an acid addition salt thereof, protective groups are removed, if required, and the compound of formula (I), its salts or hydrates thereof are recovered.

13. A compound represented by formula (IX) 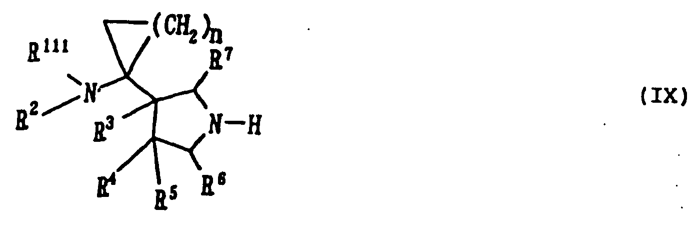 {wherein R¹¹¹ represents a hydrogen atom, an alkyl group having 1 to 6 carbon atoms or a protective group of the amino group; R² represents a hydrogen atom or an alkyl group having 1 to 6 carbon atoms,
wherein the alkyl group may have at least one substituent selected from the group consisting of a hydroxyl group, a halogen atom, an alkylthio group having 1 to 6 carbon atoms and an alkoxyl group having 1 to 6 carbon atoms; R³ represents a hydrogen atom, a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms,
wherein the alkyl group may have at least one substituent selected from the group consisting of a hydroxyl group, a halogen atom and an alkoxyl group having 1 to 6 carbon atoms;
R⁴ represents a hydrogen atom, a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, and R⁵ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms,
wherein the alkyl group may have at least one substituent selected from the group consisting of a hydroxyl group, a halogen atom and an alkoxyl group having 1 to 6 carbon atoms, and
R⁴ and R⁵ may be combined to form a hydroxyimino group, a methylene chain having 3 to 6 carbon atoms (so as to form a spiro cyclic structure together with the pyrrolidine ring) or an alkyloxyimino group having 1 to 6 carbon atoms ; R⁶ and R⁷, each independently represents a hydrogen atom or an alkyl group having 1 to 6 carbon atoms; and n is an integer of 1 to 3}; or an acid addition salt thereof.

14. The compound and acid addition salts thereof according to claim 13, namely 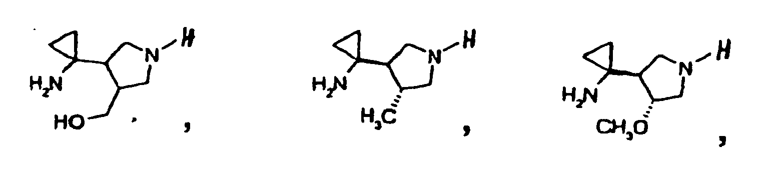 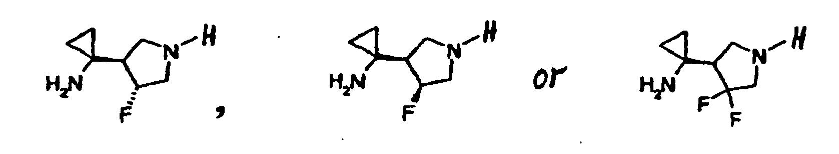 wherein the amino group may be protected.

15. The compound and acid addition salts thereof, according to claim 13 or 14,
wherein the amino group-protecting group is selected from alkoxycarbonyl groups such as tert-butoxycarbonyl, 2,2,2-trichloroethoxycarbonyl, aralkyloxycarbonyl groups such as benzyloxycarbonyl, p-methoxybenzyloxycarbonyl, p-nitrobenzyloxycarbonyl, acyl groups such as acetyl, methoxyacetyl, trifluoroacetyl, chloroacetyl, pivaloyl, formyl, benzoyl, alkyl or aralkyl groups such as tert-butyl, benzyl, p-nitrobenzyl, p-methoxybenzyl, triphenylmethyl ethers such as methoxymethyl, tert-butoxymethyl, tetrahydropyranyl, 2,2,2-trichloroethoxymethyl and silyl groups such as trimethylsilyl, isopropyldimethylsilyl, tert-butyldimethylsilyl, tribenzylsilyl, tert-butyldiphenylsilyl.

16. A pharmaceutical preparation which comprises a compound, its salt or a hydrate thereof as defined in any one of claims 1 to 11 as an active ingredient.

17. An antibacterial drug which comprises a compound, its salt or a hydrate thereof as defined in any one of claims 1 to 11 as an active ingredient.

18. Use of a compound, its salt or a hydrate thereof as defined in any one of claims 1 to 11, for preparing a pharmaceutical for treating, preventing or alleviating infectious diseases.

## Patentansprüche

1. Verbindung der Formel (I): 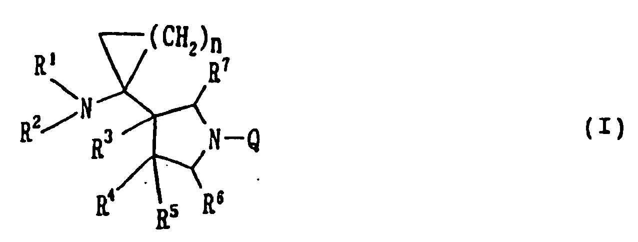 {worin R¹ für ein Wasserstoffatom oder eine Alkylgruppe mit 1 bis 6 Kohlenstoffatomen steht.
R² für ein Wasserstoffatom oder ein Alkylgruppe mit 1 bis 6 Kohlenstoffatomen steht, wobei die Alkylgruppe wenigstens einen unter einer Hydroxylgruppe, einem Halogenatom, einer Alkylthiogruppe mit 1 bis 6 Kohlenstoffatomen und einer Alkoxylgruppe mit 1 bis 6 Kohlenstoffatomen ausgewählten Substituenten aufweisen kann;
R³ für ein Wasserstoffatom, eine Hydroxylgruppe, ein Halogenatom, eine Carbamoylgruppe, eine Alkylgruppe mit 1 bis 6 Kohlenstoffatomen, eine Alkoxylgruppe mit 1 bis 6 Kohlenstoffatomen oder eine Alkylthiogruppe mit 1 bis 6 Kohlenstoffatomen steht, wobei die Alkylgruppe wenigstens einen unter einer Hydroxylgruppe, einem Halogenatom und einer Alkoxylgruppe mit 1 bis 6 Kohlenstoffatomen ausgewählten Substituenten aufweisen kann;
R⁴ für ein Wasserstoffatom, eine Hydroxylgruppe, ein Halogenatom, eine Carbamoylgruppe, eine Alkylgruppe mit 1 bis 6 Kohlenstoffatomen, eine Alkoxylgruppe mit 1 bis 6 Kohlenstoffatomen oder eine Alkylthiogruppe mit 1 bis 6 Kohlenstoffatomen steht, und R⁵ für eine Hydroxylgruppe, ein Halogenatom, eine Carbamoylgruppe, eine Alkylgruppe mit 1 bis 6 Kohlenstoffatomen, eine Alkoxylgruppe mit 1 bis 6 Kohlenstoffatomen oder eine Alkylthiogruppe mit 1 bis 6 Kohlenstoffatomen steht, wobei die Alkylgruppe wenigstens einen unter einer Hydroxylgruppe, einem Halogenatom und einer Alkoxylgruppe mit 1 bis 6 Kohlenstoffatomen ausgewählten Substituenten aufweisen kann, und
R⁴ und R⁵ zusammen eine Hydroxyiminogruppe, eine Methylenkette mit 3 bis 6 Kohlenstoffatomen (so dass zusammen mit dem Pyrrolidinring eine spirozyklische Struktur gebildet wird) oder eine Alkyloxyiminogruppe mit 1 bis 6 Kohlenstoffatomen bilden können;
R⁶ und R⁷ jeweils unabhängig voneinander für ein Wasserstoffatom oder eine Alkylgruppe mit 1 bis 6 Kohlenstoffatomen stehen;
n eine ganze Zahl mit einem Wert von 1 bis 3 ist; und
Q eine Partialstruktur der Formel (II): 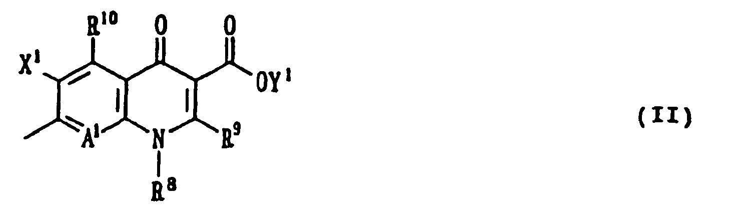 ist,
[worin R⁸ für eine 2-Halogencyclopropylgruppe steht;
R⁹ für ein Wasserstoffatom oder eine Alkylthiogruppe mit 1 bis 6 Kohlenstoffatomen steht,
oder worin R⁸ und R⁹ zusammen eine zyklische Struktur bilden können, an der ein Teil des Grundgerüsts beteiligt ist, und der Ring als ringbildendes Atom ein Schwefelatom enthalten und des weiteren als Substituent eine Alkylgruppe mit 1 bis 6 Kohlenstoffatomen aufweisen kann,
X¹ für ein Halogenatom oder ein Wasserstoffatom steht;
R¹⁰ für ein Wasserstoffatom, eine Aminogruppe, eine Hydroxylgruppe, eine Thiolgruppe, eine Halogenmethylgruppe, eine Alkylgruppe mit 1 bis 6 Kohlenstoffatomen, eine Alkenylgruppe mit 2 bis 6 Kohlenstoffatomen, eine Alkinylgruppe mit 2 bis 6 Kohlenstoffatomen oder eine Alkoxylgruppe mit 1 bis 6 Kohlenstoffatomen steht, wobei die Aminogruppe wenigstens einen unter einer Formylgruppe, einer Alkylgruppe mit 1 bis 6 Kohlenstoffatomen und einer Acylgruppe mit 2 bis 5 Kohlenstoffatomen ausgewählten Substituenten aufweisen kann;
A¹ für ein Stickstoffatom oder eine Partialstruktur der Formel (III): 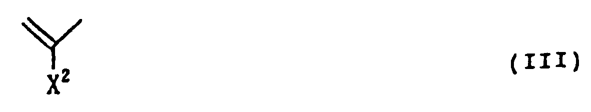 steht,
(worin X² für ein Wasserstoffatom, eine Aminogruppe, ein Halogenatom, eine Cyanogruppe, eine Halogenmethylgruppe, eine Halogenmethoxylgruppe, eine Alkylgruppe mit 1 bis 6 Kohlenstoffatomen, eine Alkenylgruppe mit 2 bis 6 Kohlenstoffatomen, eine Alkinylgruppe mit 2 bis 6 Kohlenstoffatomen oder eine Alkoxylgruppe mit 1 bis 6 Kohlenstoffatomen steht, wobei die Aminogruppe wenigstens einen unter einer Formylgruppe, einer Alkylgruppe mit 1 bis 6 Kohlenstoffatomen und einer Acylgruppe mit 2 bis 5 Kohlenstoffatomen ausgewählten Substituenten aufweisen kann, und
X² und R⁸ zusammen eine zyklische Struktur bilden können, an der ein Teil des Grundgerüsts beteiligt ist, und der Ring als ringbildendes Atom ein Sauerstoffatom, ein Stickstoffatom oder ein Schwefelatom enthalten und des weiteren als Substituenten eine Alkylgruppe mit 1 bis 6 Kohlenstoffatomen aufweisen kann); und
Y¹ für ein Wasserstoffatom, eine Phenylgruppe, eine Acetoxymethylgruppe, eine Pivaloyloxymethylgruppe, eine Ethoxycarbonylgruppe, eine Cholingruppe, eine Dimethylaminoethylgruppe, eine 5-Indanylgruppe, eine Phthalidinylgruppe, eine 5-Alkyl-2-oxo-1,3-dioxol-4-ylmethylgruppe, eine 3-Acetoxy-2-oxobutylgruppe, eine Alkylgruppe mit 1 bis 6 Kohlenstoffatomen, eine Alkoxymethylgruppe mit 2 bis 7 Kohlenstoffatomen oder eine aus einer Alkylengruppe mit 1 bis 6 Kohlenstoffatomen und einer Phenylgruppe zusammengesetzte Phenylalkylgruppe steht]
oder Q eine Partialstruktur der Formel (IV): 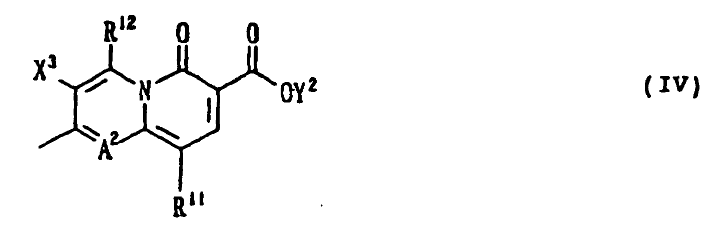 ist,
[worin R¹¹ für eine 2-Halogencyclopropylgruppe steht;
R¹² für ein Wasserstoffatom, eine Aminogruppe, eine Hydroxylgruppe, eine Thiolgruppe, eine Halogenmethylgruppe, eine Alkylgruppe mit 1 bis 6 Kohlenstoffatomen, eine Alkenylgruppe mit 2 bis 6 Kohlenstoffatomen, eine Alkinylgruppe mit 2 bis 6 Kohlenstoffatomen oder eine Alkoxylgruppe mit 1 bis 6 Kohlenstoffatomen steht, wobei die Aminogruppe wenigstens einen unter einer Formylgruppe, einer Alkylgruppe mit 1 bis 6 Kohlenstoffatomen und einer Acylgruppe mit 2 bis 5 Kohlenstoffatomen ausgewählten Substituenten aufweisen kann;
X³ für ein Halogenatom oder ein Wasserstoffatom steht;
A² für ein Stickstoffatom oder eine Partialstruktur der Formel (V): 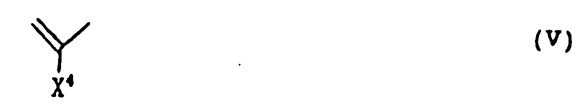 steht,
(worin X⁴ für ein Wasserstoffatom, eine Aminogruppe, ein Halogenatom, eine Cyanogruppe, eine Halogenmethylgruppe, eine Halogenmethoxylgruppe, eine Alkylgruppe mit 1 bis 6 Kohlenstoffatomen, eine Alkenylgruppe mit 2 bis 6 Kohlenstoffatomen, eine Alkinylgruppe mit 2 bis 6 Kohlenstoffatomen oder eine Alkoxylgruppe mit 1 bis 6 Kohlenstoffatomen steht, wobei die Aminogruppe wenigstens einen unter einer Formylgruppe, einer Alkylgruppe mit 1 bis 6 Kohlenstoffatomen und einer Acylgruppe mit 2 bis 5 Kohlenstoffatomen ausgewählten Substituenten aufweisen kann, und
X⁴ und R¹¹ zusammen eine zyklische Struktur bilden können, an der ein Teil des Grundgerüsts beteiligt ist, und der Ring als ringbildendes Atom ein Sauerstoffatom, ein Stickstoffatom oder ein Schwefelatom enthalten und des weiteren als Substituenten eine Alkylgruppe mit 1 bis 6 Kohlenstoffatomen aufweisen kann);
Y² für ein Wasserstoffatom, eine Phenylgruppe, eine Acetoxymethylgruppe, eine Pivaloyloxymethylgruppe, eine Ethoxycarbonylgruppe, eine Cholingruppe, eine Dimethylaminoethylgruppe, eine 5-Indanylgruppe, eine Phthalidinylgruppe, eine 5-Alkyl-2-oxo-1,3-dioxol-4-ylmethylgruppe, eine 3-Acetoxy-2-oxobutylgruppe, eine Alkylgruppe mit 1 bis 6 Kohlenstoffatomen, eine Alkoxymethylgruppe mit 2 bis 7 Kohlenstoffatomen oder eine aus einer Alkylengruppe mit 1 bis 6 Kohlenstoffatomen und einer Phenylgruppe zusammengesetzte Phenylalkylgruppe steht],
und ihre Salze und Hydrate davon.

2. Verbindung, ihre Salze und Hydrate davon nach Anspruch 1, wobei Q in der Formel (I) eine Struktur der Formel (II) besitzt.

3. Verbindung, ihre Salze und Hydrate davon nach Anspruch 2, wobei R⁸ eine 1,2-cis-2-Halogencyclopropylgruppe ist.

4. Verbindung, ihre Salze und Hydrate davon nach Anspruch 3, wobei die 1,2-cis-2-Halogencyclopropylgruppe in der Formel (I) ein stereochemisch reiner Substituent ist.

5. Verbindung, ihre Salze und Hydrate davon, nach Anspruch 4, wobei die Halogencyclopropylgruppe in der Formel (I) eine (1 R,2S)-2-Halogencyclopropylgruppe ist.

6. Verbindung, ihre Salze und Hydrate davon nach Anspruch 3, 4 oder 5, wobei das Halogenatom der Halogencyclopropylgruppe in der Formel (I) ein Fluoratom ist.

7. Verbindung, ihre Salze und Hydrate davon nach einem der vorhergehenden Ansprüche, wobei die Verbindung der Formel (I) eine stereochemisch reine Verbindung ist.

8. Verbindung, ihre Salze und Hydrate davon nach Anspruch 1, nämlich 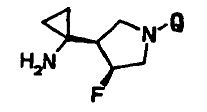
worin Q eine unter den Gruppen: 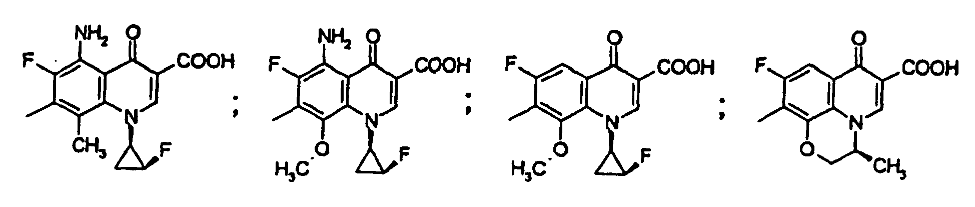 ausgewählte Partialstruktur ist.

9. Verbindung, ihre Salze und Hydrate davon nach Anspruch 1, nämlich 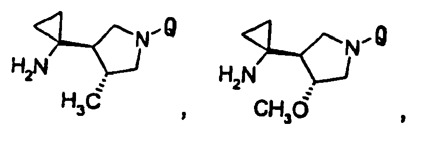 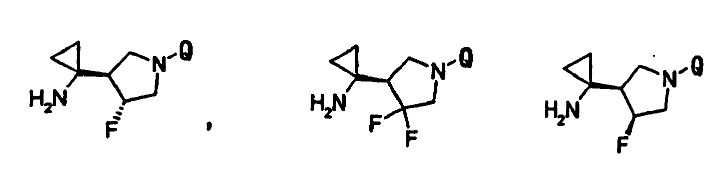
worin Q eine Partialstruktur: 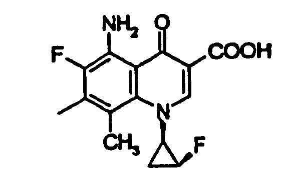 ist.

10. Verbindung, ihre Salze und Hydrate davon nach Anspruch 1, nämlich
5-Amino-7-[(3R,4R)-3-(1-aminocyclopropyl)-4-methyl-1-pyrrolidinyl]-6-fluor-1-[(1R,2S)-2-fluorcyclopropyl]-1,4-dihydro-8-methyl-4-oxochinolin-3-carbonsäure;
5-Amino-7-[4-(R)-(1-aminocyclopropyl)-3-(R)-methoxy-1-pyrrolidinyl]-6-fluor-1-[2-(S)-fluor-1-(R)-cyclopropyl]-1,4-dihydro-8-methyl-4-oxochinolin-3-carbonsäure;
5-Amino-7-[4-(R)-(1-aminocyclopropyl)-3-(R)-fluor-1-pyrrolidinyl]-6-fluor-1-[2-(S)-fluor-1-(R)-cyclopropyl]-1,4-dihydro-8-methyl-4-oxochinolin-3-carbonsäurehydrochlorid;
5-Amino-7-[4-(R)-(1-aminocyclopropyl)-3,3-difluor-1-pyrrolidinyl]-6-fluor-1-[2-(S)-fluor-1-(R)-cyclopropyl]-1,4-dihydro-8-methyl-4-oxochinolin-3-carbonsäurehydrochlorid;
5-Amino-7-[4-(R)-(1-aminocyclopropyl)-3-(S)-fluor-1-pyrrolidinyl]-6-fluor-1-[2-(S)-fluor-1-(R)-cyclopropyl]-1,4-dihydro-8-methyl-4-oxochinolin-3-carbonsäurehydrochlorid;
5-Amino-7-[4-(R)-(1-aminocyclopropyl)-3-(S)-fluor-1-pyrrolidinyl]-6-fluor-1-[2-(S)-fluor-1-(R)-cyclopropyl]-1,4-dihydro-8-methoxy-4-oxochinolin-3-carbonsäure;
7-[4-(R)-(1-Aminocyclopropyl)-3-(S)-fluor-1-pyrrolidinyl]-6-fluor-1-[2-(S)-fluor-1-(R)-cyclopropyl]-1,4-dihydro-8-methoxy-4-oxochinolin-3-carbonsäure;
5-Amino-7-[cis-4-(1-aminocyclobutyl)-3-fluor-1-pyrrolidinyl]-6-fluor-1-[2-(S)-fluor-1-(R)-cyclopropyl]-1,4-dihydro-8-methyl-4-oxochinolin-3-carbonsäure.

11. Verbindung, ihre Salze und Hydrate davon nach Anspruch 1, nämlich
10-[4-(R)-(1-Aminocyclopropyl)-3-(R)-fluor-1-pyrrolidinyl]-9-fluor-2,3-dihydro-3-(S)-methyl-7-oxo-7H-pyrido[1.2.3-de][1.4]benzoxazin-6-carbonsäure;
10-[4-(R)-(1-Aminocyclopropyl)-3,3-difluor-1-pyrrolidinyl]-9-fluor-2,3-dihydro-3-(S)-methyl-7-oxo-7H-pyrido[1.2.3-de][1.4]benzoxazin-6-carbonsäure;
10-[4-(R)-(1-Aminocyclopropyl)-3-(S)-fluor-1-pyrrolidinyl]-9-fluor-2,3-dihydro-3-(S)-methyl-7-oxo-7H-pyrido[1.2.3-de][1.4]benzoxazin-6-carbonsäure;

12. Verfahren zur Herstellung einer Verbindung, ihre Salze und Hydrate davon nach einem der vorhergehenden Ansprüche, wobei man eine Verbindung der Formel (VI): 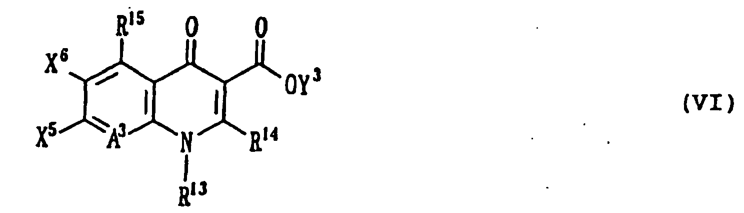 [worin X⁵ eine Abgangsgruppe ist; Y³ der in Anspruch 1 definierte Rest Y¹ oder eine Gruppe der Formel (VII): 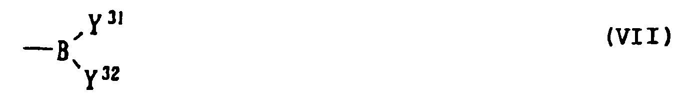 ist,
[worin Y³¹ und Y³² jeweils für ein Fluoratom oder eine Alkylcarbonyloxygruppe mit 2 bis 5 Kohlenstoffatomen stehen), und R¹³, R¹⁴, R¹⁵, A³ und X⁶ den in Anspruch 1 definierten Resten R⁸, R⁹, R¹⁰, A¹ und X¹ entsprechen],
oder eine Verbindung der Formel (VIII): 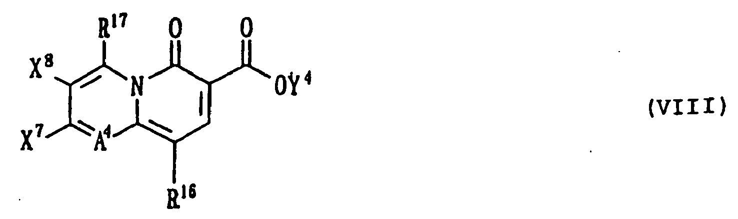
[worin X⁷ eine Abgangsgruppe ist; und R¹⁶, R¹⁷, A⁴, X⁸ und Y⁴ den in Anspruch 1 definierten Resten R¹¹, R¹², A², X³ und Y² entsprechen],
mit einer Verbindung der Formel (IX): [worin R¹¹¹ der in Anspruch 1 definierte Rest R¹ oder eine Aminoschutzgruppe is und R², R³, R⁴, R⁵, R⁶, R⁷ und n wie in Anspruch 1 definiert sind], oder mit einem Säureadditionssalz davon umsetzt, Schutzgruppen erforderlichenfall entfernt und die Verbindung der Formel (I), ihre Salze oder Hydrate davon gewinnt. 13. Verbindung der Formel (IX): 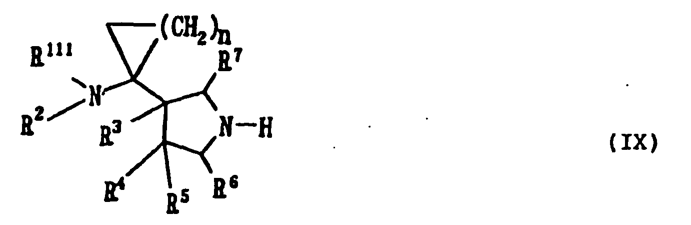 [worin R¹¹¹ der in Anspruch 1 definierte Rest R¹ oder eine Aminoschutzgruppe ist und R², R³, R⁴, R⁵, R⁷ und n wie in Anspruch 1 definiert sind], oder mit einem Säureadditionssalz davon umsetzt, Schutzgruppen erforderlichenfalls entfernt und die Verbindung der Formel (I), ihre Salze oder Hydrate davon gewinnt.

13. Verbindung der Formel (IX): 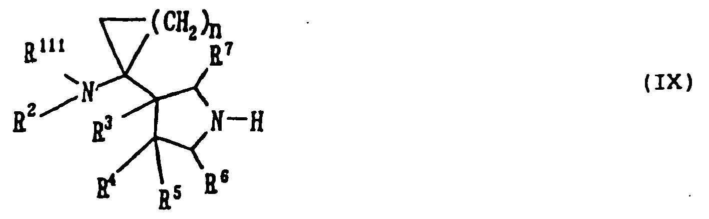
{worin R¹¹¹ für ein Wasserstoffatom, eine Alkylgruppe mit 1 bis 6 Kohlenstoffatomen oder eine Aminoschutzgruppe steht; R² für ein Wasserstoffatom oder eine Alkylgruppe mit 1 bis 6 Kohlenstoffatomen steht, wobei die Akylgruppe wenigstens einen unter einer Hydroxylgruppe, einem Halogenatom, einer Alkylthiogruppe mit 1 bis 6 Kohlenstoffatomen und einer Alkoxylgruppe mit 1 bis 6 Kohlenstoffatomen ausgewählten Substituenten aufweisen kann;
R³ für ein Wasserstoffatom, eine Hydroxylgruppe, ein Halogenatom, eine Carbamoylgruppe, eine Alkylgruppe mit 1 bis 6 Kohlenstoffatomen, eine Alkoxylgruppe mit 1 bis 6 Kohlenstoffatomen oder eine Alkylthiogruppe mit 1 bis 6 Kohlenstoffatomen steht, wobei die Alkylgruppe wenigstens einen unter einer Hydroxylgruppe, einem Halogenatom und einer Alkoxylgruppe mit 1 bis 6 Kohlenstoffatomen ausgewählten Substituenten aufweisen kann;
R⁴ für ein Wasserstoffatom, eine Hydroxylgruppe, ein Halogenatom, eine Carbamoylgruppe, eine Alkylgruppe mit 1 bis 6 Kohlenstoffatomen, eine Alkoxylgruppe mit 1 bis 6 Kohlenstoffatomen oder eine Alkylthiogruppe mit 1 bis 6 Kohlenstoffatomen steht, und
R⁵ für eine Hydroxylgruppe, ein Halogenatom, eine Carbamoylgruppe, eine Alkylgruppe mit 1 bis 6 Kohlenstoffatomen, eine Alkoxylgruppe mit 1 bis 6 Kohlenstoffatomen oder eine Alkylthiogruppe mit 1 bis 6 Kohlenstoffatomen steht, wobei die Alkylgruppe wenigstens einen unter einer Hydroxylgruppe, einem Halogenatom und einer Alkoxylgruppe mit 1 bis 6 Kohlenstoffatomen ausgewählten Substituenten aufweisen kann, und
R⁴ und R⁵ zusammen eine Hydroxyiminogruppe, eine Methylenkette mit 3 bis 6 Kohlenstoffatomen (so dass zusammen mit dem Pyrrolidinring eine spirozyklische Struktur gebildet wird) oder eine Alkyloxyiminogruppe mit 1 bis 6 Kohlenstoffatomen bilden können;
R⁶ und R⁷ jeweils unabhängig voneinander für ein Wasserstoffatom oder eine Alkylgruppe mit 1 bis 6 Kohlenstoffatomen stehen; und
n eine ganze Zahl mit einem Wert von 1 bis 3 ist];
oder ein Säureadditionssalz davon.

14. Verbindung und Säureadditionssalze davon nach Anspruch 13, nämlich 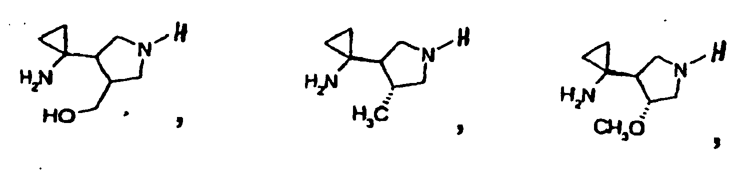 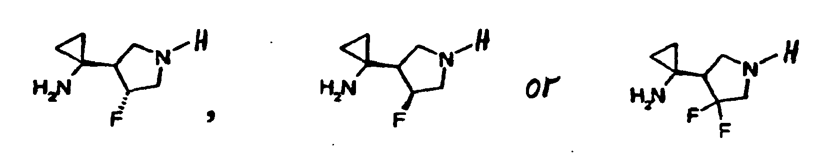
worin die Aminogruppe geschützt sein kann.

15. Verbindung und Säureadditionssalze davon nach Anspruch 13 oder 14, worin die Aminoschutzgruppe ausgewählt ist unter Alkoxycarbonylgruppen wie tert-Butoxycarbonyl, 2,2,2-Trichlorethoxycarbonyl, Aralkyloxycarbonylgruppen wie Benzyloxycarbonyl, p-Methoxybenzyloxycarbonyl, p-Nitrobenzyloxycarbonyl, Acylgruppen wie Acetyl, Methoxyacetyl, Trifluoracetyl, Chloracetyl, Pivaloyl, Formyl, Benzoyl, Alkyl- und Aralkylgruppen wie tert-Butyl, Benzyl, p-Nitrobenzyl, p-Methoxybenzyl, Triphenylmethylether wie Methoxymethyl, tert-Butoxymethyl, Tetrahydropyranyl, 2,2,2-Trichlorethoxymethyl, und Silylgruppen wie Trimethylsilyl, Isopropyldimethylsilyl, tert-Butyldimethylsilyl, Tribenzylsilyl, tert-Butyldiphenylsilyl.

16. Pharmazeutische Zubereitung, die eine Verbindung, ihre Salze oder ein Hydrat davon, wie in einem der Ansprüche 1 bis 11 definiert, als aktiven Inhaltsstoff umfasst.

17. Antibakterielles Arzneimittel, dass eine Verbindung, ihr Salz oder ein Hydrat davon, wie in einem der Ansprüche 1 bis 11 definiert, als aktiven Inhaltsstoff umfasst.

18. Verwendung einer Verbindung, ihres Salzes oder eines Hydrates davon, wie in einem der Ansprüche 1 bis 11 definiert, zur Herstellung eines pharmazeutischen Mittels zur Behandlung, Prävention oder Linderung infektiöser Erkrankungen.

## Revendications

1. Composé représenté par la formule (I) : 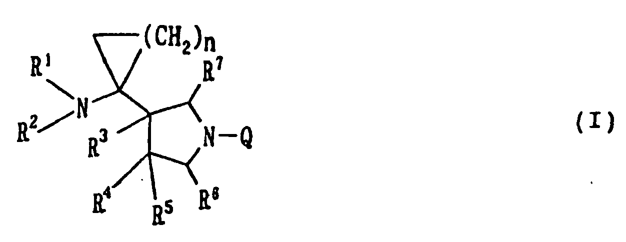
{dans laquelle R¹ représente un atome d'hydrogène ou un groupe alkyle ayant de 1 à 6 atomes de carbone ;
R² représente un atome d'hydrogène ou un groupe alkyle ayant de 1 à 6 atomes de carbone,
où le groupe alkyle peut porter au moins un substituant choisi dans l'ensemble constitué par un groupe hydroxyle, un atome d'halogène, un groupe alkylthio ayant de 1 à 6 atomes de carbone et un groupe alcoxy ayant de 1 à 6 atomes de carbone ;
R³ représente un atome d'hydrogène, un groupe hydroxyle, un atome d'halogène, un groupe carbamoyle, un groupe alkyle ayant de 1 à 6 atomes de carbone, un groupe alcoxy ayant de 1 à 6 atomes de carbone ou un groupe alkylthio ayant de 1 à 6 atomes de carbone,
où le groupe alkyle peut porter au moins un substituant choisi dans l'ensemble constitué par un groupe hydroxyle, un atome d'halogène et un groupe alcoxy ayant de 1 à 6 atomes de carbone ;
R⁴ représente un atome d'hydrogène, un groupe hydroxyle, un atome d'halogène, un groupe carbamoyle, un groupe alkyle ayant de 1 à 6 atomes de carbone, un groupe alcoxy ayant de 1 à 6 atomes de carbone ou un groupe alkylthio ayant de 1 à 6 atomes de carbone, et R⁵ représente un groupe hydroxyle, un atome d'halogène, un groupe carbamoyle, un groupe alkyle ayant de 1 à 6 atomes de carbone, un groupe alcoxy ayant de 1 à 6 atomes de carbone ou un groupe alkylthio ayant de 1 à 6 atomes de carbone,
où le groupe alkyle peut porter au moins un substituant choisi dans l'ensemble constitué par un groupe hydroxyle, un atome d'halogène et un groupe alcoxy ayant de 1 à 6 atomes de carbone, et
R⁴ et R⁵ peuvent être combinés pour former un groupe hydroxyimino, une chaîne méthylène ayant de 3 à 6 atomes de carbone (de façon à former une structure cyclique spiro conjointement avec le cycle pyrrolidine) ou un groupe alkyloxyimino ayant de 1 à 6 atomes de carbone ;
chacun de R⁶ et R⁷ représente indépendamment un atome d'hydrogène ou un groupe alkyle ayant de 1 à 6 atomes de carbone ;
n est un entier de 1 à 3 ; et
Q est une structure partielle représentée par la formule (II) : 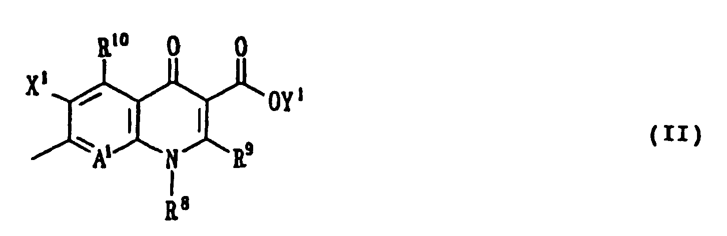
[dans laquelle R⁸ représente un groupe 2-halogénocyclopropyle ;
R⁹ représente un atome d'hydrogène ou un groupe alkylthio ayant de 1 à 6 atomes de carbone,
ou dans laquelle R⁸ et R⁹ peuvent être combinés pour former une structure cyclique comprenant une partie du noyau mère, et le cycle peut contenir un atome de soufre servant d'atome constitutif du cycle et peut en outre porter, en tant que substituant, un groupe alkyle ayant de 1 à 6 atomes de carbone,
X¹ représente un atome d'halogène ou un atome d'hydrogène ;
R¹⁰ représente un atome d'hydrogène, un groupe amino, un groupe hydroxyle, un groupe thiol, un groupe halogénométhyle, un groupe alkyle ayant de 1 à 6 atomes de carbone, un groupe alcényle ayant de 2 à 6 atomes de carbone, un groupe alcynyle ayant de 2 à 6 atomes de carbone ou un groupe alcoxy ayant de 1 à 6 atomes de carbone,
où le groupe amino peut porter au moins un substituant choisi dans l'ensemble constitué par un groupe formyle, un groupe alkyle ayant de 1 à 6 atomes de carbone et un groupe acyle ayant de 2 à 5 atomes de carbone ;
A¹ représente un atome d'azote, ou une structure partielle représentée par la formule (III) : 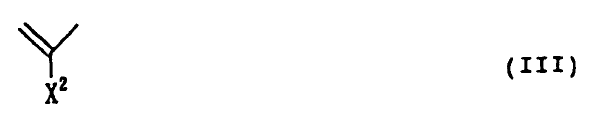 (dans laquelle X² représente un atome d'hydrogène, un groupe amino, un atome d'halogène, un groupe cyano, un groupe halogénométhyle, un groupe halogénométhoxy, un groupe alkyle ayant de 1 à 6 atomes de carbone, un groupe alcényle ayant de 2 à 6 atomes de carbone, un groupe alcynyle ayant de 2 à 6 atomes de carbone ou un groupe alcoxy ayant de 1 à 6 atomes de carbone,
où le groupe amino peut porter au moins un substituant choisi dans le groupe constitué par un groupe formyle, un groupe alkyle ayant de 1 à 6 atomes de carbone et un groupe acyle ayant de 2 à 5 atomes de carbone, et
X² et R⁸ peuvent être combinés pour former une structure cyclique comprenant une partie du noyau mère, et le cycle peut contenir un atome d'oxygène, un atome d'azote ou un atome de soufre en tant qu'atome constitutif du cycle et peut en outre porter, en tant que substituant, un groupe alkyle ayant de 1 à 6 atomes de carbone) ; et
Y¹ représente un atome d'hydrogène, un groupe phényle, un groupe acétoxyméthyle, un groupe pivaloyloxyméthyle, un groupe éthoxycarbonyle, un groupe choline, un groupe diméthylaminoéthyle, un groupe 5-indanyle, un groupe phtalidinyle, un groupe 5-alkyl-2-oxo-1,3-dioxol-4-ylméthyle, un groupe 3-acétoxy-2-oxobutyle, un groupe alkyle ayant de 1 à 6 atomes de carbone, un groupe alcoxyméthyle ayant de 2 à 7 atomes de carbone ou un groupe phénylalkyle composé d'un groupe alkylène ayant de 1 à 6 atomes de carbone et d'un groupe phényle] ; ou bien
Q est une structure partielle représentée par la formule (IV) : 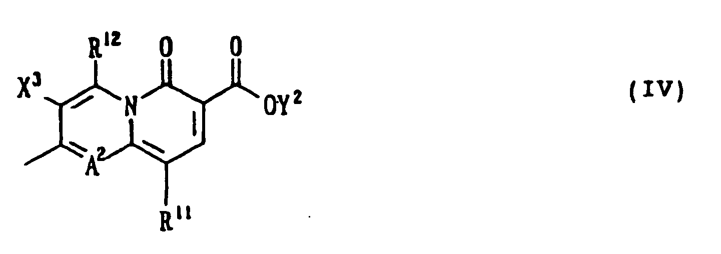
[dans laquelle R¹¹ représente un groupe 2-halogénocyclopropyle ;
R¹² représente un atome d'hydrogène, un groupe amino, un groupe hydroxyle, un groupe thiol, un groupe halogénométhyle, un groupe alkyle ayant de 1 à 6 atomes de carbone, un groupe alcényle ayant de 2 à 6 atomes de carbone, un groupe alcynyle ayant de 2 à 6 atomes de carbone ou un groupe alcoxy ayant de 1 à 6 atomes de carbone,
où le groupe amino peut porter au moins un substituant choisi dans le groupe constitué par un groupe formyle, un groupe alkyle ayant de 1 à 6 atomes de carbone et un groupe acyle ayant de 2 à 5 atomes de carbone ;
X³ représente un atome d'halogène ou un atome d'hydrogène ;
A² représente un atome d'azote ou une structure partielle représentée par la formule (V) : 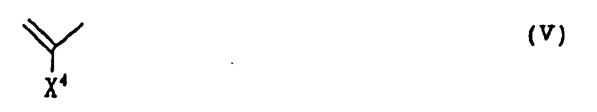
(dans laquelle X⁴ représente un atome d'hydrogène, un groupe amino, un atome d'halogène, un groupe cyano, un groupe halogénométhyle, un groupe halogénométhoxy, un groupe alkyle ayant de 1 à 6 atomes de carbone, un groupe alcényle ayant de 2 à 6 atomes de carbone, un groupe alcynyle ayant de 2 à 6 atomes de carbone ou un groupe alcoxy ayant de 1 à 6 atomes de carbone,
où le groupe amino peut porter au moins un substituant choisi dans l'ensemble constitué par un groupe formyle, un groupe alkyle ayant de 1 à 6 atomes de carbone et un groupe acyle ayant de 2 à 5 atomes de carbone, et
X⁴ et R¹¹ peuvent être combinés pour former une structure cyclique contenant une partie du noyau mère, et le cycle peut contenir un atome d'oxygène, un atome d'azote ou un atome de soufre en tant qu'atome constitutif du cycle et peut en outre porter, en tant que substituant, un groupe alkyle ayant de 1 à 6 atomes de carbone) ; et
Y² représente un atome d'hydrogène, un groupe phényle, un groupe acétoxyméthyle, un groupe pivaloyloxyméthyle, un groupe éthoxycarbonyle, un groupe choline, un groupe diméthylaminoéthyle, un groupe 5-indanyle, un groupe phtalidinyle, un groupe 5-alkyl-2-oxo-1,3-dioxol-4-ylméthyle, un groupe 3-acétoxy-2-oxobutyle, un groupe alkyle ayant de 1 à 6 atomes de carbone, un groupe alcoxyméthyle ayant de 2 à 7 atomes de carbone ou un groupe phénylalkyle composé d'un groupe alkylène ayant de 1 à 6 atomes de carbone et d'un groupe phényle]},
et ses sels et hydrates.

2. Composé, et ses sels et hydrates, selon la revendication 1, dans lesquels Q dans la formule (I) a une structure représentée par la formule (II).

3. Composés, et ses sels et hydrates, selon la revendication 2, dans lesquels R¹¹ est un groupe 1,2-cis-2-halogénocyclopropyle.

4. Composés, et ses sels et hydrates, selon la revendication 3, dans lesquels le groupe 1,2-cis-2-halogénocyclopropyle dans la formule (I) est un substituant stéréochimiquement pur.

5. Composés, et ses sels et hydrates, selon la revendication 4, dans lesquels le groupe halogénocyclopropyle dans la formule (I) est un groupe (1R,2S)-2-halogénocyclopropyle.

6. Composés, et ses sels et hydrates, selon la revendication 3, 4 ou 5, dans lesquels l'atome d'halogène du groupe halogénocyclopropyle dans la formule (I) est un atome de fluor.

7. Composés, et ses sels et hydrates, selon l'une quelconque des revendications précédentes, dans lesquels le composé de formule (I) est un composé stéréochimiquement pur.

8. Composés, et ses sels et hydrates, selon la revendication 1, à savoir 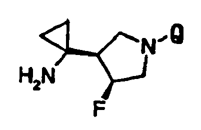
dans lesquels Q est une structure partielle choisie dans l'ensemble constitué par :

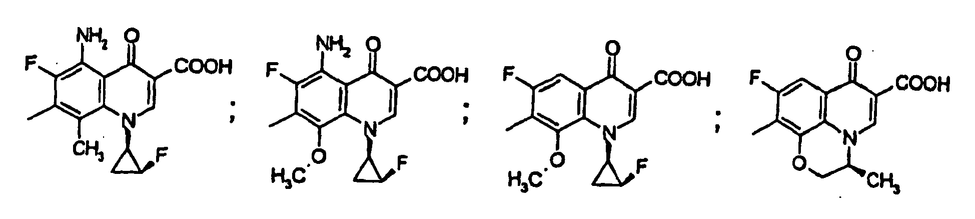

9. Composé, et ses sels et hydrates, selon la revendication 1, à savoir : 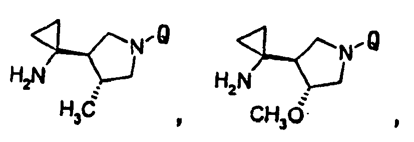 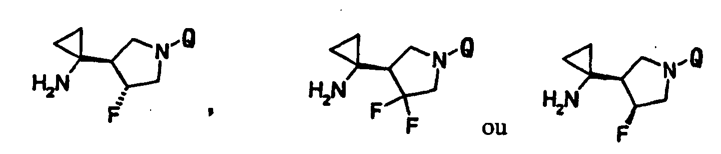 dans lesquels Q est la structure partielle :

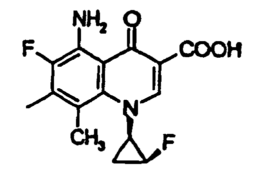

10. Composé, et ses sels et hydrates, selon la revendication 1, à savoir les suivants
acide 5-amino-7-[(3R,4R)-3-(1-aminocyclopropyl)-4-méthyl-1-pyrrolidinyl]-6-fluoro-1-[(1R,2S)-2-fluorocyclopropyl]-1,4-dihydro-8-méthyl-4-oxoquinoline-3-carboxylique ;
acide 5-amino-7-[4-(R)-(1-aminocylopropyl)-3-(R)-méthoxy-1-pyrrolidinyl]-6-fluoro-1-[2-(S)-fluoro-1-(R)-cyclopropyl]-1,4-dihydro-8-méthyl-4-oxoquinoline-3-carboxylique ;
chlorhydrate d'acide 5-amino-7-[4-(R)-(1-aminocyclopropyl)-3-(R)-fluoro-1-pyrrolidinyl]-6-fluoro-1-[2-(S)-fluoro-1-(R)-cyclopropyl]-1,4-dihydro-8-méthyl-4-oxoquinoline-3-carboxylique ;
chlorhydrate d'acide 5-amino-7-[4-(R)-(1-aminocyclopropyl)-3,3-difluoro-1-pyrrolidinyl]-6-fluoro-1-[2-(S)-fluoro-1-(R)-cyclopropyl]-1,4-dihydro-8-méthyl-4-oxoquinoline-3-carboxylique ;
chlorhydrate d'acide 5-amino-7-[4-(R)-(1-aminocyclopropyl)-3-(S)-fluoro-1-pyrrolidinyl]-6-fluoro-1-[2-(S)-fluoro-1-(R)-cyclopropyl]-1,4-dihydro-8-méthyl-4-oxoquinoline-3-carboxylique ;
acide 5-amino-7-[4-(R)-(1-aminocyclopropyl)-3-(S)-fluoro-1-pyrrolidinyl]-6-fluoro-1-[2-(S)-fluoro-1-(R)-cyclopropyl]-1,4-dihydro-8-méthoxy-4-oxoquinoline-3-carboxylique ;
acide 7-[4-(R)-(1-aminocyclopropyl)-3-(S)-fluoro-1-pyrrolidinyl]-6-fluoro-1-[2-(S)-fluoro-1-(R)-cyclopropyl]-1,4-dihydro-8-méthoxy-4-oxoquinoline-3-carboxylique ;
acide 5-amino-7-[cis-4-(1-aminocyclobutyl)-3-fluoro-1-pyrrolidinyl]-6-fluoro-1-[2-(S)-fluoro-1-(R)-cyclopropyl]-1,4-dihydro-8-méthyl-4-oxoquinoline-3-carboxylique.

11. Composé, et ses sels et hydrates, selon la revendication 1, à savoir les suivants
acide 10-[4-(R)-(1-aminocyclopropyl)-3-(R)-fluoro-1-pyrrolidinyl)-9-fluoro-2,3-dihydro-3-(S)-méthyl-7-oxo-7H-pyrido[1.2.3-de][1.4]benzoxazine-6-carboxylique ;
acide 10-[4-(R)-(1-aminocyclopropyl)-3,3-difluoro-1-pyrrolidinyl)-9-fluoro-2,3-dihydro-3-(S)-méthyl-7-oxo-7H-pyrido[1.2.3-de][1.4]benzoxazine-6-carboxylique ;
acide 10-[4-(R)-(1-aminocyclopropyl)-3-(S)-fluoro-1-pyrrolidinyl)-9-fluoro-2,3-dihydro-3-(S)-méthyl-7-oxo-7H-pyrido[1.2.3-de][1.4]benzoxazine-6-carboxylique.

12. Procédé pour produire un composé, et ses sels et hydrates, selon l'une quelconque des revendications précédentes, procédé dans lequel un composé représenté par la formule (VI) : 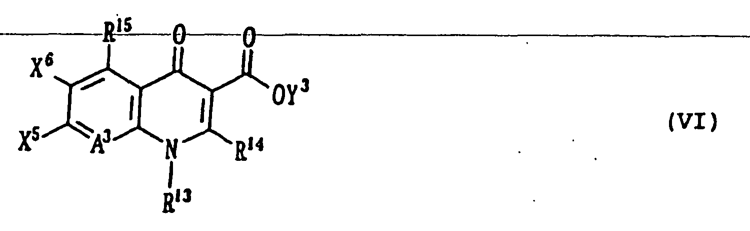 [dans laquelle X⁵ est un groupe partant ; Y³ est Y¹ tel que défini dans la revendication 1 ou un groupe représenté par la formule (VII) : 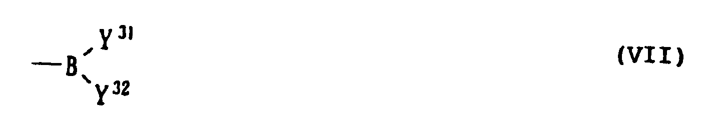 (dans laquelle chacun de Y³¹ et Y³² est un atome de fluor ou un groupe alkylcarbonyloxy ayant de 2 à 5 atomes de carbone), et
R¹³, R¹⁴, R¹⁵, A³ et X⁶ correspondent à R⁸, R⁹, R¹⁰, A¹ et X¹ tels que définis dans la revendication 1],
ou un composé représenté par la formule (VIII) : 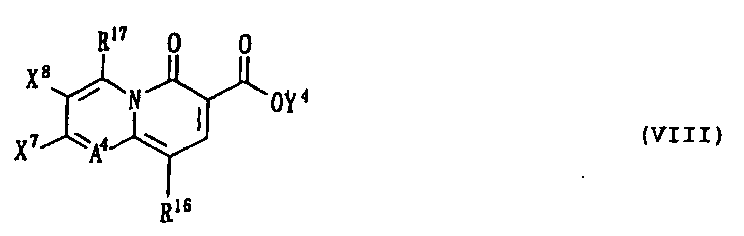
[dans laquelle X⁷ est un groupe partant ; et R¹⁶, R¹⁷, A⁴, X⁸ et Y⁴ correspondent à R¹¹, R¹², A², X³ et Y² tels que définis dans la revendication 1],
est laissé à réagir avec un composé représenté par la formule (IX) : 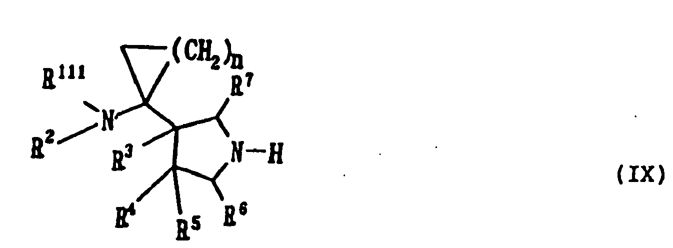 [dans laquelle R¹¹¹ est R¹ tel que défini dans la revendication 1 ou un groupe protecteur du groupe amino et R², R³, R⁴, R⁵, R⁶, R⁷ et n sont tels que définis dans la revendication 1] ou avec un sel d'addition d'acide de celui-ci, les groupes protecteurs sont éliminés, si c'est nécessaire, et le composé de formule (I), et ses sels et hydrates, sont récupérés.

13. Composé représenté par la formule (IX) : 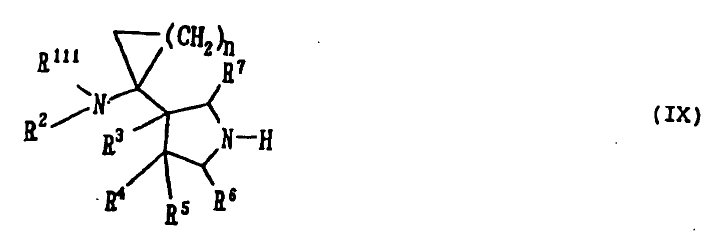 [dans laquelle R¹¹¹ représente un atome d'hydrogène, un groupe alkyle ayant de 1 à 6 atomes de carbone ou un groupe protecteur du groupe amino ; R² représente un atome d'hydrogène ou un groupe alkyle ayant de 1 à 6 atomes de carbone,
où le groupe alkyle peut porter au moins un substituant choisi dans le groupe constitué par un groupe hydroxyle, un atome d'halogène, un groupe alkylthio ayant de 1 à 6 atomes de carbone et un groupe alcoxy ayant de 1 à 6 atomes de carbone ;
R³ représente un atome d'hydrogène, un groupe hydroxyle, un atome d'halogène, un groupe carbamoyle, un groupe alkyle ayant de 1 à 6 atomes de carbone, un groupe alcoxy ayant de 1 à 6 atomes de carbone ou un groupe alkylthio ayant de 1 à 6 atomes de carbone,
où le groupe alkyle peut porter au moins un substituant choisi dans l'ensemble constitué par un groupe hydroxyle, un atome d'halogène et un groupe alcoxy ayant de 1 à 6 atomes de carbone ;
R⁴ représente un atome d'hydrogène, un groupe hydroxyle, un atome d'halogène, un groupe carbamoyle, un groupe alkyle ayant de 1 à 6 atomes de carbone, un groupe alcoxy ayant de 1 à 6 atomes de carbone ou un groupe alkylthio ayant de 1 à 6 atomes de carbone, et R⁵ représente un groupe hydroxyle, un atome d'halogène, un groupe carbamoyle, un groupe alkyle ayant de 1 à 6 atomes de carbone, un groupe alcoxy ayant de 1 à 6 atomes de carbone ou un groupe alkylthio ayant de 1 à 6 atomes de carbone,
où le groupe alkyle peut porter au moins un substituant choisi dans l'ensemble constitué par un groupe hydroxyle, un atome d'halogène et un groupe alcoxy ayant de 1 à 6 atomes de carbone, et
R⁴ et R⁵ peuvent être combinés pour former un groupe hydroxyimino, une chaîne méthylène ayant de 3 à 6 atomes de carbone (de façon à former une structure cyclique spiro conjointement avec le cycle pyrrolidine) ou un groupe alkyloxyimino ayant de 1 à 6 atomes de carbone ;
chacun de R⁶ et R⁷ représente indépendamment un atome d'hydrogène ou un groupe alkyle ayant de 1 à 6 atomes de carbone ; et n est un entier de 1 à 3] ;
ou un sel d'addition d'acide de ce composé.

14. Composé, et ses sels d'addition d'acide, selon la revendication 13, à savoir : 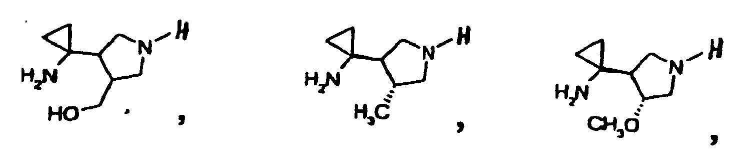 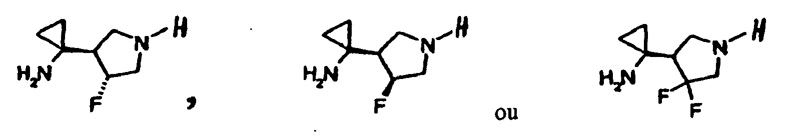
dans lesquels le groupe amino peut être protégé.

15. Composé, et ses sels d'addition d'acide, selon la revendication 13 ou 14,
dans lesquels le groupe protecteur de groupe amino est choisi parmi les groupes alcoxycarbonyle tels que tert-butoxycarbonyle, 2,2,2-trichloroéthoxycarbonyle, les groupes aralkyloxycarbonyle tels que benzyloxycarbonyle, p-méthoxybenzyloxycarbonyle, p-nitrobenzyloxycarbonyle, les groupes acyle tels qu'acétyle, méthoxyacétyle, trifluoroacétyle, chloroacétyle, pivaloyle, formyle, benzoyle, les groupes alkyle ou aralkyle tels que tert-butyle, benzyle, p-nitrobenzyle, p-méthoxybenzyle, les triphénylméthyléthers tels que méthoxyméthyle, tert-butoxyméthyle, tétrahydropyranyle, 2,2,2-trichloroéthoxyméthyle, et les groupes silyle tels que triméthylsilyle, isopropyldiméthylsilyle, tert-butyldiméthylsilyle, tribenzylsilyle, tert-butyldiphénylsilyle.

16. Préparation pharmaceutique qui comprend un composé, son sel ou un de ses hydrates, tel que défini dans l'une quelconque des revendications 1 à 11, à titre d'ingrédient actif.

17. Médicament antibactérien qui comprend un composé, son sel ou un de ses hydrates, tel que défini dans l'une quelconque des revendications 1 à 11, en tant qu'ingrédient actif.

18. Utilisation d'un composé, de son sel ou d'un de ses hydrates tel que défini dans l'une quelconque des revendications 1 à 11, pour préparer une composition pharmaceutique destinée à traiter, prévenir ou soulager des maladies infectieuses.
